# EUROPEAN PATENT APPLICATION

(11) **EP 4 796 121 A2**
(43) Date of publication of application: **26.08.2026**
(21) Application number: 26179699.9
(22) Date of filing: 16.03.2018
(51) Int. Cl.: A61K 39/00

(54) **COMBINATION OF RNA AND IMMUNE CHECKPOINT INHIBITORS**

(30) Priority: 17.03.2017 WO PCT/EP2017/056427
(62) Document number(s) of the earlier application(s) in accordance with Art. 76 EPC: 18710886.5 / 3 595 715
(71) Applicant: CureVac SE, 72076 Tübingen (DE); Boehringer Ingelheim International GmbH, 55216 Ingelheim am Rhein (DE)
(72) Inventor: HEIDENREICH, Regina, 72076 Tübingen (DE); FIEDLER, Katja, 72076 Tübingen (DE); FOTIN-MLECZEK, Mariola, 72076 Tübingen (DE); KOWALCZYK, Aleksandra, 72076 Tübingen (DE); ELBERS, Knut, 55216 Ingelheim am Rhein (DE); WURM, Melanie, 55216 Ingelheim am Rhein (DE)
(74) Representative: Graf von Stosch Patentanwaltsgesellschaft mbH

(57) **Abstract**

The present invention relates to the field of biomedicine, and in particular to the field of therapeutic nucleic acids. The present invention provides a novel combination of an RNA encoding an epitope and immune checkpoint inhibitors. A pharmaceutical composition, vaccine, and kit-of-parts comprising said combination are also provided. Furthermore, the present invention relates to the combination, (pharmaceutical) composition, vaccine or kit-of-parts for use in medicine, and in particular in the treatment and/or prophylaxis of cancer, infectious diseases and other diseases and disorders.

## Description

The present invention *inter alia* relates to a combination comprising an RNA comprising at least one coding sequence encoding at least one epitope of an antigen, at least one PD-1 pathway inhibitor and at least one LAG-3 pathway inhibitor, a (pharmaceutical) composition and kit-of-parts comprising said combination, as well as uses thereof in medicine and in particular therapy of a variety of diseases.

One of the hallmarks of cancer is the ability of the malignant cell to escape eradication by the immune system. The discovery of tumor antigens expressed on the surface of malignant cells sparked the hypothesis of cancer immune surveillance, where the adaptive immune system is responsible for preventing the development of cancer in immunocompetent hosts. Despite the recent advances with immune checkpoint-directed approaches, the concept of "immunotherapy" dates back to the 19th century and comprises distinct strategies, including vaccines, non-specific cytokines, and adoptive cell therapies.

This concept is based on the insight that the immune system can, in principle, be activated by antigens such as cancer antigens and, once primed, elicit an immune response which may effect cancer cell destruction. Unfortunately, the successful development of anti-cancer immunity is often hampered by a plethora of factors that can directly determine the adequacy of the immune response. Cancer cells can induce immune tolerance via multiple mechanisms, including regulatory immune cells, immunosuppressive chemokines, and immune checkpoints that suppress immune effector functions. One such evasive strategies of cancer cells involves the upregulation of certain surface ligands that mediate T-cell anergy or exhaustion by binding to negative regulatory T cell surface molecules which are upregulated in activated T cells to dampen their activity. These inhibitory molecules were termed negative co-stimulatory molecules due to their homology to the T cell co-stimulatory molecule CD28. These proteins, also referred to as immune checkpoint proteins, function in multiple pathways including the attenuation of early activation signals, competition for positive co-stimulation and direct inhibition of antigen presenting cells (Bour-Jordan et al., 2011. Immunol Rev. 241(1):180-205; PMID: 21488898). One member of this protein family is programmed death-1 (PD-1) and its ligands B7-H1/PD-L1 (CD274) and B7-DC/PD-L2 (CD273). The main function of the PD-1 pathway is the blockade of T cell activity. Thus, the interaction of PD-1 on activated T-cells and PD-L1 on tumor cells or antigen-presenting cells inhibits T-cell responses, e.g. T-cell mediated tumor cell killing. Another immune checkpoint protein is LAG-3, which is upregulated on activated T cells and a subset of natural killer cells. One ligand of LAG-3 is the MHC class II, which is expressed on antigen-presenting cells. Similarly to PD-1 signalling, the LAG-3 pathway is thought to damped T cell activity and effector functions.

Immune checkpoint therapy aims to reverse immunotolerance by targeting regulatory pathways in T cells including the interaction of PD-1/PD-L1 or LAG-3/MHC-II, thereby enhancing their effector functions. A wide variety of new immune-based cancer therapies are being currently developed for solid tumors. Immune checkpoint inhibitors have demonstrated huge potential as treatment option for solid tumors and other cancers. In particular, several monoclonal antibodies directed against PD-1, and PD-L1, as well as LAG-3 have been developed. Said antibodies usually act by blocking or disrupting the interaction between PD-1 and LAG-3 with their respective ligands, thereby preventing T-cell inhibition and restoring T cell mediated anti-tumor immune responses.

To date, two checkpoint inhibitors targeting PD-1 received the US Food and Drug Administration (FDA) approval for previously treated metastatic NSCLC: nivolumab and pembrolizumab. However, despite the extraordinary developmental effort in the field of tumor immunetherapy, the treatment of solid tumors and other cancers still represents an area of high unmet medical need. There is still a considerable number of tumor and cancer patients who do not benefit from therapy with immune checkpoint inhibitors alone.

So far, clinical trials with checkpoint inhibitors all showed approximately 10% to 40% objective response rates in the analyzed types of malignancies at late disease stages including melanoma, non-small-cell lung cancer, mismatch repair-deficient colorectal cancer or metastatic Merkel cell carcinoma. Therefore, treatment of solid tumors or other cancers in patients, particularly those not responding to immune checkpoint inhibition alone, remains an area of high unmet medical need.

(Chronic) infections represent another major clinical burden and are often characterized by resistances of the infectious pathogens to available antibiotic or antiviral therapeutics. In (chronic) infections, T cells are exposed to persistent antigen and/or inflammatory signals. This scenario is often associated with the deterioration of T cell function: a state called "exhaustion". Exhausted T cells lose robust effector functions, express multiple inhibitory receptors and are defined by an altered transcriptional programme. T cell exhaustion is often associated with inefficient control of persisting infections. Currently available therapies commonly rely on small organic molecules targeting the infective pathogens, but are often hampered by rapidly evolving resistances. There is thus an urgent need in the art to provide novel therapeutics capable of revitalizing exhausted pathogen-specific T cells in order to reinvigorate T cell mediated immunity against persisting pathogens.

It is an object of the present invention to comply with these needs and to provide improved therapeutic approaches for treatment of cancers, infectious diseases and other diseases and conditions defined herein. The object underlying the present invention is solved by the claimed subject matter.

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" e.g., a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" e.g., a composition which is "substantially free" from Y may be completely free from Y. Where necessary, the word "substantially" may be omitted from the definition of the invention.

The term "about" in relation to a numerical value x means x ± 10%.

In the present invention, if not otherwise indicated, different features of alternatives and embodiments may be combined with each other.

For the sake of clarity and readability the following definitions are provided. Any technical feature mentioned for these definitions may be read on each and every embodiment of the invention. Additional definitions and explanations may be specifically provided in the context of these embodiments.

### Definitions

Adaptive immune response: The adaptive immune response is typically understood to be an antigen-specific response of the immune system. Antigen specificity allows for the generation of responses that are tailored, for example, to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naive T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells, which induces their proliferation and differentiation into armed effector T cells. The most important function of effector T cells is the killing of infected cells by CD8+ cytotoxic T cells and the activation of macrophages by Th1 cells, which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory B cells and Memory T cells that are the keys to long-lived specific immunity.

Artificial nucleic acid molecule: An artificial nucleic acid molecule may typically be understood to be a nucleic acid molecule, e.g. a DNA or an RNA, that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides, which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term "wild type" may be understood as a sequence occurring in nature. Further, the term "artificial nucleic acid molecule" is not restricted to mean "one single molecule" but is, typically, understood to comprise an ensemble of identical molecules. Accordingly, it may relate to a plurality of identical molecules contained in an aliquot.

Cellular immunity/cellular immune response:Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

DNA: DNA is the usual abbreviation for deoxy-ribonucleic acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerise by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA sequence. DNA may be single stranded or double stranded. In the double stranded form, the nucleotides of the first strand typically hybridize with the nucleotides of the second strand, e.g. by A/T-base-pairing and G/C-base-pairing.

Fragment of a sequence: A fragment of a sequence may typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

Heterologous sequence: Two sequences are typically understood to be "heterologous" if they are not derivable from the same gene. I.e., although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Immunogen: In the context of the present invention, an immunogen may be typically understood to be a compound that is able to stimulate an immune response. Preferably, an immunogen is a peptide, polypeptide, or protein. In a particularly preferred embodiment, an immunogen in the sense of the present invention is the product of translation of a provided nucleic acid molecule, preferably an artificial nucleic acid molecule as defined herein. Typically, an immunogen elicits at least an adaptive immune response.

immunostimulatory composition: In the context of the invention, an immunostimulatory composition may be typically understood to be a composition containing at least one component which is able to induce an immune response or from which a component, which is able to induce an immune response, is derivable. Such immune response may be preferably an innate immune response or a combination of an adaptive and an innate immune response. Preferably, an immunostimulatory composition in the context of the invention contains at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) in the context of the invention may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. The pharmaceutical composition according to the present invention may comprise one or more such substances. Typically, a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide monomers, which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets, which may be translated into a peptide or protein. An open reading frame preferably contains a start codon, i.e. a combination of three subsequent nucleotides coding usually for the amino acid methionine (ATG), at its 5'-end and a subsequent region, which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "(protein) coding sequence" or, preferably, "coding sequence".

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Protein A protein typically comprises one or more peptides or polypeptides. A protein is typically folded into 3-dimensional form, which may be required for the protein to exert its biological function.

Restriction site: A restriction site, also termed restriction enzyme recognition site, is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts both strands of the nucleotide sequence.

RNA, mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. In vivo, transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino-acid sequence of a particular peptide or protein. Typically, a mature mRNA comprises a 5'-cap, a 5'-UTR, an open reading frame, a 3'-UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an . alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term "transfection" refers to the introduction of nucleic acid molecules, such as DNA or RNA (e.g. mRNA) molecules, into cells, preferably into eukaryotic cells. In the context of the present invention, the term "transfection" encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vector: The term "vector" refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector, which allows the convenient storage of a nucleic acid molecule, for example, of an mRNA molecule. Thus, the vector may comprise a sequence corresponding, e.g., to a desired mRNA sequence or a part thereof, such as a sequence corresponding to the coding sequence and the 3'-UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA polymerase promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector, which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

Vehicle: A vehicle is typically understood to be a material that is suitable for storing, transporting, and/or administering a compound, such as a pharmaceutically active compound. For example, it may be a physiologically acceptable liquid, which is suitable for storing, transporting, and/or administering a pharmaceutically active compound.

The present invention is in part based on the surprising discovery that the combined administration of an RNA encoding a tumor antigen together with PD-1 and LAG-3 pathway inhibitors is capable of effectively boosting anti-tumoral immune responses. The combination of said RNA and both inhibitors did not only result in a significantly reduced tumor growth, but was also capable of effecting complete tumor eradication -whereas no complete tumor remission occurred upon administration of PD-1 and LAG-3 checkpoint inhibitors alone. Only the combination of RNA with PD-1 and LAG-3 blockade acted synergistically to induce a significant increase in survival -in comparison to the single treatments with unspecific RNA, single inhibitors or even the specific RNA. The combination of immune checkpoint inhibition of PD-1 and LAG-3 with therapeutic RNA vaccines thus represent an attractive treatment approach not only to improve anti-tumor immune response and clinical outcome, but also for combating infectious diseases and other diseases and conditions described herein.

In a first aspect, the present invention thus relates to a combination comprising: (i) at least one RNA, said RNA comprising at least one coding sequence encoding at least one epitope of an antigen; (ii) at least one PD-1 pathway inhibitor; and (iii) at least one LAG-3 pathway inhibitor.

The inventive combination thus comprises at least one RNA encoding at least one epitope. Said RNA may thus encode one or several epitopes, such as 2, 3, 4, 5, 6, 7, 8, 9, 10 or more epitopes, in at least one coding sequence (or "coding region") of said RNA. Thus, the RNA may comprise one coding region encoding 1, 2, 3, 4, 5, 6, 7, 8, 9 or 10 different epitopes. Alternatively, said RNA may comprise more than one coding region encoding more than one epitope. Said RNA is also referred to as an "epitope-encoding RNA" herein.

It will be understood that the term "RNA" refers to ribonucleic acid molecules characterized by the specific succession of their nucleotides joined to form said molecules (i.e. their RNA sequence). The term "RNA" may thus be used to refer to RNA molecules or RNA sequences as will be readily understood by the skilled person in the respective context. For instance, the term "at least one RNA" as used in the context of the inventive combination preferably refers to at least one RNA molecule present in said combination (said molecule being characterized, *inter alia,* by its particular RNA sequence). The term "RNA" in the context of sequence modifications will be understood to relate to modified RNA sequences, but typically also includes the resulting RNA molecules (which are modified with regard to their RNA sequence).

The term "epitope" or "antigenic determinant" typically refers to the part of an antigen which is recognized by the adaptive immune system. An "antigen" is a substance, which is capable of being recognized (typically via its epitope(s)) by the immune system, preferably by the adaptive immune system, and which is capable of eliciting an antigen-specific immune response, e.g. by formation of antibodies and/or antigen-specific T cells as part of an adaptive immune response. Typically, an antigen may be or may comprise a peptide or protein, which may be presented to (antigen-specific) T-cells on MHC surface molecules by antigen-presenting cells. In the context of the present invention, an antigen may be the product of translation of a provided nucleic acid molecule, preferably an epitope-encoding RNA as defined herein. In this context, also fragments or variants of an antigen (such as a peptide or a protein) comprising at least one epitope are understood as antigens.

As used herein, the term "epitope" in particular refers to a part or fragment of an antigen presented on a MHC surface molecule. Such a fragment comprising or consisting of an epitope as used herein may typically comprise from about 5 to about 20 amino acids. Epitopes can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC surface molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form. The term "epitope" includes "conformational" (or "discontinuous") epitopes, which are composed of discontinuous sequences of the amino acids of the antigen but are brought together in the three-dimensional structure, and "linear" epitopes, which are formed by a continuous sequence of amino acids from the antigen.

The "epitope-encoding" RNA of the inventive combination may encode a full-length (peptide or protein) antigen, or a variant or fragment thereof. Said full-length (peptide or protein) antigen, or variant or fragment thereof, comprises or consists of or provides at least one (functional) epitope, i.e. said antigenic peptide or protein (or its variant or fragment) preferably either comprises or consists of a native epitope (preferably recognized by B cells) or is processed and/or bound to provide a MHC-bound epitope (preferably recognized by T cells), said epitope preferably being functional, i.e. capable of inducing the desired adaptive immune response in a subject. Encoded antigens, variants or fragments thus can be of any length, as long as they comprise, consist of or provide at least one functional epitope, which is capable of inducing the desired adaptive immune response in a subject. An "epitope-encoding" RNA may thus encode at least one or more of (i) a full-length antigen sequence (or variant thereof) as defined herein, or (ii) a fragment of said antigen sequence (or variant thereof) as defined herein. Said antigen fragment may be a short stretch of amino acids forming a linear or conformational epitope, or may be a fragment that is processed and bound by an MHC surface molecule. Antigen fragments preferably comprise at least one "functional" epitope, i.e. which is capable of inducing the desired (adaptive) immune response.

### Full-length antigens

### Wild-type antigens

In preferred embodiments, the at least one coding sequence of the epitope-encoding RNA, in particular its RNA sequence, of the inventive combination may comprises a coding sequence encoding a "full-length" antigen as defined herein. The term "full-length antigen" as used herein typically refers to an antigen that substantially comprises the entire amino acid sequence of the naturally occuring (wild-type) antigen.

A naturally occuring (wild-type) antigen may be encoded by a naturally occuring (wild-type) nucleic acid sequence, in particular RNA sequence, or (due to the degeneracy of the genetic code) by a nucleic acid sequence "variant". Thus, in preferred embodiments, the epitope-encoding RNA of the inventive combination comprises a wild-type nucleic acid sequence or a nucleic acid sequence "variant" encoding a full-length, wild-type antigen as defined herein. According to preferred embodiments, said antigen is selected from the antigens listed in List 1 below.

### Variants

According to further preferred embodiments, the epitope-encoding RNA, in particular its RNA sequence, comprises at least one coding sequence encoding a variant of an antigen as defined herein.

Preferably, the sequence of an antigen "variant" or "sequence variant" differs in at least one amino acid residue from the amino acid sequence of the naturally occuring (wild-type) antigen serving as a reference (or "parent") sequence. Variant antigens thus preferably comprise at least one amino acid mutation, substitution, insertion or deletion as compared to their respective reference sequence. Preferably, the term "variant" as used herein comprises any homolog, isoform or transcript variant of a protein antigen as defined herein, wherein the homolog, isoform or transcript variant is preferably characterized by a degree of identity or homology, respectively, as defined herein.

An antigen "variant" encoded by the at least one coding sequence of the RNA of the inventive combination may comprise at least one amino acid substitution as compared to the wild-type (naturally occurring) antigen amino acid sequence. Said substitution may be selected from a conservative or non-conservative substitution. In some embodiments, it is preferred that a protein "variant" encoded by the at least one coding sequence of the epitope-encoding RNA comprises at least one conservative amino acid substitution, wherein amino acids, which originate from the same class, are exchanged for one another. In particular, these are amino acids having aliphatic side chains, positively or negatively charged side chains, aromatic groups in the side chains or amino acids, the side chains of which can form hydrogen bridges, e.g. side chains which have a hydroxyl function. By conservative constitution, e.g. an amino acid having a polar side chain may be replaced by another amino acid having a corresponding polar side chain, or, for example, an amino acid characterized by a hydrophobic side chain may be substituted by another amino acid having a corresponding hydrophobic side chain (e.g. serine (threonine) by threonine (serine) or leucine (isoleucine) by isoleucine (leucine)).

The "variant" may also comprise amino acid mutations, insertions, deletions and/or non-conservative substitutions, in particular, at those sequence positions, which do not impair the functionality of the epitope(s) of the encoded antigen(s).

Preferably, a "variant" of an antigen may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective naturally occuring (wild-type) antigen.

### Fragments

According to further preferred embodiments, the at least one coding sequence of the epitope-encoding RNA as defined herein may encode a fragment of an antigen (or a variant thereof). Said fragment can be of any length, provided that it preferably comprises at least one functional epitope.

In the context of the present invention, a "fragment" of an antigen (or a variant thereof) may comprise a sequence of an antigen (or a variant thereof) as defined above, which is, with regard to its amino acid sequence (or its encoding nucleic acid sequence), N-terminally, C-terminally and/or intrasequentially truncated compared to the amino acid sequence of the naturally occuring antigen or a variant thereof (or its encoding nucleic acid sequence). Such truncation may thus occur either on the amino acid level or on the nucleic acid level, respectively. A sequence identity with respect to such a fragment as defined herein therefore preferably refers to the entire antigen (or a variant thereof) as defined herein or to the entire (coding) nucleic acid sequence of such an antigen (or a variant thereof).

A "fragment" of antigen (or a variant thereof) may comprise or consist of an amino acid sequence of said antigen (or a variant thereof) as defined herein, having a length of about 5 to about 20 or even more amino acids and which is preferably processed and presented by an MHC complex. Preferably, a fragment of an antigen (or a variant thereof) may comprise or consist of an amino acid sequence of said antigen (or a variant thereof) as defined herein, which has a length of about 6 to about 20 or even more amino acids, e.g. a fragment as processed and presented by MHC class I molecules, preferably having a length of about 8 to about 10 amino acids, e.g. 8, 9, or 10, (or even 6, 7, 11, or 12 amino acids), or a fragment as processed and presented by MHC class II molecules, preferably having a length of about 13 or more amino acids, e.g. 13, 14, 15, 16, 17, 18, 19, 20 or even more amino acids, wherein the fragment may be selected from any part of the amino acid sequence. These fragments are typically recognized by T-cells in the form of a complex consisting of the peptide fragment and an MHC molecule, i.e. the fragments are typically not recognized in their "native" or "free" form, but rather in MHC-bound form.

Preferably, a ""fragment" of an antigen (or a variant thereof) encoded by the at least one coding sequence of the epitope-encoding RNA of the inventive combination may typically comprise or consist of an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective full-length wild-type antigen (or variant thereof).

It is envisaged that the at least one antigen "fragment" encoded by the epitope-encoding RNA may be a (N-terminally, C-terminally and/or intrasequentially) truncated fragment of (a) a wild-type antigen or (b) an antigen variant as defined herein. The term "fragment" may however also include "fragment variants" comprising or consisting of an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97% to a fragment of (a) a wild-type antigen or (b) an antigen variant as defined herein.

### Antigens

### Tumor antigens

The present inventors surprisingly discovered that an RNA encoding a tumor antigen was capable of providing expression of the tumor antigen *in vivo.* Unexpectedly, provision of said RNA as a vaccine in combination with PD-1 and LAG-3 pathway inhibitors was capable of inducing an improved anti-tumor immune response.

In preferred embodiments, the epitope encoded by the at least one coding sequence of the RNA is an epitope of a tumor antigen as defined herein, which is derived from or associated with a tumor or a cancer disease. Preferably, the tumor is a malignant tumor. The tumor antigen is preferably an antigen associated with a cancer disease. A tumor antigen as used herein is typically derived from a tumor cell, preferably a mammalian tumor cell. A tumor antigen is preferably located in or on the surface of a tumor cell derived from a mammalian, preferably from a human, tumor, such as a systemic or a solid tumor.

Tumor antigens may also be selected from proteins, which are overexpressed in tumor cells compared to a normal cell. Furthermore, the expression "tumor antigen" also includes an antigen expressed in cells, which are (were) not themselves (or originally not themselves) degenerated but are associated with the supposed tumor. Antigens, which are connected with tumor-supplying vessels or (re)formation thereof, in particular those antigens, which are associated with neovascularization, e.g. growth factors, such as VEGF, bFGF etc., are also included herein. Further antigens connected with a tumor include antigens from cells or tissues typically embedding the tumor. Further, some substances (usually proteins or peptides) are expressed in patients suffering (knowingly or not-knowingly) from a cancer disease and they occur in increased concentrations in the body fluids of said patients. Some substances may also referred to herein as "tumor antigens", without being antigens in the strictest sense of an immune response inducing substance. The class of tumor antigens can be divided further into tumor-specific antigens (TSAs) and tumor-associated-antigens (TAAs), both of which are comprised by the expression as used herein. TSAs can only be presented by tumor cells and never by normal "healthy" cells. They typically result from a tumor specific mutation. TAAs, which are more common, are usually presented by both tumor and healthy cells. These antigens are recognized and the antigen-presenting cell can be destroyed by cytotoxic T cells. Additionally, tumor antigens can also occur on the surface of the tumor in the form of, e.g., a mutated receptor. In this case, they can be recognized by antibodies.

As used herein, the term "tumor antigen" preferably refers to any one of the tumor antigens provided in List 1 below. The at least one coding sequence of the epitope-encoding RNA of the inventive combination thus preferably encodes an epitope derived from a tumor antigen selected from the antigens provided in List 1, or a fragment or variant thereof.

In this context, it is further preferred that the at least one coding sequence of the epitope-encoding RNA encodes a tumor antigen, or a fragment or variant thereof, wherein the tumor antigen is an antigen selected from the antigens listed in List 1.

**List 1: List of tumor antigens (Gene Name (Protein Accession No. of UniProt, RefSeq or Genbank)):**
1A01_HLA-A/m (P30443); 1A02 (P01892); 5T4 (Q13641); ACRBP (Q8NEB7); AFP (P02771); AKAP4 (Q5JQC9); alpha-actinin-_4/m (B4DSX0); alpha-actinin-_4/m (B4E337); alpha-actinin-_4/m (O43707); alpha-methylacyl-coenzyme_A_racemase (A0A024RE16); alpha-methylacyl-coenzyme_A_racemase (A8KAC3); ANDR (P10275); ART-4 (Q9ULX3); ARTC1/m (P52961); AURKB (Q96GD4); B2MG (P61769); B3GN5 (Q9BYG0); B4GN1 (Q00973); B7H4 (Q7Z7D3); BAGE-1 (Q13072); BASI (P35613); BCL-2 (A9QXG9); bcr/abl (A9UEZ4); bcr/abl (A9UEZ7); bcr/abl (A9UEZ8); bcr/abl (A9UEZ9); bcr/abl (A9UF00); bcr/abl (A9UF01); bcr/abl (A9UF03); bcr/abl (A9UF04); bcr/abl (A9UF05); bcr/abl (A9UF06); bcr/abl (A9UF08); beta-catenin/m (P35222); beta-catenin/m (Q8WYA6); BING-4 (015213); BIRC7 (Q96CA5); BRCA1/m (A0A024R1V0); BRCA1/m (A0A024R1V7); BRCA1/m (A0A024R1Z8); BRCA1/m (A0A068BFX7); BRCA1/m (C6YB45); BRCA1/m (C6YB47); BRCA1/m (G3XAC3); BY55 (095971); calreticulin (B4DHR1); calreticulin (B4E2Y9); calreticulin (P27797); calreticulin (Q96L12); CAMEL (O95987); CASP-8/m (Q14790); CASPA (Q92851-4); cathepsin_B (A0A024R374); cathepsin_B (P07858); cathepsin_L (A0A024R276); cathepsin_L (P07711); cathepsin_L (Q9HBQ7); CD1A (P06126); CD1B (P29016); CD1C (P29017); CD1D (P15813); CD1E (P15812); CD20 (P11836); CD22 (O60926); CD22 (P20273); CD22 (Q0EAF5); CD276 (Q5ZPR3); CD33 (B4DF51); CD33 (P20138); CD33 (Q546G0); CD3E (P07766); CD3Z (P20963); CD44_Isoform_1 (P16070); CD44_lsoform_6 (P16070-6); CD4 (P01730); CD52 (P31358); CD52 (Q6IBD0); CD52 (V9HWN9); CD55 (B1AP15); CD55 (D3DT85); CD55 (D3DT86); CD55 (P08174); CD56 (P13591); CD80 (A0N0P2); CD80 (P33681); CD86 (P42081); CD8A (P01732); CDC27/m (G5EA36); CDC27/m (P30260); CDE30 (P28908); CDK4/m (A0A024RBB6); CDK4/m (P11802); CDK4/m (Q6LC83); CDK4/m (Q96BE9); CDKN2A/m (D1LYX3); CDKN2A/m (G3XAG3); CDKN2A/m (K7PML8); CDKN2A/m (L8E941); CDKN2A/m (Q8N726); CEA (NP_004354); CEAM6 (P40199); CH3L2 (Q15782); CLCA2 (Q9UQC9); CML28 (Q9NQT4); CML66 (Q96RS6); COA-1/m (Q5T124); coactosin-like_protein (Q14019); collagen_XXIII (L8EAS4); collagen_XXIII (Q86Y22); COX-2 (Q6ZYK7); CP1B1 (Q16678); CSAG2 (Q9Y5P2-2); CSAG2 (Q9Y5P2); CT45A1 (Q5HYN5); CT55 (Q8WUE5); CT-_9/BRD6 (Q58F21); CTAG2_lsoform_LAGE-1A (O75638-2); CTAG2_lsoform_LAGE-1B (O75638); CTCFL (Q8NI51); Cten (Q8IZW8); cyclin_B1 (P14635); cyclin_D1 (P24385); cyp-B (P23284); DAM-10 (P43366); DEP1A (Q5TB30); E7 (P03129); E7 (P06788); E7 (P17387); E7 (P06429); E7 (P27230); E7 (P24837); E7 (P21736); E7 (P26558); E7 (P36831); E7 (P36833); E7 (Q9QCZ1); E7 (081965); E7 (Q80956); EF1A2 (Q05639); EFTUD2/m (Q15029); EGFR (A0A0B4J1Y5); EGFR (E7BSV0); EGFR (L0R6G1); EGFR (P00533-2); EGFR (P00533); EGFR (Q147T7); EGFR (Q504U8); EGFR (Q8NDU8); EGLN3 (Q9H6Z9); ELF2/m (B7Z720); EMMPRIN (Q54A51); EpCam (P16422); EphA2 (P29317); EphA3 (P29320); EphA3 (Q6P4R6); ErbB3 (B3KWG5); ErbB3 (B4DGQ7); ERBB4 (Q15303); ERG (P11308); ETV6 (P41212); EWS (Q01844); EZH2 (F2YMM1); EZH2 (G3XAL2); EZH2 (L0R855); EZH2 (Q15910); EZH2 (S4S3R8); FABP7 (015540); FCGR3A (P08637); FGF5 (P12034); FGF5 (Q60518); FGFR2 (P21802); fibronectin (A0A024R5I6); fibronectin (A0A024RB01); fibronectin (A0A024RDT9); fibronectin (A0A024RDV5); fibronectin (A6NH44); fibronectin (A8K6A5); fibronectin (B2R627); fibronectin (B3KXM5); fibronectin (B4DIC5); fibronectin (B4DN21); fibronectin (B4DS98); fibronectin (B4DTH2); fibronectin (B4DTK1); fibronectin (B4DU16); fibronectin (B7Z3W5); fibronectin (B7Z939); fibronectin (G5E9X3); fibronectin (Q9H382); FOS (P01100); FOXP3 (Q9BZS1); FUT1 (P19526); G250 (Q16790); GAGE-1 (AAA82744); GAGE-2 (Q6NT46); GAGE-3 (Q13067); GAGE-4 (Q13068); GAGE-5 (Q13069); GAGE-6 (Q13070); GAGE7b (O76087); GAGE-8_GAGE-2D (Q9UEU5); GASR (P32239); GnT-V (Q09328); GPC3 (I6QTG3); GPC3 (P51654); GPC3 (Q8IYG2); GPNMB/m (A0A024RA55); GPNMB/m (Q14956); GPNMB/m (Q8IXJ5); GPNMB/m (Q96F58); GRM3 (Q14832); HAGE (Q9NXZ2); hepsin (B2ZDQ2); hepsin (P05981); Her2/neu (B4DTR1); Her2/neu (L8E8G2); Her2/neu (P04626); Her2/neu (Q9UK79); HLA-A2/m (Q95387); HLA-A2/m (Q9MYF8); homeobox_NKX3.1 (Q99801); HOM-TES-85 (B2RBQ6); HOM-TES-85 (Q9P127); HPG1 Pubmed: 12543784); HS71A (P0DMV8); HS71B (P0DMV9); HST-2 (P10767); hTERT (O94807); iCE (O00748); IF2B3 (O00425); IL10 (P22301); IL-13Ra2 (Q14627); IL2-RA (P01589); IL2-RB (P14784); IL2-RG (P31785); IL-5 (P05113); IMP3 (Q9NV31); ITA5 (P08648); ITB1 (P05556); ITB6 (P18564); kallikrein-2 (A0A024R4J4); kallikrein-2 (A0A024R4N3); kallikrein-2 (B0AZU9); kallikrein-2 (B4DU77); kallikrein-2 (P20151); kallikrein-2 (Q6T774); kallikrein-2 (Q6T775); kallikrein-4 (A0A0C4DFQ5); kallikrein-4 (Q5BQA0); kallikrein-4 (Q96PT0); kallikrein-4 (Q96PT1); kallikrein-4 (Q9Y5K2); KI20A (O95235); KIAA0205 (Q92604); KIF2C (Q99661); KK-LC-1 (Q5H943); LDLR (P01130); LGMN (Q99538); LIRB2 (Q8N423); LY6K (Q17RY6); MAGA5 (P43359); MAGA8 (P43361); MAGAB (P43364); MAGE-A10 (A0A024RC14); MAGE-A12 (P43365); MAGE-A1 (P43355); MAGE-A2 (P43356); MAGE-A3 (P43357); MAGE-A4 (A0A024RC12); MAGE-A4 (P43358); MAGE-A4 (Q1RN33); MAGE-A6 (A8K072); MAGE-A6 (P43360); MAGE-A6 (Q6FHI5); MAGE-A9 (P43362); MAGE-B10 (Q96LZ2); MAGE-B16 (A2A368); MAGE-B17 (A8MXT2); MAGE-_B1 (Q96TG1); MAGE-B2 (015479); MAGE-B3 (015480); MAGE-B4 (015481); MAGE-B5 (Q9BZ81); MAGE-B6 (Q8N7X4); MAGE-C1 (O60732); MAGE-C2 (Q9UBF1); MAGE-C3 (Q8TD91); MAGE-D1 (Q9Y5V3); MAGE-D2 (Q9UNF1); MAGE-D4 (Q96JG8); MAGE-_E1 (Q6IAI7); MAGE-E1_(MAGE1) (Q9HCl5); MAGE-E2 (Q8TD90); MAGE-F1 (Q9HAY2); MAGE-H1 (Q9H213); MAGEL2 (Q9UJ55); mammaglobin_A (Q13296); mammaglobin_A (Q6NX70); MART-1/melan-A (Q16655); MART-2 (Q5VTY9); MC1_R (Q01726); MC1_R (Q1JUL4); MC1_R (Q1JUL6); MC1_R (Q1JUL8); MC1_R (Q1JUL9); MC1_R (Q1JUM0); MC1_R (Q1JUM2); MC1_R (Q1JUM3); MC1_R (Q1JUM4); MC1_R (Q1JUM5); MC1_R (Q6UR92); MC1_R (Q6UR94); MC1_R (Q6UR95); MC1_R (Q6UR96); MC1_R (Q6UR97); MC1_R (Q6UR98); MC1_R (Q6UR99); MC1_R (Q6URA0); MC1_R (Q86YW1); MC1_R (V9Q5S2); MC1_R (V9Q671); MC1_R (V9Q783); MC1_R (V9Q7F1); MC1_R (V9Q8N1); MC1_R (V9Q977); MC1_R (V9Q9P5); MC1_R (V9Q9R8); MC1_R (V9QAE0); MC1_R (V9QAR2); MC1_R (V9QAW3); MC1_R (V9QB02); MC1_R (V9QB58); MC1_R (V9QBY6); MC1_R (V9QC17); MC1_R (V9QC66); MC1_R (V9QCQ4); MC1_R (V9QDF4); MC1_R (V9QDN7); MC1_R (V9QDQ6); M-CSF (P09603); mesothelin (Q13421); MITF (O75030-8); MITF (O75030-9); MITF (O75030); MMP1_1 (B3KQS8); MMP7 (P09237); MUC-1 (AAA60019); MUM-1/m (NP_116242); MUM-2/m (Q9Y5R8); MYCN (P04198); MYO1A (Q9UBC5); MYO1B (O43795); MYO1C (000159); MYO1D (O94832); MYO1E (Q12965); MYO1F (000160); MYO1G (B0I1T2); MYO1H (NP_001094891); NA17 (Q3V5L5); NA88-A Pubmed: 10790436); Neo-PAP (Q9BWT3); NFYC/m (Q13952); NGEP (Q6IWH7); NPM (P06748); NRCAM (Q92823); NSE (P09104); NUF2 (Q9BZD4); NY-ESO-1 (P78358); OA1 (P51810); OGT (015294); OS-9 (B4DH11); OS-9 (B4E321); OS-9 (B7Z8E7); OS-9 (Q13438); osteocalcin (P02818); osteopontin (A0A024RDE2); osteopontin (A0A024RDE6); osteopontin (A0A024RDJ0); osteopontin (B7Z351); osteopontin (F2YQ21); osteopontin (P10451); p53 (P04637); PAGE-4 (O60829); PAI-1 (P05121); PAI-2 (P05120); PAP (Q06141); PAP (Q53S56); PATE (Q8WXA2); PAX3 (P23760); PAX5 (Q02548); PD1L1 (Q9NZQ7); PDCD1 (Q15116); PDEF (O95238); PECA1 (P16284); PGCB (Q96GW7); PGFRB (P09619); Pim-1_-Kinase (A0A024RD25); Pin-1 (015428); Pin-1 (Q13526); Pin-1 (Q49AR7); PLAC1 (Q9HBJ0); PMEL (P40967); PML (P29590); POTEF (A5A3E0); POTE (Q86YR6); PRAME (A0A024R1E6); PRAME (P78395); PRDX5/m (P30044); PRM2 (P04554); prostein (Q96JT2); proteinase-3 (D6CHE9); proteinase-3 (P24158); PSA (P55786); PSB9 (P28065); PSCA (D3DWI6); PSCA (O43653); PSGR (Q9H255); PSM (Q04609); PTPRC (NP_002829); RAB8A (P61006); RAGE-1 (Q9UQ07); RARA (P10276); RASH (P01112); RASK (P01116); RASN (P01111); RGS5 (015539); RHAMM/CD168 (O75330); RHOC (P08134); RSSA (P08865); RU1 (Q9UHJ3); RU2 (Q9UHG0); RUNX1 (Q01196); S-100 (V9HW39); SAGE (Q9NXZ1); SART-_1 (O43290); SART-2 (Q9UL01); SART-3 (Q15020); SEPR (Q12884); SERPINB5 (P36952); SIA7F (Q969X2); SIA8A (Q92185); SIAT9 (Q9UNP4); SIRT2/m (A0A024R0G8); SIRT2/m (Q8IXJ6); SOX10 (P56693); SP17 (Q15506); SPNXA (Q9NS26); SPXN3 (Q5MJ09); SSX-1 (Q16384); SSX-2 (Q16385); SSX3 (Q99909); SSX-4 (O60224); ST1A1 (P50225); STAG2 (Q8N3U4-2); STAMP-1 (Q8NFT2); STEAP-1 (A0A024RA63); STEAP-1 (Q9UHE8); Survivin-2B (015392-2); survivin (015392); SYCP1 (A0A024R0I2); SYCP1 (B7ZLS9); SYCP1 (Q15431); SYCP1 (Q3MHC4); SYT-SSX-1 (A4PIV7); SYT-SSX-1 (A4PIV8); SYT-SSX-2 (A4PIV9); SYT-SSX-2 (A4PIW0); TARP (Q0VGM3); TCRg (A2JGV3); TF2AA (P52655); TGFB1 (P01137); TGFR2 (P37173); TGM-4 (B2R7D1); TIE2 (Q02763); TKTL1 (P51854); TPI/m (P60174); TRGV11 (Q99601); TRGV9 (A4D1X2); TRGV9 (Q99603); TRGV9 (Q99604); TRPC1 (P48995); TRP-p8 (Q7Z2W7); TSG10 (Q9BZW7); TSPY1 (Q01534); TVC_TRGV3 (M13231.1); TX101 (Q9BY14-2); tyrosinase (A0A024DBG7); tyrosinase (L8B082); tyrosinase (L8B086); tyrosinase (L8B0B9); tyrosinase (O75767); tyrosinase (P14679); tyrosinase (U3M8N0); tyrosinase (U3M9D5); tyrosinase (U3M9J2); TYRP1 (P17643); TYRP2 (P40126); UPA (Q96NZ9); VEGFR1 (B5A924); WT1 (A0A0H5AUY0); WT1 (P19544); WT1 (Q06250) and XAGE1 (Q9HD64).

### Full-length and wild-type tumor antigens

According to preferred embodiments, the at least one coding sequence of the epitope-encoding RNA of the inventive combination encodes a full-length tumor antigen (and thus at least one epitope comprised by the same), wherein the tumor antigen is an antigen selected from the antigens listed in List 1, preferably an antigen selected from the antigens defined by the database accession number provided under the respective column in List 1.

Accordingly, the at least one coding sequence of the epitope-encoding RNA of the inventive combination may preferably comprise or consist of a wild-type nucleic acid sequence encoding a full-length tumor antigen (and thus at least one epitope comprised by the same), wherein the tumor antigen is an antigen selected from the antigens listed in List 1, preferably a tumor antigen selected from the antigens defined by the database accession number provided under the respective column in List 1.

Alternatively, the at least one coding sequence of the epitope-encoding RNA of the inventive combination comprise or consist of a nucleic acid sequence "variant" which differs from the respective naturally occuring (wild-type) nucleic acid sequence in at least one nucleic acid residue, preferably without resulting (due to the degenerated genetic code) in an alteration of the encoded amino acid sequence. Thus, nucleic acid sequence "variants" may encode full-length, wild-type tumor antigens. According to preferred embodiments, said "variant" nucleic acid sequence encoding a full-length, wild-type tumor antigen comprises or consists of a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97% with a wild-type nucleic acid sequence encoding a tumor antigen as defined in List 1, preferably an antigen selected from the antigens defined by the database accession number provided under the respective column in List 1, or a variant or fragment thereof.

### Tumor antigen variants

According to further preferred embodiments, the at least one coding sequence of the epitope-encoding RNA of the inventive combination encodes a variant of a tumor antigen (and thus at least one epitope comprised by the same), wherein the tumor antigen is an antigen selected from the antigens listed in List 1, preferably an antigen selected from the antigens defined by the database accession number provided under the respective column in List 1. A "variant" of said tumor antigen may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective naturally occuring (wild type) full-length tumor antigen, wherein the tumor antigen is an antigen selected from the antigens listed in List 1, preferably an antigen selected from the antigens defined by the database accession number provided under the respective column in List 1.

Accordingly, the at least one coding sequence of the epitope-encoding RNA of the inventive combination may preferably comprise or consist of a nucleic acid sequence "variant" encoding a variant of a tumor antigen (and at least one epitope comprised by the same), wherein the tumor antigen is an antigen selected from the antigens listed in List 1, preferably an antigen selected from the antigens defined by the database accession number provided under the respective column in List 1. According to preferred embodiments, said nucleic acid sequence "variant" comprises or consists of a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97% with a wild-type nucleic acid sequence encoding a tumor antigen as defined in List 1, preferably an antigen selected from the antigens defined by the database accession number provided under the respective column in List 1, or a variant or fragment thereof.

### Tumor antigen fragments

According to further preferred embodiments, the at least one coding sequence of the RNA of the inventive combination encodes a fragment of a tumor antigen (or a variant thereof) preferably comprising at least one epitope of said tumor antigen (or variant thereof), wherein the tumor antigen is an antigen selected from the antigens listed in List 1, preferably an antigen selected from the antigens defined by the database accession number in List 1, or a variant thereof. A fragment of said tumor antigen (or variant thereof) may typically comprise or consist of an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective full-length tumor antigen (or variant thereof), wherein the tumor antigen is an antigen selected from the antigens listed in List 1, preferably an antigen selected from the antigens defined by the database accession number in List 1.

Accordingly, the at least one coding sequence of the epitope-encoding RNA of the inventive combination may preferably comprise or consist of a nucleic acid sequence "fragment" encoding a fragment of a tumor antigen (and at least one epitope comprised by the same), wherein the tumor antigen is an antigen selected from the antigens listed in List 1, preferably an antigen selected from the antigens defined by the database accession number provided in List 1. According to preferred embodiments, said nucleic acid sequence "fragment" comprises or consists of a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97% with a nucleic acid sequence encoding a full-length tumor antigen as defined in List 1 (or a variant thereof), preferably an antigen selected from the antigens defined by the database accession number provided in List 1 (or a variant thereof).

According to preferred embodiments, the at least one coding sequence of the epitope-encoding RNA thus encodes a tumor antigen (or variant thereof), or a fragment of said tumor antigen (or variant thereof), wherein the tumor antigen is preferably selected from the group consisting of 1A01_HLA-A/m; 1A02; 5T4; ACRBP; AFP; AKAP4; alpha-actinin-_4/m; alpha-methylacyl-coenzyme_A_racemase; ANDR; ART-4; ARTC1/m; AURKB; B2MG; B3GN5; B4GN1; B7H4; BAGE-1; BASI; BCL-2; bcr/abl; beta-catenin/m; BING-4; BIRC7; BRCA1/m; BY55; calreticulin; CAMEL; CASP-8/m; CASPA; cathepsin_B; cathepsin_L; CD1A; CD1B; CD1C; CD1D; CD1E; CD20; CD22; CD276; CD33; CD3E; CD3Z; CD44_lsoform_1; CD44_lsoform_6; CD4; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen_XXIII; COX-2; CP1B1; CSAG2; CT45A1; CT55; CT-_9/BRD6; CTAG2_lsoform_LAGE-1A; CTAG2_Isoform_LAGE-1B; CTCFL; Cten; cyclin_B1; cyclin_D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_Version_1; FCGR3A_Version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8_(GAGE-2D); GASR; GnT-V; GPC3; GPNMB/m; GRM3; HAGE; hepsin; Her2/neu; HLA-A2/m; homeobox_NKX3.1; HOM-TES-85; HPG1; HS71A; HS71B; HST-2; hTERT; iCE; IF2B3; IL10; IL-13Ra2; IL2-RA; IL2-RB; IL2-RG; IL-5; IMP3; ITA5; ITB1; ITB6; kallikrein-2; kallikrein-4; KI20A; KIAA0205; KIF2C; KK-LC-1; LDLR; LGMN; LIRB2; LY6K; MAGA5; MAGA8; MAGAB; MAGE-A10; MAGE-A12; MAGE-A1; MAGE-A2; MAGE-A3; MAGE-A4; MAGE-A6; MAGE-A9; MAGE-B10; MAGE-B16; MAGE-B17; MAGE-_B1; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-_E1; MAGE-E1_(MAGE1); MAGE-E2; MAGE-F1; MAGE-H1; MAGEL2; mammaglobin_A; MART-1/melan-A; MART-2; MC1_R; M-CSF; mesothelin; MITF; MMP1_1; MMP7; MUC-1; MUM-1/m; MUM-2/m; MYCN; MYO1A; MYO1B; MYO1C; MYO1D; MYO1E; MYO1F; MYO1G; MYO1H; NA17; NA88-A; Neo-PAP; NFYC/m; NGEP; NPM; NRCAM; NSE; NUF2; NY-ESO-1; OA1; OGT; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1_-Kinase; Pin-1; PLAC1; PMEL; PML; POTEF; POTE; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-_1; SART-2; SART-3; SEPR; SERPINB5; SIA7F; SIA8A; SIAT9; SIRT2/m; SOX10; SP17; SPNXA; SPXN3; SSX-1; SSX-2; SSX3; SSX-4; ST1A1; STAG2; STAMP-1; STEAP-1; Survivin-2B; survivin; SYCP1; SYT-SSX-1; SYT-SSX-2; TARP; TCRg; TF2AA; TGFB1; TGFR2; TGM-4; TIE2; TKTL1; TPI/m; TRGV11; TRGV9; TRPC1; TRP-p8; TSG10; TSPY1; TVC_(TRGV3); TX101; tyrosinase; TYRP1; TYRP2; UPA; VEGFR1; WT1; and XAGE1. Said tumor antigen or variant or fragment thereof preferably comprises at least one functional epitope.

Further useful antigens, variants, fragments and derivatives thereof, and RNAs encoding the same as well as compositions comprising said RNAs are disclosed in WO2013120500 and WO2013120626 which are incorporated by reference herein in its entirety, and said RNAs and compositions are equally envisaged as part of the inventive combination.

Suitable tumor antigens or variants or fragments thereof in the sense of the present invention are the nucleic acid sequences according to SEQ ID NOs: 505-4033; 4561-4591 of the patent application WO2017182634, SEQ ID NOs: 1 - 26 of the patent application WO2009046974, and SEQ ID NOs: 505-4033 of the patent application WO2017182634 which are included herewith by reference.

### Combinations of tumor antigens

According to further preferred embodiments, the inventive combination comprises a plurality or more than one, preferably 2 to 20, more preferably 2 to 20, most preferably 2 to 6 of epitope-encoding RNAs as defined herein. Said combinations typically comprise more than one epitope-encoding RNAs, preferably encoding different peptides or proteins which comprise or provide preferably different epitopes, particularly of different tumor antigens, or pathogenic antigens.

According to particularly preferred embodiments, the inventive combination comprises at least one RNA coding for the tumour antigens selected from NY-ESO-1, MAGE-C1, MAGE-C2, Survivin, (optionally) 5T4 and (optionally) MUC-1, or variants or fragments of any of the aforementioned tumor antigens.

In this context, it is particularly preferred that the inventive combination (specifically when envisaged for use in the treatment of lung cancer, particularly non-small lung cancer (NSCLC)), comprises a plurality (typically at least 1, 2, 3, 4, 5, 6 or more than 10, e.g. 2 to 10, preferably 2 to 6), preferably six epitope-encoding RNAs, said epitope-encoding RNAs preferably being monocistronic and encoding
a) at least one epitope of NY-ESO-1, or a fragment, variant or derivative thereof; and
d) at least one epitope of MAGE-C1, or a fragment, variant or derivative thereof; and
e) at least one epitope of MAGE-C2, or a fragment, variant or derivative thereof; and;
f) at least one epitope of Survivin, or a fragment, variant or derivative thereof; and; and optionally
g) at least one epitope of 5T4, or a fragment, variant or derivative thereof; and; and optionally
h) at least one epitope of MUC-1, or a fragment, variant or derivative thereof; and; and optionally

In a particularly preferred embodiment the combination comprises at least one RNA, preferably at least two RNAs, more preferably at least three RNAs, even more preferably at least four RNAs, even more preferably at least five RNAs, or even more preferably at least six RNAs, each comprising at least one coding sequence selected from RNA sequences comprising or consisting of an RNA sequence of SEQ ID NOs: 1, 2, 3, 4, 5 or 6 (SEQ ID NOs: 19, 20, 21, 22, 23 and 24 of PCT/EP2014/002299 ) or a RNA sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97% to SEQ ID NOs: 1, 2, 3, 4, 5 or 6 (SEQ ID NOs: 19, 20, 21, 22, 23 and 24 of PCT/EP2014/002299). Further useful antigens, variants, fragments and derivatives thereof, and RNAs encoding the same as well as compositions comprising said RNAs are disclosed in PCT/EP2008/008503 (published under WO 2009/046974 A2) and PCT/EP2014/002299 (published under WO 2015/024666 A1) which are incorporated by reference herein in its entirety, and said RNAs and compositions are equally envisaged as part of the inventive combination.

In the following, various embodiments, designs and modifications of the epitope-encoding RNA of the inventive combination are described. Said epitope-encoding RNA may for instance be (a) mono-, bi- or multicistronic, (b) modified as regards their chemistry and/or sequence, and/or (c) may comprise various structural elements such as 5' Caps, Poly(A) sequences or signals, Poly(C) sequences, UTRs, histone stem loops, signal peptides, etc.

It will be noted that the PD-1 and/or the LAG-3 inhibitor of the inventive combination may be provided in the form of a nucleic acid, e.g. an RNA encoding said PD-1 and/or LAG-3 inhibitor (for instance an antibody, fusion protein or soluble receptor). Such nucleic acids, in particular RNAs encoding PD-1 and/or LAG-3 inhibitors may comprise the same modifications as described in the context of the epitope-encoding RNA herein. That is, such "inhibitor-encoding" nucleic acids, and in particular RNAs, may be (a) mono-, bi- or multicistronic, (b) modified as regards their chemistry and/or sequence and/or (c) may comprise various structural elements as described herein, such as 5' Caps, Poly(A) sequences or signals, Poly(C) sequences, UTRs, histone stem loops, signal peptides, etc. That is, the following disclosure regarding modifications/designs according to (a)-(c) described below in the context of epitope-encoding RNAs is equally applicable to nucleic acids, and in particular RNAs, encoding PD-1 and/or LAG-3 inhibitors, *mutatis mutandis.*

### Mono-, bi- or multicistronic RNAs

According to some embodiments of the present invention, the epitope-encoding RNA is mono-, bi-, or multicistronic, preferably as defined herein. Bi- or multicistronic RNAs typically comprise two (bicistronic) or more (multicistronic) open reading frames (ORF). An open reading frame in this context is a sequence of codons that is translatable into a peptide or protein. The coding sequences in a bi- or multicistronic epitope-encoding RNA preferably encode distinct epitopes (or antigens or variants or fragments thereof comprising said epitopes) as defined herein. Bi- or even multicistronic epitope-encoding RNAs, may encode, for example, at least two, three, four, five, six or more (preferably different) epitopes (or antigens or variants or fragments thereof comprising said epitopes) as defined herein . The term "encoding two or more epitopes/pathway inhibitors" may mean, without being limited thereto, that the bi- or even multicistronic epitope-encoding RNA, may encode e.g. at least two, three, four, five, six or more (preferably different) epitopes (or antigens or variants or fragments thereof comprising said epitopes).

In some embodiments, the coding sequences encoding two or more epitopes (or antigens or variants or fragments thereof comprising said epitopes) may be separated in the bi- or multicistronic RNA by at least one IRES (internal ribosomal entry site) sequence. The term "IRES" (internal ribosomal entry site) refers to an RNA sequence that allows for translation initiation. An IRES can function as a sole ribosome binding site, but it can also serve to provide a bi- or even multicistronic epitope-encoding RNA as defined above, which encodes several epitopes (or antigens or variants or fragments thereof comprising said epitopes), which are to be translated by the ribosomes independently of one another. Examples of IRES sequences, which can be used according to the invention, are those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukoma virus (MLV), simian immunodeficiency viruses (SIV) or cricket paralysis viruses (CrPV).

According to further embodiments the at least one coding sequence of the epitope-encoding RNA of the inventive combination may encode at least two, three, four, five, six, seven, eight and more epitopes (or antigens or variants or fragments thereof comprising said epitopes) as defined herein linked with or without an amino acid linker sequence, wherein said linker sequence can comprise rigid linkers, flexible linkers, cleavable linkers (e.g., self-cleaving peptides) or a combination thereof. Therein, epitopes (or antigens or variants or fragments thereof comprising said epitopes) may be identical or different or a combination thereof.

Preferably, the epitope-encoding RNA comprises a length of about 50 to about 20000, or 100 to about 20000 nucleotides, preferably of about 250 to about 20000 nucleotides, more preferably of about 500 to about 10000, even more preferably of about 500 to about 5000.

The epitope-encoding RNA of the inventive combination may further be single stranded or double stranded. When provided as a double stranded RNA, the epitope-encoding RNA of the inventive combination preferably comprises a sense and a corresponding antisense strand.

In preferred embodiments, the epitope-encoding RNA of the inventive combination is an mRNA, a viral RNA or a replicon RNA, preferably a mRNA.

### RNA Modifications

The nucleic acids defined herein, in particular the at least one epitope-encoding RNA of the inventive combination, or any other nucleic acid defined herein, may be provided in the form of modified nucleic acids. Suitable nucleic acid modifications envisaged in the context of the present invention are described below. As indicated above, the expression "any other nucleic acid as defined herein" particularly refers to nucleic acids, specifically RNAs, disclosed herein as encoding PD-1 or LAG-3 pathway inhibitors (e.g. antibodies, antagonistic binding proteins, peptides, fusion proteins, soluble receptors). The expression may, but typically does not, refer to antagonistic nucleic acids as disclosed herein.

According to preferred embodiments, the at least one epitope-encoding RNA (sequence) of the inventive combination (or any other nucleic acid, in particular RNA, as defined herein), is modified as defined herein. In this context, a modification as defined herein preferably leads to a stabilization of said RNA or other nucleic acids as defined herein. More preferably, the invention thus provides the inventive combination comprising a stabilized epitope-encoding RNA (or any other nucleic acid, in particular RNA, as defined herein).

According to preferred embodiments, the epitope-encoding RNA of the inventive combination (or any other nucleic acid, in particular RNA, as defined herein) may thus be provided as a "stabilized" epitope-encoding RNA, in particular mRNA, i.e. which is essentially resistant to *in vivo* degradation (e.g. by an exo- or endo-nuclease).

Such stabilization can be effected, for example, by a modified phosphate backbone of the epitope-encoding RNA (or any other nucleic acid, in particular RNA, as defined herein). A backbone modification in connection with the present invention is a modification in which phosphates of the backbone of the nucleotides contained in said RNA (or any other nucleic acid, in particular RNA, as defined herein) are chemically modified. Nucleotides that may be preferably used in this connection contain e.g. a phosphorothioate-modified phosphate backbone, preferably at least one of the phosphate oxygens contained in the phosphate backbone being replaced by a sulfur atom. Stabilized epitope-encoding RNAs (or other nucleic acids, in particular RNAs, as defined herein) may further include, for example: non-ionic phosphate analogues, such as, for example, alkyl and aryl phosphonates, in which the charged phosphonate oxygen is replaced by an alkyl or aryl group, or phosphodiesters and alkylphosphotriesters, in which the charged oxygen residue is present in alkylated form. Such backbone modifications typically include, without implying any limitation, modifications from the group consisting of methylphosphonates, phosphoramidates and phosphorothioates (e.g. cytidine-5'-O-(1-thiophosphate)).

In the following, specific modifications are described, which are preferably capable of "stabilizing" the epitope-encoding RNA of the inventive combination (or any other nucleic acid, in particular RNA, as defined herein).

### Chemical modifications

The term "modification" as used herein may refer to chemical modifications comprising backbone modifications as well as sugar modifications or base modifications.

In this context, a "modified" epitope-encoding RNA (or any other nucleic acid, in particular RNA, as defined herein) may contain nucleotide analogues/modifications, e.g. backbone modifications, sugar modifications or base modifications. A backbone modification in connection with the present invention is a modification, in which phosphates of the backbone of the nucleotides contained in said epitope-encoding RNA (or any other nucleic acid, in particular RNA, as defined herein) herein are chemically modified. A sugar modification in connection with the present invention is a chemical modification of the sugar of the nucleotides of the epitope-encoding RNA (or any other nucleic acid, in particular RNA, as defined herein). Furthermore, a base modification in connection with the present invention is a chemical modification of the base moiety of the nucleotides of the epitope-encoding RNA (or any other nucleic acid, in particular RNA, as defined herein). In this context, nucleotide analogues or modifications are preferably selected from nucleotide analogues, which are applicable for transcription and/or translation.

### Sugar Modifications:

The modified nucleosides and nucleotides, which may be incorporated into a "modified" epitope-encoding RNA (or any other nucleic acid, in particular RNA, as defined herein) can be modified in the sugar moiety. For example, the 2" hydroxyl group (OH) can be modified or replaced with a number of different "oxy" or "deoxy" substituents. Examples of "oxy" -2" hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG), -O(CH₂CH₂O)nCH₂CH₂OR; "locked" nucleic acids (LNA) in which the 2" hydroxyl is connected, e.g., by a methylene bridge, to the 4" carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH₂; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified epitope-encoding RNA (or any other nucleic acid, in particular RNA, as defined herein) can include nucleotides containing, for instance, arabinose as the sugar.

### Backbone Modifications:

The phosphate backbone may further be modified in the (modified) nucleosides and nucleotides, which may be incorporated into a modified epitope-encoding RNA (or any other nucleic acid, in particular RNA, as defined herein).

The phosphate groups of the backbone can be modified by replacing one or more of the oxygen atoms with a different substituent. Further, the (modified) nucleosides and nucleotides can include the full replacement of an unmodified phosphate moiety with a modified phosphate as described herein.

Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

### Base Modifications:

The modified nucleosides and nucleotides, which may be incorporated into a "modified" epitope-encoding RNA (or any other nucleic acid, in particular RNA, as defined herein) can further be modified in the nucleobase moiety.

Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group. In some embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2"-Amino-2"-deoxycytidine-triphosphate, 2-thiocytidine-5'-triphosphate, 2-thiouridine-5'-triphosphate, 2"-Fluorothymidine-5'-triphosphate, 2"-O-Methyl-inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2"-deoxycytidine-5'-triphosphate, 5-Bromo-2"-deoxyuridine-5'-triphosphate, 5-iodocytidine-5'-triphosphate, 5-lodo-2"-deoxycytidine-5'-triphosphate, 5-iodouridine-5'-triphosphate, 5-Iodo-2"-deoxyuridine-5'-triphosphate, 5-methylcytidine-5'-triphosphate, 5-methyluridine-5'-triphosphate, 5-Propynyl-2"-deoxycytidine-5'-triphosphate, 5-Propynyl-2"-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate. In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl- 1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine. In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine. In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. In some embodiments, the nucleotide can be modified on the major groove face and can include replacing hydrogen on C-5 of uracil with a methyl group or a halo group. In specific embodiments, a modified nucleoside is 5'-O-(1-thiophosphate)-adenosine, 5'-O-(1-thiophosphate)-cytidine, 5'-O-(1-thiophosphate)-guanosine, 5'-O-(1-thiophosphate)-uridine or 5'-O-(1-thiophosphate)-pseudouridine.

In some embodiments, a modified RNA (or any modified other nucleic acid, in particular RNA, as defined herein) may comprise nucleoside modifications selected from 6-aza-cytidine, 2-thio-cytidine, α-thio-cytidine, Pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, Pyrrolo-cytidine, inosine, α-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloro-purine, N6-methyl-adenosine, α-thio-adenosine, 8-azido-adenosine, 7-deaza-adenosine. In some embodiments, a modified epitope-encoding RNA (or any other nucleic acid, in particular RNA, as defined herein) does not comprise any of the chemical modifications as described herein. Said modified epitope-encoding RNA (or any other nucleic acid, in particular RNA, as defined herein) may comprise a lipid modification or a sequence modification as described below.

### Lipid modifications

According to further embodiments, a modified epitope-encoding RNA (or any other nucleic acid, in particular RNA, as defined herein) contains at least one lipid modification.

Such a lipid-modified epitope-encoding RNA (or said other nucleic acid, in particular RNA) typically comprises (i) an epitope-encoding RNA as defined herein (or said other nucleic acid, in particular RNA), (ii) at least one linker covalently linked with said epitope-encoding RNA (or said other nucleic acid, in particular RNA), and (iii) at least one lipid covalently linked with the respective linker.

Alternatively, the lipid-modified epitope-encoding RNA (or other nucleic acid as defined herein) comprises at least one epitope-encoding RNA (or said other nucleic acid, in particular RNA) and at least one (bifunctional) lipid covalently linked (without a linker) with said epitope-encoding RNA (or said other nucleic acid, in particular RNA).

Alternatively, the lipid-modified epitope-encoding RNA (or any other nucleic acid, in particular RNA, as defined herein) comprises (i) an epitope-encoding RNA (or said other nucleic acid, in particular RNA), (ii) at least one linker covalently linked with said epitope-encoding RNA (or said other nucleic acid, in particular RNA), and (iii) at least one lipid covalently linked with the respective linker, and also (iv) at least one (bifunctional) lipid covalently linked (without a linker) with said epitope-encoding RNA (or said other nucleic acid, in particular RNA).

In this context, it is particularly preferred that the lipid modification is present at the terminal ends of a linear epitope-encoding RNA sequence (or any other nucleic acid sequence defined herein).

### Sequence modifications

According to preferred embodiments, the epitope-encoding RNA of the inventive combination, preferably an mRNA, or any other nucleic acid as defined herein (in particular nucleic acids encoding PD-1 or LAG-3 pathway inhibitors as defined herein) comprises at least one sequence modification as described below.

### G/C content modification

According to preferred embodiments, the epitope-encoding RNA of the inventive combination, preferably an mRNA, (or any other nucleic acid, in particular RNA, as defined herein) may be modified, and thus stabilized, by modifying the guanosine/cytosine (G/C) content of said RNA (or said other nucleic acid, in particular RNA), preferably of the at least one coding sequence of said RNA (or said other nucleic acid, in particular RNA). In other words, the epitope-encoding RNA of the inventive combination (or any other nucleic acid, in particular RNA, as defined herein) may be G/C modified.

A "G/C-modified" RNA typically comprises an RNA sequence that is based on a modified wild-type RNA sequence and comprises an altered number of guanosine and/or cytosine nucleotides as compared to said wild-type RNA sequence. Such an altered number of G/C nucleotides may be generated by substituting codons containing adenosine or thymidine nucleotides by "synonymous" codons containing guanosine or cytosine nucleotides. Accordingly, the codon substitutions preferably do not alter the encoded amino acid residues, but exclusively alter the G/C content of the nucleic acid molecule.

In a particularly preferred embodiment of the present invention, the G/C content of the coding sequence of the epitope-encoding RNA of the inventive combination (or any other nucleic acid, in particular RNA, as defined herein) is modified, particularly increased, compared to the G/C content of the coding sequence of the respective wild-type RNA, i.e. the unmodified RNA (or of said other nucleic acid, in particular RNA). The amino acid sequence encoded by the RNA (or any other nucleic acid, in particular RNA, as defined herein) is preferably not modified as compared to the amino acid sequence encoded by the respective wild-type RNA (or said other nucleic acid, in particular RNA).

Such modification of the epitope-encoding RNA of the inventive combination (or any other nucleic acid, in particular RNA, as defined herein) is based on the fact that the sequence of any RNA (or other nucleic acid, in particular RNA) region to be translated is important for efficient translation of said RNA (or said other nucleic acid, in particular RNA). Thus, the composition of the RNA (or said other nucleic acid, in particular RNA) and the sequence of various nucleotides are important. In particular, sequences having an increased G (guanosine)/C (cytosine) content are more stable than sequences having an increased A (adenosine)/U (uracil) content.

According to the invention, the codons of the epitope-encoding RNA (or any other nucleic acid, in particular RNA, as defined herein) are therefore varied compared to the respective wild-type RNA (or said other nucleic acid, in particular RNA), while retaining the translated amino acid sequence, such that they include an increased amount of G/C nucleotides.

In respect to the fact that several codons code for one and the same amino acid (so-called degeneration of the genetic code), the most favourable codons for the stability can be determined (so-called alternative codon usage). Depending on the amino acid to be encoded by the epitope-encoding RNA (or any other nucleic acid, in particular RNA, as defined herein), there are various possibilities for modification of the RNA (or said other nucleic acid, in particular RNA) sequence, compared to its wild-type sequence. In the case of amino acids, which are encoded by codons, which contain exclusively G or C nucleotides, no modification of the codon is necessary.

Thus, the codons for Pro (CCC or CCG), Arg (CGC or CGG), Ala (GCC or GCG) and Gly (GGC or GGG) require no modification, since no A or U is present. In contrast, codons which contain A and/or U nucleotides can be modified by substitution of other codons, which code for the same amino acids but contain no A and/or U. Examples of these are: the codons for Pro can be modified from CCU or CCA to CCC or CCG; the codons for Arg can be modified from CGU or CGA or AGA or AGG to CGC or CGG; the codons for Ala can be modified from GCU or GCA to GCC or GCG; the codons for Gly can be modified from GGU or GGA to GGC or GGG. In other cases, although A or U nucleotides cannot be eliminated from the codons, it is however possible to decrease the A and U content by using codons which contain a lower content of A and/or U nucleotides. Examples of these are: the codons for Phe can be modified from UUU to UUC; the codons for Leu can be modified from UUA, UUG, CUU or CUA to CUC or CUG; the codons for Ser can be modified from UCU or UCA or AGU to UCC, UCG or AGC; the codon for Tyr can be modified from UAU to UAC; the codon for Cys can be modified from UGU to UGC; the codon for His can be modified from CAU to CAC; the codon for Gln can be modified from CAA to CAG; the codons for Ile can be modified from AUU or AUA to AUC; the codons for Thr can be modified from ACU or ACA to ACC or ACG; the codon for Asn can be modified from AAU to AAC; the codon for Lys can be modified from AAA to AAG; the codons for Val can be modified from GUU or GUA to GUC or GUG; the codon for Asp can be modified from GAU to GAC; the codon for Glu can be modified from GAA to GAG; the stop codon UAA can be modified to UAG or UGA. In the case of the codons for Met (AUG) and Trp (UGG), on the other hand, there is no possibility of sequence modification. The substitutions listed above can be used either individually or in all possible combinations to increase the G/C content of the at least one RNA of the inventive combination (or any other nucleic acid, in particular RNA, as defined herein) compared to its particular wild-type RNA (or said other nucleic acid, in particular RNA) sequence (i.e. the original sequence). Thus, for example, all codons for Thr occurring in the wild-type sequence can be modified to ACC (or ACG). Preferably, however, for example, combinations of the above substitution possibilities are used:
substitution of all codons coding for Thr in the original sequence (wild-type RNA) to ACC (or ACG) and
substitution of all codons originally coding for Ser to UCC (or UCG or AGC); substitution of all codons coding for Ile in the original sequence to AUC and
substitution of all codons originally coding for Lys to AAG and
substitution of all codons originally coding for Tyr to UAC; substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Arg to CGC (or CGG); substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Gly to GGC (or GGG) and
substitution of all codons originally coding for Asn to AAC; substitution of all codons coding for Val in the original sequence to GUC (or GUG) and
substitution of all codons originally coding for Phe to UUC and
substitution of all codons originally coding for Cys to UGC and
substitution of all codons originally coding for Leu to CUG (or CUC) and
substitution of all codons originally coding for Gln to CAG and
substitution of all codons originally coding for Pro to CCC (or CCG); etc.

Preferably, the G/C content of the coding sequence of the epitope-encoding RNA of the inventive combination (or any other nucleic acid, in particular RNA, as defined herein) is increased by at least 7%, more preferably by at least 15%, particularly preferably by at least 20%, compared to the G/C content of the coding sequence of the wild-type RNA (or said other nucleic acid, in particular RNA), which codes for at least one epitope as defined herein.

According to preferred embodiments, at least 5%, 10%, 20%, 30%, 40%, 50%, 60%, more preferably at least 70 %, even more preferably at least 80% and most preferably at least 90%, 95% or even 100% of the substitutable codons in the region coding for an epitope (or antigen or variant or fragment thereof comprising said epitope) as defined herein or the whole sequence of the wild type RNA (or said other nucleic acid, in particular RNA) sequence are substituted, thereby increasing the G/C content of said sequence.

In this context, it is particularly preferable to increase the G/C content of the epitope-encoding RNA of the inventive combination (or any other nucleic acid, in particular RNA, as defined herein), preferably of its at least one coding sequence, to the maximum (i.e. 100% of the substitutable codons) as compared to the wild-type sequence.

A further preferred modification of the epitope-encoding RNA of the inventive combination (or any other nucleic acid, in particular RNA, as defined herein) is based on the finding that the translation efficiency is also determined by a different frequency in the occurrence of tRNAs in cells. Thus, if so-called "rare codons" are present in the epitope-encoding RNA of the inventive combination (or said other nucleic acid, in particular RNA) to an increased extent, the corresponding modified RNA (or said other nucleic acid, in particular RNA) sequence is translated to a significantly poorer degree than in the case where codons coding for relatively "frequent" tRNAs are present.

In some preferred embodiments, in modified epitope-encoding RNAs (or any other nucleic acid, in particular RNA, as defined herein) defined herein, the region which codes for an epitope (or antigen or variant or fragment thereof comprising said epitope) is modified compared to the corresponding region of the wild-type RNA (or said other nucleic acid, in particular RNA) such that at least one codon of the wild-type sequence, which codes for a tRNA which is relatively rare in the cell, is exchanged for a codon, which codes for a tRNA which is relatively frequent in the cell and carries the same amino acid as the relatively rare tRNA.

Thereby, the sequences of the epitope-encoding RNA of the inventive combination (or any other nucleic acid, in particular RNA, as defined herein) is modified such that codons for which frequently occurring tRNAs are available are inserted. In other words, according to the invention, by this modification all codons of the wild-type sequence, which code for a tRNA which is relatively rare in the cell, can in each case be exchanged for a codon, which codes for a tRNA which is relatively frequent in the cell and which, in each case, carries the same amino acid as the relatively rare tRNA.

Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons, which use for the particular amino acid the tRNA which occurs the most frequently, e.g. the Gly codon, which uses the tRNA, which occurs the most frequently in the (human) cell, are particularly preferred.

According to the invention, it is particularly preferable to link the sequential G/C content which is increased, in particular maximized, in the modified epitope-encoding RNA of the inventive combination (or any other nucleic acid, in particular RNA, as defined herein), with the "frequent" codons without modifying the encoded amino acid sequence, e.g. of the epitope (or antigen or variant or fragment thereof comprising said epitope) encoded by the coding sequence of said epitope-encoding RNA. Such preferred embodiments allow the provision of a particularly efficiently translated and stabilized (modified) RNA of the inventive combination (or any other nucleic acid, in particular RNA, as defined herein).

The determination of a modified epitope-encoding RNA (or any other nucleic acid, in particular RNA, as defined herein) as described above (increased G/C content; exchange of tRNAs) can be carried out using the computer program explained in WO 02/098443, the disclosure content of which is included in its full scope in the present invention. Using this computer program, the nucleotide sequence of any desired nucleic acid, in particular RNA, can be modified with the aid of the genetic code or the degenerative nature thereof such that a maximum G/C content results, in combination with the use of codons which code for tRNAs occurring as frequently as possible in the cell, the amino acid sequence coded by the modified nucleic acid, in particular RNA, preferably not being modified compared to the non-modified sequence.

Alternatively, it is also possible to modify only the G/C content or only the codon usage compared to the original sequence. The source code in Visual Basic 6.0 (development environment used: Microsoft Visual Studio Enterprise 6.0 with Servicepack 3) is also described in WO 02/098443.

In further preferred embodiments of the present invention, the A/U content in the environment of the ribosome binding site of the epitope-encoding RNA of the inventive combination (or any other nucleic acid, in particular RNA, as defined herein) is increased compared to the A/U content in the environment of the ribosome binding site of its respective wild-type RNA (or said other nucleic acid, in particular RNA).

This modification (an increased A/U content around the ribosome binding site) increases the efficiency of ribosome binding to said epitope-encoding RNA (or any other nucleic acid, in particular RNA, as defined herein). An effective binding of the ribosomes to the ribosome binding site (Kozak sequence: SEQ ID NO: 7; the AUG forms the start codon) in turn has the effect of an efficient translation of the epitope-encoding RNA (or any other nucleic acid, in particular RNA, as defined herein).

According to further embodiments of the present invention, the epitope-encoding RNA of the inventive combination (or any other nucleic acid, in particular RNA, as defined herein) may be modified with respect to potentially destabilizing sequence elements. Particularly, the coding sequence and/or the 5' and/or 3' untranslated region of said epitope-encoding RNA (or said other nucleic acid, in particular RNA) may be modified compared to the respective wild-type RNA (or said other wild-type nucleic acid) such that it contains no destabilizing sequence elements, the encoded amino acid sequence of the modified RNA (or said other nucleic acid, in particular RNA) preferably not being modified compared to its respective wild-type RNA (or said other wild-type nucleic acid).

It is known that, for example in sequences of eukaryotic RNAs, destabilizing sequence elements (DSE) occur, to which signal proteins bind and regulate enzymatic degradation of RNA in vivo. For further stabilization of the modified epitope-encoding RNA, optionally in the region which encodes an epitope (or an antigen or variant or fragment thereof comprising said epitope), or any other nucleic acid as defined herein, one or more such modifications compared to the corresponding region of the wild-type RNA (or said other wild-type nucleic acid) can therefore be carried out, so that no or substantially no destabilizing sequence elements are contained there.

According to the invention, DSE present in the untranslated regions (3'- and/or 5'-UTR) can also be eliminated from the epitope-encoding RNA (or any other nucleic acid, in particular RNA, as defined herein) by such modifications. Such destabilizing sequences are e.g. AU-rich sequences (AURES), which occur in 3'-UTR sections of numerous unstable RNAs (Caput et al., Proc. Natl. Acad. Sci. USA 1986, 83: 1670 to 1674). The epitope-encoding RNA of the inventive combination (or any other nucleic acid, in particular RNA, as defined herein) is therefore preferably modified compared to the respective wild-type RNA (or said respective other wild-type nucleic acid) such that said epitope-encoding RNA (or said other nucleic acid, in particular RNA) contains no such destabilizing sequences. This also applies to those sequence motifs which are recognized by possible endonucleases, e.g. the sequence GAACAAG, which is contained in the 3'-UTR segment of the gene encoding the transferrin receptor (Binder et al., EMBO J. 1994, 13: 1969 to 1980). These sequence motifs are also preferably removed from said epitope-encoding RNA (or any other nucleic acid, in particular RNA, as defined herein).

### Sequences adapted to human codon usage:

A further preferred modification of the epitope-encoding RNA of the inventive combination (or any other nucleic acid, in particular RNA, as defined herein) is based on the finding that codons encoding the same amino acid typically occur at different frequencies. According to further preferred embodiments, in the modified epitope-encoding RNA (or said other nucleic acid, in particular RNA), the coding sequence is modified compared to the corresponding region of the respective wild-type RNA (or said other wild-type nucleic acid) such that the frequency of the codons encoding the same amino acid corresponds to the naturally occurring frequency of that codon according to the human codon usage as e.g. shown in Table 1.

For example, in the case of the amino acid alanine (Ala) present in an amino acid sequence encoded by the at least one coding sequence of the epitope-encoding RNA of the inventive combination (or any other nucleic acid, in particular RNA, as defined herein), the wild type coding sequence is preferably adapted in a way that the codon "GCC" is used with a frequency of 0.40, the codon "GCT" is used with a frequency of 0.28, the codon "GCA" is used with a frequency of 0.22 and the codon "GCG" is used with a frequency of 0.10 etc. (see Table 1).

**Table 1: Human codon usage table**

| Amino acid | codon | fraction | /1000 |
|---|---|---|---|
| Ala | GCG | 0.10 | 7.4 |
| Ala | GCA | 0.22 | 15.8 |
| Ala | GCT | 0.28 | 18.5 |
| Ala | GCC* | 0.40 | 27.7 |
| Cys | TGT | 0.42 | 10.6 |
| Cys | TGC* | 0.58 | 12.6 |
| Asp | GAT | 0.44 | 21.8 |
| Asp | GAC* | 0.56 | 25.1 |
| Glu | GAG* | 0.59 | 39.6 |
| Glu | GAA | 0.41 | 29.0 |
| Phe | TTT | 0.43 | 17.6 |
| Phe | TTC* | 0.57 | 20.3 |
| Gly | GGG | 0.23 | 16.5 |
| Gly | GGA | 0.26 | 16.5 |
| Gly | GGT | 0.18 | 10.8 |
| Gly | GGC* | 0.33 | 22.2 |
| His | CAT | 0.41 | 10.9 |
| His | CAC* | 0.59 | 15.1 |
| Ile | ATA | 0.14 | 7.5 |
| Ile | ATT | 0.35 | 16.0 |
| Ile | ATC* | 0.52 | 20.8 |
| Lys | AAG* | 0.60 | 31.9 |
| Lys | AAA | 0.40 | 24.4 |
| Leu | TTG | 0.12 | 12.9 |
| Leu | TTA | 0.06 | 7.7 |
| Leu | CTG* | 0.43 | 39.6 |
| Leu | CTA | 0.07 | 7.2 |
| Leu | CTT | 0.12 | 13.2 |
| Leu | CTC | 0.20 | 19.6 |
| Met | ATG* | 1 | 22.0 |
| Asn | AAT | 0.44 | 17.0 |
| Asn | AAC* | 0.56 | 19.1 |
| Pro | CCG | 0.11 | 6.9 |
| Pro | CCA | 0.27 | 16.9 |
| Pro | CCT | 0.29 | 17.5 |
| Pro | CCC* | 0.33 | 19.8 |
| Gln | CAG* | 0.73 | 34.2 |
| Gln | CAA | 0.27 | 12.3 |
| Arg | AGG | 0.22 | 12.0 |
| Arg | AGA* | 0.21 | 12.1 |
| Arg | CGG | 0.19 | 11.4 |
| Arg | CGA | 0.10 | 6.2 |
| Arg | CGT | 0.09 | 4.5 |
| Arg | CGC | 0.19 | 10.4 |
| Ser | AGT | 0.14 | 12.1 |
| Ser | AGC* | 0.25 | 19.5 |
| Ser | TCG | 0.06 | 4.4 |
| Ser | TCA | 0.15 | 12.2 |
| Ser | TCT | 0.18 | 15.2 |
| Ser | TCC | 0.23 | 17.7 |
| Thr | ACG | 0.12 | 6.1 |
| Thr | ACA | 0.27 | 15.1 |
| Thr | ACT | 0.23 | 13.1 |
| Thr | ACC* | 0.38 | 18.9 |
| Val | GTG* | 0.48 | 28.1 |
| Val | GTA | 0.10 | 7.1 |
| Val | GTT | 0.17 | 11.0 |
| Val | GTC | 0.25 | 14.5 |
| Trp | TGG* | 1 | 13.2 |
| Tyr | TAT | 0.42 | 12.2 |
| Tyr | TAC* | 0.58 | 15.3 |
| Stop | TGA* | 0.61 | 1.6 |
| Stop | TAG | 0.17 | 0.8 |
| Stop | TAA | 0.22 | 1.0 |

| | | | |
|---|---|---|---|
| *: most frequent codon | | | |

### Codon-optimized sequences:

As described above it is preferred according to the invention, that all codons of the wild-type sequence which code for a tRNA, which is relatively rare in the cell, are exchanged for a codon which codes for a tRNA, which is relatively frequent in the cell and which, in each case, carries the same amino acid as the relatively rare tRNA.

Therefore, it is particularly preferred that the most frequent codons are used for each encoded amino acid (see Table 1, most frequent codons are marked with asterisks). Such an optimization procedure increases the codon adaptation index (CAI) and ultimately maximises the CAI. In the context of the invention, sequences with increased or maximized CAI are typically referred to as "codon-optimized" sequences and/or CAI increased and/or maximized sequences. According to preferred embodiments, the epitope-encoding RNA of the inventive combination (or any other nucleic acid, in particular RNA, as defined herein) comprises at least one coding sequence, wherein the coding sequence is codon-optimized as described herein. More preferably, the codon adaptation index (CAI) of the at least one coding sequence is at least 0.5, at least 0.8, at least 0.9 or at least 0.95. Most preferably, the codon adaptation index (CAI) of the at least one coding sequence is 1.

For example, in the case of the amino acid alanine (Ala) present in the amino acid sequence encoded by the at least one coding sequence of the epitope-encoding RNA of the inventive combination (or any other nucleic acid, in particular RNA, as defined herein), the wild type coding sequence is adapted in a way that the most frequent human codon "GCC" is always used for said amino acid, or for the amino acid Cysteine (Cys), the wild type sequence is adapted in a way that the most frequent human codon "TGC" is always used for said amino acid etc.

### C-optimized sequences:

According to preferred embodiments, the epitope-encoding RNA of the inventive combination (or any other nucleic acid, in particular RNA, as defined herein) is modified by modifying, preferably increasing, the cytosine (C) content of said epitope-encoding RNA (or said other nucleic acid, in particular RNA), in particular in its at least one coding sequence.

In preferred embodiments, the C content of the coding sequence of the epitope-encoding RNA of the inventive combination (or any other nucleic acid, in particular RNA, as defined herein) is modified, preferably increased, compared to the C content of the coding sequence of the respective wild-type RNA, i.e. the unmodified RNA (or the respective other wild type nucleic acid). The amino acid sequence encoded by the at least one coding sequence of the epitope-encoding RNA of the inventive combination is preferably not modified as compared to the amino acid sequence encoded by the respective wild-type mRNA (or the respective other wild type nucleic acid).

In preferred embodiments, said modified epitope-encoding RNA (or any other nucleic acid, in particular RNA, as defined herein) is modified such that at least 10%, 20%, 30%, 40%, 50%, 60%, 70% or 80%, or at least 90% of the theoretically possible maximum cytosine-content or even a maximum cytosine-content is achieved.

In further preferred embodiments, at least 10%, 20%, 30%, 40%, 50%, 60%, 70%, 80%, 90% or even 100% of the codons of the wild-type RNA sequence, which are "cytosine content optimizable" are replaced by codons having a higher cytosine-content than the ones present in the wild type sequence.

In further preferred embodiments, some of the codons of the wild type coding sequence may additionally be modified such that a codon for a relatively rare tRNA in the cell is exchanged by a codon for a relatively frequent tRNA in the cell, provided that the substituted codon for a relatively frequent tRNA carries the same amino acid as the relatively rare tRNA of the original wild type codon. Preferably, all of the codons for a relatively rare tRNA are replaced by a codon for a relatively frequent tRNA in the cell, except codons encoding amino acids, which are exclusively encoded by codons not containing any cytosine, or except for glutamine (Gln), which is encoded by two codons each containing the same number of cytosines.

In further preferred embodiments of the present invention, the modified epitope-encoding RNA (or any other nucleic acid, in particular RNA, as defined herein) is modified such that at least 80%, or at least 90% of the theoretically possible maximum cytosine-content or even a maximum cytosine-content is achieved by means of codons, which code for relatively frequent tRNAs in the cell, wherein the amino acid sequence remains unchanged.

Due to the naturally occurring degeneracy of the genetic code, more than one codon may encode a particular amino acid. Accordingly, 18 out of 20 naturally occurring amino acids are encoded by more than one codon (with Tryp and Met being an exception), e.g. by 2 codons (e.g. Cys, Asp, Glu), by three codons (e.g. Ile), by 4 codons (e.g. Al, Gly, Pro) or by 6 codons (e.g. Leu, Arg, Ser). However, not all codons encoding the same amino acid are utilized with the same frequency under in vivo conditions. Depending on each single organism, a typical codon usage profile is established.

The term "cytosine content-optimizable codon" as used within the context of the present invention refers to codons, which exhibit a lower content of cytosines than other codons encoding the same amino acid. Accordingly, any wild type codon, which may be replaced by another codon encoding the same amino acid and exhibiting a higher number of cytosines within that codon, is considered to be cytosine-optimizable (C-optimizable). Any such substitution of a C-optimizable wild type codon by the specific C-optimized codon within a wild type coding sequence increases its overall C-content and reflects a C-enriched modified RNA sequence.

According to some preferred embodiments, the epitope-encoding RNA of the inventive combination (or any other nucleic acid, in particular RNA, as defined herein), and in particular its at least one coding sequence, comprises or consists of a C-maximized sequence containing C-optimized codons for all potentially C-optimizable codons.

Accordingly, 100% or all of the theoretically replaceable C-optimizable codons are preferably replaced by C-optimized codons over the entire length of the coding sequence.

In this context, cytosine-content optimizable codons are codons, which contain a lower number of cytosines than other codons coding for the same amino acid.

Any of the codons GCG, GCA, GCU codes for the amino acid Ala, which may be exchanged by the codon GCC encoding the same amino acid, and/or
the codon UGU that codes for Cys may be exchanged by the codon UGC encoding the same amino acid, and/or
the codon GAU which codes for Asp may be exchanged by the codon GAC encoding the same amino acid, and/or
the codon that UUU that codes for Phe may be exchanged for the codon UUC encoding the same amino acid, and/or
any of the codons GGG, GGA, GGU that code Gly may be exchanged by the codon GGC encoding the same amino acid, and/or
the codon CAU that codes for His may be exchanged by the codon CAC encoding the same amino acid, and/or
any of the codons AUA, AUU that code for Ile may be exchanged by the codon AUC, and/or
any of the codons UUG, UUA, CUG, CUA, CUU coding for Leu may be exchanged by the codon CUC encoding the same amino acid, and/or
the codon AAU that codes for Asn may be exchanged by the codon AAC encoding the same amino acid, and/or
any of the codons CCG, CCA, CCU coding for Pro may be exchanged by the codon CCC encoding the same amino acid, and/or
any of the codons AGG, AGA, CGG, CGA, CGU coding for Arg may be exchanged by the codon CGC encoding the same amino acid, and/or
any of the codons AGU, AGC, UCG, UCA, UCU coding for Ser may be exchanged by the codon UCC encoding the same amino acid, and/or
any of the codons ACG, ACA, ACU coding for Thr may be exchanged by the codon ACC encoding the same amino acid, and/or
any of the codons GUG, GUA, GUU coding for Val may be exchanged by the codon GUC encoding the same amino acid, and/or
the codon UAU coding for Tyr may be exchanged by the codon UAC encoding the same amino acid.

In any of the above instances, the number of cytosines is increased by 1 per exchanged codon. Exchange of all non C-optimized codons (corresponding to C-optimizable codons) of the coding sequence results in a C-maximized coding sequence. In the context of the invention, at least 70%, preferably at least 80%, more preferably at least 90%, of the non C-optimized codons within the at least one coding sequence of the epitope-encoding RNA of the inventive combination (or any other nucleic acid, in particular RNA, as defined herein) are replaced by C-optimized codons.

It may be preferred that for some amino acids the percentage of C-optimizable codons replaced by C-optimized codons is less than 70%, while for other amino acids the percentage of replaced codons is higher than 70% to meet the overall percentage of C-optimization of at least 70% of all C-optimizable wild type codons of the coding sequence.

Preferably, in a C-optimized epitope-encoding RNA (or any other nucleic acid, in particular RNA, as defined herein), at least 50% of the C-optimizable wild type codons for any given amino acid are replaced by C-optimized codons, e.g. any modified C-enriched RNA (or other nucleic acid, in particular RNA) preferably contains at least 50% C-optimized codons at C-optimizable wild type codon positions encoding any one of the above mentioned amino acids Ala, Cys, Asp, Phe, Gly, His, Ile, Leu, Asn, Pro, Arg, Ser, Thr, Val and Tyr, preferably at least 60%.

In this context codons encoding amino acids, which are not cytosine content-optimizable and which are, however, encoded by at least two codons, may be used without any further selection process. However, the codon of the wild type sequence that codes for a relatively rare tRNA in the cell, e.g. a human cell, may be exchanged for a codon that codes for a relatively frequent tRNA in the cell, wherein both code for the same amino acid. Accordingly, the relatively rare codon GAA coding for Glu may be exchanged by the relative frequent codon GAG coding for the same amino acid, and/or
the relatively rare codon AAA coding for Lys may be exchanged by the relative frequent codon AAG coding for the same amino acid, and/or
the relatively rare codon CAA coding for Gln may be exchanged for the relative frequent codon CAG encoding the same amino acid.

In this context, the amino acids Met (AUG) and Trp (UGG), which are encoded by only one codon each, remain unchanged. Stop codons are not cytosine-content optimized, however, the relatively rare stop codons amber, ochre (UAA, UAG) may be exchanged by the relatively frequent stop codon opal (UGA).

The single substitutions listed above may be used individually as well as in all possible combinations in order to optimize the cytosine-content of the modified epitope-encoding RNA (or any other nucleic acid, in particular RNA, as described herein) compared to its respective wild-type nucleic acid sequence.

Accordingly, the at least one coding sequence as defined herein may be changed compared to the coding sequence of the respective wild type RNA (or other wild-type nucleic acid) in such a way that an amino acid encoded by at least two or more codons, of which one comprises one additional cytosine, such a codon may be exchanged by the C-optimized codon comprising one additional cytosine, wherein the amino acid is preferably unaltered compared to the wild type sequence.

According to particularly preferred embodiments, the inventive combination comprises an epitope-encoding RNA comprising at least one coding sequence as defined herein, wherein (a) the G/C content of the at least one coding sequence of said epitope-encoding RNA is increased compared to the G/C content of the corresponding coding sequence of the corresponding wild-type RNA, and/or (b) wherein the C content of the at least one coding sequence of said epitope-encoding RNA is increased compared to the C content of the corresponding coding sequence of the corresponding wild-type RNA, and/or (c) wherein the codons in the at least one coding sequence of said epitope-encoding RNA are adapted to human codon usage, wherein the codon adaptation index (CAI) is preferably increased or maximized in the at least one coding sequence of said epitope-encoding RNA, and wherein the amino acid sequence encoded by said epitope-encoding RNA is preferably not being modified compared to the amino acid sequence encoded by the corresponding wild-type RNA.

### 5' Cap

According to further preferred embodiments of the invention, a modified epitope-encoding RNA (or any other nucleic acid, in particular RNA) as defined herein, can be modified by the addition of a so-called "5' cap" structure, which preferably stabilizes said epitope-encoding RNA (or said other nucleic acid, in particular RNA) as described herein.

A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an mRNA. m7GpppN is the 5'-cap structure, which naturally occurs in mRNA transcribed by polymerase II and is therefore preferably not considered as modification comprised in a modified mRNA in this context. Accordingly, a "modified" epitope-encoding RNA (or any other nucleic acid, in particular RNA, as defined herein) may comprise a m7GpppN as 5'-cap, but additionally said modified epitope-encoding RNA (or other nucleic acid) typically comprises at least one further modification as defined herein.

Further examples of 5'cap structures include glyceryl, inverted deoxy abasic residue (moiety), 4",5' methylene nucleotide, 1-(beta-D-erythrofuranosyl) nucleotide, 4"-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4"-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2"-inverted nucleotide moiety, 3'-2"-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate, moiety. These modified 5'-cap structures are regarded as at least one modification in this context.

Particularly preferred modified 5'-cap structures are cap1 (methylation of the ribose of the adjacent nucleotide of m7G), cap2 (additional methylation of the ribose of the 2nd nucleotide downstream of the m7G), cap3 (additional methylation of the ribose of the 3rd nucleotide downstream of the m7G), cap4 (methylation of the ribose of the 4th nucleotide downstream of the m7G), ARCA (anti-reverse cap analogue, modified ARCA (e.g. phosphothioate modified ARCA), inosine, N1-methyl-guanosine, 2"-fluoro-guanosine, 7-deaza-guanosine, 8-oxo-guanosine, 2-amino-guanosine, LNA-guanosine, and 2-azido-guanosine.

### Poly(A)

According to further preferred embodiments, the epitope-encoding RNA of the inventive combination (or any other nucleic acid, in particular RNA, as defined herein) contains a poly(A) sequence or poly(A) tail.

A poly(A) sequence, also called poly(A) tail or 3'-poly(A) tail, is typically understood to be a sequence of adenosine nucleotides, e.g., of up to about 400 adenosine nucleotides, e.g. from about 20 to about 400, preferably from about 50 to about 400, more preferably from about 50 to about 300, even more preferably from about 50 to about 250, most preferably from about 60 to about 250 adenosine nucleotides. As used herein, a poly(A) sequence may also comprise about 10 to 200 adenosine nucleotides, preferably about 10 to 100 adenosine nucleotides, more preferably about 40 to 80 adenosine nucleotides or even more preferably about 50 to 70 adenosine nucleotides. A poly(A) sequence is typically located at the 3'end of an RNA, in particular a mRNA.

Accordingly, in further preferred embodiments, the epitope-encoding RNA of the inventive combination (or any other nucleic acid, in particular RNA, as defined herein) contains at its 3' terminus a poly(A) tail of typically about 10 to 200 adenosine nucleotides, preferably about 10 to 100 adenosine nucleotides, more preferably about 40 to 80 adenosine nucleotides or even more preferably about 50 to 70 adenosine nucleotides.

Preferably, the poly(A) sequence in the epitope-encoding RNA of the inventive combination (or said other nucleic acid, in particular RNA) is derived from a DNA template by RNA *in vitro* transcription. Alternatively, the poly(A) sequence may also be obtained *in vitro* by common methods of chemical-synthesis without being necessarily transcribed from a DNA-progenitor.

Moreover, poly(A) sequences, or poly(A) tails may be generated by enzymatic polyadenylation of the epitope-encoding RNA of the inventive combination (or said other nucleic acid, in particular RNA) using commercially available polyadenylation kits and corresponding protocols known in the art. Polyadenylation is typically understood to be the addition of a poly(A) sequence to a nucleic acid molecule, such as an RNA molecule, e.g. to a premature mRNA. Polyadenylation may be induced by a so-called polyadenylation signal. This signal is preferably located within a stretch of nucleotides at the 3'-end of the mRNA to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises a step of polyadenylation.

Accordingly, the epitope-encoding RNA of the inventive combination (or any other nucleic acid, in particular RNA, as defined herein) may comprise a polyadenylation signal which conveys polyadenylation to a (transcribed) RNA by specific protein factors (e.g. cleavage and polyadenylation specificity factor (CPSF), cleavage stimulation factor (CstF), cleavage factors I and II (CF I and CF II), poly(A) polymerase (PAP)).

In this context, a consensus polyadenylation signal is preferred comprising the NN(U/T)ANA consensus sequence. In a particularly preferred aspect, the polyadenylation signal comprises one of the following sequences: AA(U/T)AAA or A(U/T)(U/T)AAA (wherein uridine is usually present in RNA and thymidine is usually present in DNA).

### Poly(C)

According to further preferred embodiments, the epitope-encoding RNA of the inventive combination (or any other nucleic acid, in particular RNA, as defined herein) contains a poly(C) tail on the 3' terminus of typically about 10 to 200 cytosine nucleotides, preferably about 10 to 100 cytosine nucleotides, more preferably about 20 to 70 cytosine nucleotides or even more preferably about 20 to 60 or even 10 to 40 cytosine nucleotides.

### UTRs

According to preferred embodiments, the the epitope-encoding RNA of the inventive combination (or any other nucleic acid, in particular RNA, as defined herein) comprises at least one 5'- or 3'-UTR element. In this context, an UTR element comprises or consists of a nucleic acid sequence, which is derived from the 5'- or 3'-UTR of any naturally occurring gene or which is derived from a fragment, a homolog or a variant of the 5'- or 3'-UTR of a gene. Preferably, the 5'- or 3'-UTR element used according to the present invention is heterologous to the at least one coding sequence of said epitope-encoding RNA (or other nucleic acid, in particular RNA). Even if 5'- or 3'-UTR elements derived from naturally occurring genes are preferred, also synthetically engineered UTR elements may be used in the context of the present invention.

### 3' UTR

According to preferred embodiments, the epitope-encoding RNA of the inventive combination (or any other nucleic acid, in particular RNA, as defined herein) further comprises at least one 3'UTR element.

The term "3'UTR element" typically refers to a nucleic acid sequence, which comprises or consists of a nucleic acid sequence that is derived from a 3'UTR or from a variant of a 3'UTR.

Generally, the term "3'-UTR" refers to a part of a nucleic acid molecule, which is located 3' (i.e. "downstream") of an open reading frame and which is not translated into protein. In the context of the present invention, a 3'-UTR corresponds to a sequence which is located between the stop codon of the protein coding sequence, preferably immediately 3' to the stop codon of the protein coding sequence, and the poly(A) sequence of the epitope-encoding RNA (or any other nucleic acid, in particular RNA, as defined herein).

The term "corresponds to" means that the 3'-UTR sequence may be an RNA sequence, such as in the mRNA sequence used for defining the 3'-UTR sequence, or a DNA sequence, which corresponds to such RNA sequence. In the context of the present invention, the term "a 3'-UTR of a gene", such as "a 3'-UTR of a ribosomal protein gene", is the sequence, which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR.

A 3'UTR element in the sense of the present invention may represent the 3'UTR of an RNA, preferably an mRNA. Thus, in the sense of the present invention, preferably, a 3'UTR element may be the 3'UTR of an RNA, preferably of an mRNA, or it may be the transcription template for a 3'UTR of an RNA. Thus, a 3'UTR element preferably is a nucleic acid sequence which corresponds to the 3'UTR of an RNA, preferably to the 3'UTR of an mRNA, such as an mRNA obtained by transcription of a genetically engineered vector construct. Preferably, the 3'UTR element fulfils the function of a 3'UTR or encodes a sequence which fulfils the function of a 3'UTR.

Preferably, the at least one 3'UTR element comprises or consists of a nucleic acid sequence derived from the 3'UTR of a chordate gene, preferably a vertebrate gene, more preferably a mammalian gene, most preferably a human gene, or from a variant of the 3'UTR of a chordate gene, preferably a vertebrate gene, more preferably a mammalian gene, most preferably a human gene.

Preferably, the epitope-encoding RNA of the inventive combination (or any other nucleic acid, in particular RNA, as defined herein) comprises a 3'UTR element, which may be derivable from a gene that relates to an mRNA with an enhanced half-life (that provides a stable mRNA), for example a 3'UTR element as defined and described below. Preferably, the 3'-UTR element is a nucleic acid sequence derived from a 3'-UTR of a gene, which preferably encodes a stable mRNA, or from a homolog, a fragment or a variant of said gene

In particularly preferred embodiments, the 3'UTR element comprises or consists of a nucleic acid sequence, which is derived from a 3'UTR of a gene selected from the group consisting of an albumin gene, an alpha-globin gene, a beta-globin gene, a tyrosine hydroxylase gene, a lipoxygenase gene, and a collagen alpha gene, such as a collagen alpha 1(I) gene, or from a variant of a 3'UTR of a gene selected from the group consisting of an albumin gene, an alpha-globin gene, a beta-globin gene, a tyrosine hydroxylase gene, a lipoxygenase gene, and a collagen alpha gene, such as a collagen alpha 1(I) gene according to SEQ ID No. 1369-1390 of the patent application WO2013/143700, whose disclosure is incorporated herein by reference, or from a homolog, a fragment or a variant thereof.

The term "a nucleic acid sequence which is derived from the 3'UTR of a [...] gene" preferably refers to a nucleic acid sequence which is based on the 3'UTR sequence of a [...] gene or on a part thereof, such as on the 3'UTR of an albumin gene, an alpha-globin gene, a beta-globin gene, a tyrosine hydroxylase gene, a lipoxygenase gene, or a collagen alpha gene, such as a collagen alpha 1(I) gene, preferably of an albumin gene or on a part thereof. This term includes sequences corresponding to the entire sequence of the variant of the 3'UTR of a gene, i.e. the full length variant 3'UTR sequence of a gene, and sequences corresponding to a fragment of the variant 3'UTR sequence of a gene. A fragment in this context preferably consists of a continuous stretch of nucleotides corresponding to a continuous stretch of nucleotides in the full-length variant 3'UTR, which represents at least 20%, preferably at least 30%, more preferably at least 40%, more preferably at least 50%, even more preferably at least 60%, even more preferably at least 70%, even more preferably at least 80%, and most preferably at least 90% of the full-length variant 3'UTR. Such a fragment of a variant, in the sense of the present invention, is preferably a functional fragment of a variant as described herein.

### Albumin-derived 3' UTRs

In a particularly preferred embodiment, the 3'UTR element comprises or consists of a nucleic acid sequence which is derived from a 3'UTR of an albumin gene, preferably a vertebrate albumin gene, more preferably a mammalian albumin gene, most preferably a human albumin gene according to SEQ ID NO: 8 or the corresponding RNA sequence.

In this context it is particularly preferred that the epitope-encoding RNA of the inventive combination (or any other nucleic acid, in particular RNA, as defined herein) comprises a 3'-UTR element comprising a corresponding RNA sequence derived from the nucleic acids according to SEQ ID No. 1369-1390 of the patent application WO2013/143700 or a fragment, homolog or variant thereof.

Most preferably the 3'-UTR element comprises the nucleic acid sequence derived from a fragment of the human albumin gene according to SEQ ID NO: 9:
In this context, it is particularly preferred that the 3'-UTR element of the RNA according to the present invention (or any other nucleic acid, in particular RNA, as defined herein) comprises or consists of a corresponding RNA sequence of the nucleic acid sequence according to SEQ ID NO: 8 or 9.

### Globin-derived 3'UTRs

In another particularly preferred embodiment, the 3'UTR element comprises or consists of a nucleic acid sequence which is derived from a 3'UTR of an alpha-globin gene, preferably a vertebrate alpha- or beta-globin gene, more preferably a mammalian alpha-or beta-globin gene, most preferably a human alpha- or beta-globin gene according to SEQ ID NO: 10, 11 or 12 or the corresponding RNA sequences:
For example, the 3'UTR element may comprise or consist of the center, alpha-complex-binding portion of the 3'UTR of an alpha-globin gene, such as of a human alpha-globin gene, or a homolog, a fragment, or a variant of an alpha-globin gene, preferably according to SEQ ID NO: 13:
Center, alpha-complex-binding portion of the 3'UTR of an alpha-globin gene (also named herein as "muag")
GCCCGATGGGCCTCCCAACGGGCCCTCCTCCCCTCCTTGCACCG (SEQ ID NO: 13 corresponding to SEQ ID No. 1393 of the patent application WO2013/143700).

In this context it is particularly preferred that the 3'-UTR element of the epitope-encoding RNA of the inventive combination (or any other nucleic acid, in particular RNA, as defined herein) comprises or consists of a corresponding RNA sequence of the nucleic acid sequence according to SEQ ID NO: 13, or a homolog, a fragment or variant thereof.

In embodiments, the RNA as defined herein comprises a 3'-UTR as described in WO2016/107877. In this context, the disclosure of WO2016/107877 relating to 3'-UTR sequences is herewith incorporated by reference. Particularly suitable 3'-UTRs are SEQ ID NOs: 1 to 24 and SEQ ID NOs: 49 to 318 of patent application WO2016/107877, or fragments or variants of these sequences. Accordingly, the 3'-UTRs of the RNA of the present invention may comprise or consists of a corresponding RNA sequence of the nucleic acid sequence according SEQ ID NOs: 1 to 24 and SEQ ID NOs: 49 to 318 of the patent application WO2016/107877.

In embodiments, the RNA as defined herein comprises a 3'-UTR as described in WO2017/036580. In this context, the disclosure of WO2017/036580 relating to 3'-UTR sequences is herewith incorporated by reference. Particularly suitable 3'-UTRs are SEQ ID NOs: 152 to 204 of the patent application WO2017/036580, or fragments or variants of these sequences. Accordingly, the 3'-UTR of the RNA of the present invention may comprise or consist of a corresponding RNA sequence of the nucleic acid sequence according SEQ ID NOs: 152 to 204 of the patent application WO2017/036580.

### 5' UTR

According to particularly preferred embodiments, the at least one epitope-encoding RNA of the inventive combination (or any other nucleic acid, in particular RNA, as defined herein) comprises at least one 5'-untranslated region element (5'UTR element).

A 5'-UTR is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, also called regulatory elements. Such regulatory elements may be, for example, ribosomal binding sites. The 5'-UTR may be post-transcriptionally modified, for example by addition of a 5'-Cap. In the context of the present invention, a 5'-UTR corresponds to the sequence of a mature mRNA, which is located between the 5'-Cap and the start codon. Preferably, the 5'-UTR corresponds to the sequence, which extends from a nucleotide located 3' to the 5'-Cap, preferably from the nucleotide located immediately 3' to the 5'-Cap, to a nucleotide located 5' to the start codon of the protein coding sequence, preferably to the nucleotide located immediately 5' to the start codon of the protein coding sequence. The nucleotide located immediately 3' to the 5'-Cap of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence, which corresponds to such RNA sequence. In the context of the present invention, the term "a 5'-UTR of a gene" is the sequence, which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence of the 5'-UTR. By the inventive embodiments such a 5'-UTR may be provided 5'-terminal to the coding sequence. Its length is typically less than 500, 400, 300, 250 or less than 200 nucleotides. In other embodiments its length may be in the range of at least 10, 20, 30 or 40, preferably up to 100 or 150, nucleotides.

### TOP-gene derived 5' UTRs

According to particularly preferred embodiments, the epitope-encoding RNA of the inventive combination (or any other nucleic acid, in particular RNA, as defined herein) comprises at least one 5'-untranslated region element (5'UTR element) which comprises or consists of a nucleic acid sequence, which is derived from the 5'UTR of a TOP gene or which is derived from a fragment, homolog or variant of the 5'UTR of a TOP gene.

The 5'terminal oligopyrimidine tract (TOP) is typically a stretch of pyrimidine nucleotides located in the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. mRNA that contains a 5'terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP genes are typically characterized by the presence of a 5' terminal oligopyrimidine tract (TOP). Furthermore, most TOP genes are characterized by a growth-associated translational regulation. However, also TOP genes with a tissue specific translational regulation are known. As defined above, the 5'UTR of a TOP gene corresponds to the sequence of a 5'UTR of a mature mRNA derived from a TOP gene, which preferably extends from the nucleotide located 3' to the 5'-CAP to the nucleotide located 5' to the start codon. A 5'UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'UTRs of TOP genes are generally rather short. The lengths of 5'UTRs of TOP genes may vary between 20 nucleotides up to 500 nucleotides, and are typically less than about 200 nucleotides, preferably less than about 150 nucleotides, more preferably less than about 100 nucleotides. Exemplary 5'UTRs of TOP genes in the sense of the present invention are the nucleic acid sequences extending from the nucleotide at position 5 to the nucleotide located immediately 5' to the start codon (e.g. the ATG) in the sequences according to SEQ ID Nos. 1-1363 of the patent application WO2013/143700, whose disclosure is incorporated herewith by reference. In this context, a particularly preferred fragment of a 5'UTR of a TOP gene is a 5'UTR of a TOP gene lacking the 5'TOP motif. The terms "5'UTR of a TOP gene" or "5'-TOP UTR" preferably refer to the 5'UTR of a naturally occurring TOP gene.

In the context of the present invention, a "TOP motif' is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence, which represents a 5'UTR, or at the 5'end of a sequence, which codes for a 5'UTR. Thus, preferably, a stretch of 3 or more pyrimidine nucleotides is called "TOP motif" in the sense of the present invention if this stretch is located at the 5'end of a respective sequence, such as the artificial nucleic acid molecule, the 5'UTR element of the artificial nucleic acid molecule, or the nucleic acid sequence which is derived from the 5'UTR of a TOP gene as described herein. In other words, a stretch of 3 or more pyrimidine nucleotides, which is not located at the 5'-end of a 5'UTR or a 5'UTR element but anywhere within a 5'UTR or a 5'UTR element, is preferably not referred to as "TOP motif".

In particularly preferred embodiments, the 5'UTR element of the epitope-encoding RNA of the inventive combination (or any other nucleic acid, in particular RNA, as defined herein) does not comprise a TOP-motif or a 5'TOP, as defined above.

In some embodiments, the nucleic acid sequence of the 5'UTR element, which is derived from a 5'UTR of a TOP gene, terminates at its 3'-end with a nucleotide located at position 1, 2, 3, 4, 5, 6, 7, 8, 9 or 10 upstream of the start codon (e.g. A(U/T)G) of the gene or mRNA it is derived from. Thus, the 5'UTR element does not comprise any part of the protein coding sequence. Thus, preferably, the only amino acid coding part of the at least one epitope-encoding RNA of the inventive combination (or said other nucleic acid, in particular RNA) is provided by the coding sequence.

The nucleic acid sequence derived from the 5'UTR of a TOP gene is preferably derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the 5'UTR element is prefereably selected from 5'-UTR elements comprising or consisting of a nucleic acid sequence, which is derived from a nucleic acid sequence selected from the group consisting of SEQ ID Nos. 1-1363, SEQ ID NO. 1395, SEQ ID NO. 1421 and SEQ ID NO. 1422 of the patent application WO2013/143700, whose disclosure is incorporated herein by reference, from the homologs of SEQ ID Nos. 1-1363, SEQ ID NO. 1395, SEQ ID NO. 1421 and SEQ ID NO. 1422 of the patent application WO2013/143700, from a variant thereof, or preferably from a corresponding RNA sequence. The term "homologs of SEQ ID Nos. 1-1363, SEQ ID NO. 1395, SEQ ID NO. 1421 and SEQ ID NO. 1422 of the patent application WO2013/143700" refers to sequences of other species than homo sapiens, which are homologous to the sequences according to SEQ ID Nos. 1-1363, SEQ ID NO. 1395, SEQ ID NO. 1421 and SEQ ID NO. 1422 of the patent application WO2013/143700.

In preferred embodiments, the 5'UTR element of the epitope-encoding RNA of the inventive combination (or any other nucleic acid, in particular RNA, as defined herein) comprises or consists of a nucleic acid sequence, which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID Nos. 1-1363, SEQ ID NO. 1395, SEQ ID NO. 1421 and SEQ ID NO. 1422 of the patent application WO2013/143700, from the homologs of SEQ ID Nos. 1-1363, SEQ ID NO. 1395, SEQ ID NO. 1421 and SEQ ID NO. 1422 of the patent application WO2013/143700 from a variant thereof, or a corresponding RNA sequence. It is particularly preferred that the 5' UTR element is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID Nos. 1-1363, SEQ ID NO. 1395, SEQ ID NO. 1421 and SEQ ID NO. 1422 of the patent application WO2013/143700, from the homologs of SEQ ID Nos. 1-1363, SEQ ID NO. 1395, SEQ ID NO. 1421 and SEQ ID NO. 1422 of the patent application WO2013/143700, from a variant thereof, or a corresponding RNA sequence.

In particularly preferred embodiments, the 5'UTR element comprises or consists of a nucleic acid sequence, which is derived from a 5'UTR of a TOP gene encoding a ribosomal protein or from a variant of a 5'UTR of a TOP gene encoding a ribosomal protein. For example, the 5'UTR element comprises or consists of a nucleic acid sequence, which is derived from a 5'UTR of a nucleic acid sequence according to any of SEQ ID NOs: 67, 170, 193, 244, 259, 554, 650, 675, 700, 721, 913, 1016, 1063, 1120, 1138, and 1284-1360 of the patent application WO2013/143700, a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'UTR of said sequences.

In some embodiments, the epitope-encoding RNA of the inventive combination (or any other nucleic acid, in particular RNA, as defined herein) thus comprises a 5'UTR element, which comprises or consists of a nucleic acid sequence, which is derived from the 5'UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB, or from a homolog or variant thereof, wherein preferably the 5'UTR element does not comprise a TOP-motif or the 5'TOP of said genes, and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene it is derived from.

In further particularly preferred embodiments, the 5'UTR element comprises or consists of a nucleic acid sequence, which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit Vlc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit Vic gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cyto-chrome c oxidase subunit Vlc gene (COX6C), or a mammalian N-acylsphingosine ami-dohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP syn-thase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit Vlc gene (COX6C), or a human N-acylsphingosine amidohydrolase (acid ceramidase) 1 gene (ASAH1) or from a variant thereof, wherein preferably the 5'UTR element does not comprise the 5'TOP of said gene.

In some preferred embodiments, the 5'UTR element comprises or consists of a nucleic acid sequence, which is derived from a 5'UTR of a TOP gene encoding a mitochondrial ATP synthase subunit alpha or from a homolog or variant of a 5'UTR of a TOP gene encoding a mitochondrial ATP synthase subunit alpha, preferably lacking the 5'TOP motif.

In this context, the 5'UTR element preferably comprises or consists of a nucleic acid sequence which is derived from the 5'UTR of a mitochondrial ATP synthase subunit alpha gene, preferably from a vertebrate mitochondrial ATP synthase subunit alpha (ATP5A1) gene, more preferably from a mammalian mitochondrial ATP synthase subunit alpha (ATP5A1) gene, most preferably from a human mitochondrial ATP synthase subunit alpha (ATP5A1) gene, or from a variant of the 5'UTR of a mitochondrial ATP synthase subunit alpha gene, preferably from a vertebrate mitochondrial ATP synthase subunit alpha (ATP5A1) gene, more preferably from a mammalian mitochondrial ATP synthase subunit alpha (ATP5A1) gene, most preferably from a human mitochondrial ATP synthase subunit alpha (ATP5A1) gene, wherein preferably the 5'UTR element does not comprise the 5'TOP of said gene.

Accordingly, in a particularly preferred embodiment, the 5'UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 14 (5'-UTR of ATP5A1 lacking the 5' terminal oligopyrimidine tract: GCGGCTCGGCCATTTTGTCCCAGTCAGTCCGGAGGCTGCGGCTGCAGAAGTACCGCCTGCGGAGTAACTGCAA AG; corresponding to SEQ ID NO. 1414 of the patent application WO2013/143700) or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 14 or more preferably to a corresponding RNA sequence, wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

### L32 derived 5' UTR

In some preferred embodiments, the 5'UTR element comprises or consists of a nucleic acid sequence, which is derived from a 5'UTR of a TOP gene encoding a ribosomal Large protein (RPL) or from a homolog or variant of a 5'UTR of a TOP gene encoding a ribosomal Large protein (RPL). For example, the 5'UTR element comprises or consists of a nucleic acid sequence, which is derived from a 5'UTR of a nucleic acid sequence according to any of SEQ ID NOs: 67, 259, 1284-1318, 1344, 1346, 1348-1354, 1357, 1358, 1421 and 1422 of the patent application WO2013/143700, a corresponding RNA sequence, a homolog thereof, or a variant thereof as described herein, preferably lacking the 5'TOP motif.

In this context, the 5'UTR element preferably comprises or consists of a nucleic acid sequence which is derived from the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, or from a variant of the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, wherein preferably the 5'UTR element does not comprise the 5'TOP of said gene.

Accordingly, in some particularly preferred embodiments, the 5'UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 15 (5'-UTR of human ribosomal protein Large 32 lacking the 5' terminal oligopyrimidine tract: GGCGCTGCCTACGGAGGTGGCAGCCATCTCCTTCTCGGCATC; corresponding to SEQ ID NO. 1368 of the patent application WO2013/143700) or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 15 or more preferably to a corresponding RNA sequence, wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

### HSD17B4 derived 5' UTR

In some preferred embodiments, the 5'UTR element comprises or consists of a nucleic acid sequence, which is derived from a 5'UTR of a TOP gene encoding a 17-beta-hydroxysteroid dehydrogenase 4 or from a homolog or variant of a 5'UTR of a TOP gene encoding a 17-beta-hydroxysteroid dehydrogenase 4, preferably lacking the 5'TOP motif.

In this context, the 5'UTR element preferably comprises or consists of a nucleic acid sequence which is derived from the 5'UTR of a 17-beta-hydroxysteroid dehydrogenase 4 (also referred to as peroxisomal multifunctional enzyme type 2) gene, preferably from a vertebrate 17-beta-hydroxysteroid dehydrogenase 4 (HSD17B4) gene, more preferably from a mammalian 17-beta-hydroxysteroid dehydrogenase 4 (HSD17B4) gene, most preferably from a human 17-beta-hydroxysteroid dehydrogenase 4 (HSD17B4) gene, or from a variant of the 5'UTR of a 17-beta-hydroxysteroid dehydrogenase 4 gene, preferably from a vertebrate 17-beta-hydroxysteroid dehydrogenase 4 (HSD17B4) gene, more preferably from a mammalian 17-beta-hydroxysteroid dehydrogenase 4 (HSD17B4) gene, most preferably from a human 17-beta-hydroxysteroid dehydrogenase 4 (HSD17B4) gene, wherein preferably the 5'UTR element does not comprise the 5'TOP of said gene.

Accordingly, in some particularly preferred embodiments, the 5'UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 16 (5'-UTR of human 17-beta-hydroxysteroid dehydrogenase 4 lacking the 5' terminal oligopyrimidine tract: GTCCCGCAGTCGGCGTCCAGCGGCTCTGCTTGTTCGTGTGTGTGTCGTTGCAGGCCTTATTC; corresponding to SEQ ID NO: 1415 of the patent application WO2013/143700)

Accordingly, in some particularly preferred embodiments, the 5'UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 16 (5'-UTR of human 17-beta-hydroxysteroid dehydrogenase 4 lacking the 5' terminal oligopyrimidine tract: GTCCCGCAGTCGGCGTCCAGCGGCTCTGCTTGTTCGTGTGTGTGTCGTTGCAGGCCTTATTC; corresponding to SEQ ID NO: 1415 of the patent application WO2013/143700)
or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 16 or more preferably to a corresponding RNA sequence, wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In embodiments, the RNA of the invention comprises a 5'-UTR as described in WO2016/107877. In this context, the disclosure of WO2016/107877 relating to 5'-UTR sequences is herewith incorporated by reference. Particularly preferred 5'-UTRs are nucleic acid sequences according to SEQ ID NOs: 25 to 30 and SEQ ID NOs: 319 to 382 of the patent application WO2016/107877, or fragments or variants of these sequences. In this context, it is particularly preferred that the 5'-UTR of the RNA comprises or consists of a corresponding RNA sequence of the nucleic acid sequence according SEQ ID NOs: 25 to 30 and SEQ ID NOs: 319 to 382 of the patent application WO2016/107877.

In embodiments, the RNA of the invention comprises a 5'-UTR as described in WO2017/036580. In this context, the disclosure of WO2017/036580 relating to 5'-UTR sequences is herewith incorporated by reference. Particularly preferred 5'-UTRs are nucleic acid sequences according to SEQ ID NOs: 1 to 151 of the patent application WO2017/036580, or fragments or variants of these sequences. In this context, it is particularly preferred that the 5'-UTR of the RNA comprises or consists of a corresponding RNA sequence of the nucleic acid sequence according to SEQ ID NOs: 1 to 151 of the patent application WO2017/036580.

Preferably, the at least one 5'UTR element and the at least one 3'UTR element act synergistically to increase protein production from the at least one epitope-encoding RNA of the inventive combination (or any other nucleic acid, in particular RNA, as defined herein) as described above.

### Histone stem-loop

In some preferred embodiments, the epitope-encoding RNA of the inventive combination (or any other nucleic acid, in particular RNA, as defined herein) comprises a histone stem-loop sequence/structure. Such histone stem-loop sequences are preferably selected from histone stem-loop sequences as disclosed in WO 2012/019780, the disclosure of which is incorporated herewith by reference.

A histone stem-loop sequence, suitable to be used within the present invention, is preferably selected from at least one of the following formulae (I) or (II):
formula (I) (stem-loop sequence without stem bordering elements): 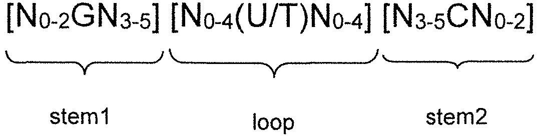
formula (II) (stem-loop sequence with stem bordering elements): 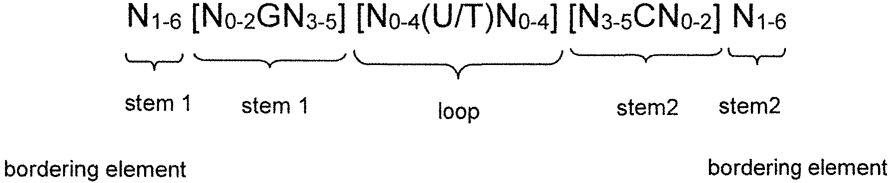
wherein:
   - stem1 or stem2 bordering elements N₁₋₆: is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
   - stem1 [N₀₋₂GN₃₋₅]: is reverse complementary or partially reverse complementary with element stem2, and is a consecutive sequence between of 5 to 7 nucleotides;
   wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof;
   wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
   wherein G is guanosine or an analogue thereof, and may be optionally replaced by a cytidine or an analogue thereof, provided that its complementary nucleotide cytidine in stem2 is replaced by guanosine;
   - loop sequence [N₀₋₄(U/T)N₀₋₄]: is located between elements stem1 and stem2, and is a consecutive sequence of 3 to 5 nucleotides, more preferably of 4 nucleotides;
   wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
   wherein U/T represents uridine, or optionally thymidine;
   - stem2 [N₃₋₅CN₀₋₂]: is reverse complementary or partially reverse complementary with element stem1, and is a consecutive sequence between of 5 to 7 nucleotides;
   wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof;
   wherein N₀₋₂ is a consecutive sequence of 0 to 2, preferably of 0 to 1, more preferably of 1 N, wherein each N is independently from another selected from a nucleotide selected from A, U, T, G or C or a nucleotide analogue thereof; and
   wherein C is cytidine or an analogue thereof, and may be optionally replaced by a guanosine or an analogue thereof provided that its complementary nucleoside guanosine in stem1 is replaced by cytidine;
   - wherein: stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one ore more bases in one stem do not have a complementary base in the reverse complementary sequence of the other stem.

According to a further preferred embodiment, the epitope-encoding RNA of the inventive combination (or any other nucleic acid, in particular RNA, as defined herein) may comprise at least one histone stem-loop sequence according to at least one of the following specific formulae (Ia) or (Ila):
formula (la) (stem-loop sequence without stem bordering elements): 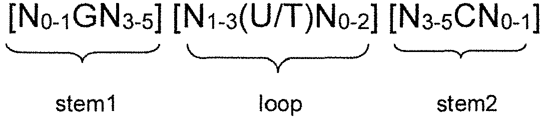
formula (IIa) (stem-loop sequence with stem bordering elements): 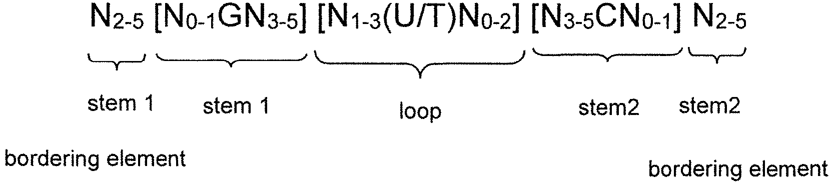
wherein:
   N, C, G, T and U are as defined above.

According to a further more particularly preferred embodiment, the epitope-encoding RNA of the inventive combination (or any other nucleic acid, in particular RNA, as defined herein) may comprise at least one histone stem-loop sequence according to at least one of the following specific formulae (Ib) or (IIb):
formula (Ib) (stem-loop sequence without stem bordering elements): 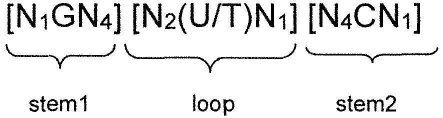
**formula (IIb)** (stem-loop sequence with stem bordering elements): 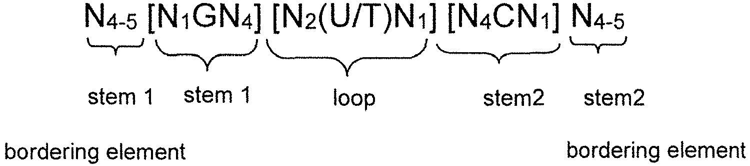
wherein:
   - N, C, G, T and U: are as defined above.

A particularly preferred histone stem-loop sequence is the sequence CAAAGGCTCTTTTCAGAGCCACCA (according to SEQ ID NO: 17) or more preferably the corresponding RNA sequence CAAAGGCUCUUUUCAGAGCCACCA (according to SEQ ID NO: 18).

### Signal peptides

According to preferred embodiments, the epitope-encoding RNA of the inventive combination (or any other nucleic acid, in particular RNA, as defined herein) may additionally or alternatively encode a secretory signal peptide.

Such signal peptides are sequences, which typically exhibit a length of about 15 to 30 amino acids and are preferably located at the N-terminus of the encoded peptide, without being limited thereto. Signal peptides as defined herein preferably allow the transport of the epitope (or the antigen or variant or fragment thereof comprising said epitope) as encoded by the at least one epitope-encoding RNA into a defined cellular compartiment, preferably the cell surface, the endoplasmic reticulum (ER) or the endosomal-lysosomal compartiment.

Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulines, signal sequences of the invariant chain of immunoglobulines or antibodies, signal sequences of Lamp1, Tapasin, Erp57, Calretikulin, Calnexin, PLAT, EPO or albumin and further membrane associated proteins or of proteins associated with the endoplasmic reticulum (ER) or the endosomal-lysosomal compartiment. Most preferably, signal sequences are derived from (human) HLA-A2; (human) PLAT; (human) sEPO; (human) ALB; (human) IgE-leader; (human) CD5; (human) IL2; (human) CTRB2; (human) IgG-HC; (human) Ig-HC; (human) Ig-LC; GpLuc; (human) Igkappa or a fragment or variant of any of the aforementioned proteins, in particular HLA-A2; HsPLAT; sHsEPO; HsALB; HsPLAT(aa1-21); HsPLAT(aa1-22); IgE-leader; HsCD5(aa1-24); HsIL2(aa1-20); HsCTRB2(aa1-18); IgG-HC(aa1-19); Ig-HC(aa1-19); Ig-LC(aa1-19); GpLuc(1-17); Mmlgkappa or a fragment or variant thereof.

Such signal peptides are preferably used in order to promote secretion of an encoded antigen (or variant or fragment thereof), that comprises the epitope as described herein. The RNA sequence encoding said signal peptide is preferably fused to the sequence encoding the antigen (or variant or fragment thereof) comprising said epitope, so that expression of said epitope-coding RNA sequence preferably yields an antigen (or variant or fragment thereof) comprising the epitope, fused to the encoded signal peptide.

Any of the above modifications may be applied to the epitope-encoding RNA of the inventive combination, and further to any nucleic acid, in particular RNA, as used in the context of the present invention and may be, if suitable or necessary, be combined with each other in any combination, provided, these combinations of modifications do not interfere with each other in the respective at least one epitope-encoding RNA (or said other nucleic acid, in particular RNA). A person skilled in the art will be able to take his choice accordingly.

### RNA constructs

The epitope-encoding RNA of the inventive combination, which comprises at least one coding sequence as defined herein (or any other nucleic acid, in particular RNA, as defined herein) may preferably comprise a 5' UTR and/or a 3' UTR optionally containing at least one histone stem-loop.

The 3' UTR of the epitope-encoding RNA of the inventive combination (or any other nucleic acid, in particular RNA, as defined herein) preferably comprises also a poly(A) and/or a poly(C) sequence as defined herein. The single elements of the 3' UTR may occur therein in any order from 5' to 3' along the sequence of the epitope-encoding RNA of the inventive combination (or said other nucleic acid, in particular RNA).

In addition, further elements as described herein, may also be contained, such as a stabilizing sequence as defined herein (e.g. derived from the UTR of a globin gene), IRES sequences, etc. Each of the elements may also be repeated in the epitope-encoding RNA of the inventive combination (or any other nucleic acid, in particular RNA, as defined herein) at least once (particularly in di- or multicistronic constructs), preferably twice or more. As an example, the single elements may be present in the epitope-encoding RNA of the inventive combination (or said other nucleic acid, in particular RNA) in the following order:
5' - coding sequence - histone stem-loop - poly(A)/(C) sequence - 3'; or
5' - coding sequence - poly(A)/(C) sequence - histone stem-loop - 3'; or
5' - coding sequence - histone stem-loop - polyadenylation signal - 3'; or
5' - coding sequence - polyadenylation signal- histone stem-loop - 3'; or
5' - coding sequence - histone stem-loop - histone stem-loop - poly(A)/(C) sequence - 3'; or
5' - coding sequence - histone stem-loop - histone stem-loop - polyadenylation signal- 3'; or
5' - coding sequence - stabilizing sequence - poly(A)/(C) sequence - histone stem-loop - 3'; or
5' - coding sequence - stabilizing sequence - poly(A)/(C) sequence - poly(A)/(C) sequence - histone stem-loop - 3'; etc.

According to further embodiments, the epitope-encoding RNA of the inventive combination (or any other nucleic acid, in particular RNA, as defined herein) preferably comprises at least one of the following structural elements: a 5'- and/or 3'- untranslated region element (UTR element), particularly a 5'-UTR element, which preferably comprises or consists of a nucleic acid sequence which is derived from the 5'-UTR of a TOP gene or from a fragment, homolog or a variant thereof, or a 5'- and/or 3'-UTR element which may preferably be derivable from a gene that provides a stable mRNA or from a homolog, fragment or variant thereof; a histone-stem-loop structure, preferably a histone-stem-loop in its 3' untranslated region; a 5'-cap structure; a poly-A tail; or a poly(C) sequence.

According to some embodiments, it is particularly preferred that - if, in addition to an epitope (or an antigen or a variant or fragment thereof comprising said epitope), a further peptide or protein is encoded by the at least one coding sequence as defined herein - the encoded peptide or protein is preferably no histone protein, no reporter protein (e.g. Luciferase, GFP and its variants (such as eGFP, RFP or BFP), and/or no marker or selection protein, including alpha-globin, galactokinase and Xanthine:Guanine phosphoribosyl transferase (GPT), hypoxanthine-guanine phosphoribosyltransferase (HGPRT), beta-galactosidase, galactokinase, alkaline phosphatase, secreted embryonic alkaline phosphatase (SEAP) or a resistance gene (such as a resistance gene against neomycin, puromycin, hygromycin and zeocin). In preferred embodiments, the epitope-encoding RNA of the inventive combination does not encode a reporter gene or a marker gene. In preferred embodiments, the epitope-encoding RNA of the inventive combination (or any other nucleic acid, in particular RNA, as defined herein) does not encode luciferase. In other embodiments, the epitope-encoding RNA of the inventive combination (or said other nucleic acid, in particular RNA) does not encode GFP or a variant thereof.

According to preferred embodiments, the epitope-encoding RNA of the inventive combination (or any other nucleic acid, in particular RNA, as defined herein) comprises, preferably in 5' to 3' direction, the following elements:
a) a 5'-CAP structure, preferably m7GpppN,
b) at least one coding sequence encoding at least one epitope of an antigen (or a PD-1 and/or LAG-3 inhibitor), or a fragment or variant thereof, as defined herein
c) a poly(A) tail, preferably consisting of 10 to 200, 10 to 100, 40 to 80 or 50 to 70 adenosine nucleotides,
d) optionally a poly(C) tail, preferably consisting of 10 to 200, 10 to 100, 20 to 70, 20 to 60 or 10 to 40 cytosine nucleotides, and
e) optionally a histone stem-loop, preferably comprising the RNA sequence according to SEQ ID NO: 18.

More preferably, the epitope-encoding RNA of the inventive combination (or any other nucleic acid, in particular RNA, as defined herein) comprises, preferably in 5' to 3' direction, the following elements:
a) a 5'-CAP structure, preferably m7GpppN,
b) at least one coding sequence encoding at least one epitope of an antigen (or a PD-1 and/or LAG-3 inhibitor), or a fragment or variant thereof, as defined herein,
c) a 3'-UTR element comprising a nucleic acid sequence, which is derived from an alpha-globin gene, preferably comprising the corresponding RNA sequence of the nucleic acid sequence according to SEQ ID NO: 13, or a homolog, a fragment or a variant thereof,
d) a poly(A) tail, preferably consisting of 10 to 200, 10 to 100, 40 to 80 or 50 to 70 adenosine nucleotides,
e) optionally a poly(C) tail, preferably consisting of 10 to 200, 10 to 100, 20 to 70, 20 to 60 or 10 to 40 cytosine nucleotides, and
f) optionally a histone stem-loop, preferably comprising the RNA sequence according to SEQ ID NO: 18.

In further preferred embodiments, the epitope-encoding RNA of the inventive combination (or any other nucleic acid, in particular RNA, as defined herein) comprises, preferably in 5' to 3' direction, the following elements:
a) a 5'-CAP structure, preferably m7GpppN,
b) a 5'-UTR element, which preferably comprises or consists of a nucleic acid sequence, which is derived from the 5'-UTR of a TOP gene, preferably comprising an RNA sequence corresponding to the nucleic acid sequence according to SEQ ID NO: 15, or a homolog, a fragment or a variant thereof,
c) at least one coding sequence encoding at least one epitope of an antigen (or a PD-1 and/or LAG-3 inhibitor), or a fragment or variant thereof as defined herein,
d) a 3'-UTR element comprising a nucleic acid sequence, which is preferably derived from an alpha-globin gene, preferably comprising the corresponding RNA sequence of the nucleic acid sequence according to SEQ ID NO: 8, or a homolog, a fragment or a variant thereof; and/or
   a 3'-UTR element comprising a nucleic acid sequence, which is derived from an albumin gene, preferably comprising the corresponding RNA sequence of the nucleic acid sequence according to SEQ ID NO: 9, or a homolog, a fragment or a variant thereof,
e) a poly(A) tail, preferably consisting of 10 to 200, 10 to 100, 40 to 80 or 50 to 70 adenosine nucleotides,
f) optionally a poly(C) tail, preferably consisting of 10 to 200, 10 to 100, 20 to 70, 20 to 60 or 10 to 40 cytosine nucleotides, and
g) optionally a histone stem-loop, preferably comprising the RNA sequence according to SEQ ID NO: 18.

In further preferred embodiments, the epitope-encoding RNA of the inventive combination (or any other nucleic acid, in particular RNA, as defined herein) comprises, preferably in 5' to 3' direction, the following elements:
a) a 5'-CAP structure, preferably m7GpppN,
b) a 5'-UTR element, which preferably comprises or consists of a nucleic acid sequence, which is derived from the 5'-UTR of a TOP gene, preferably comprising an RNA sequence corresponding to the nucleic acid sequence according to SEQ ID NO: 16, or a homolog, a fragment or a variant thereof,]
c) at least one coding sequence encoding at least one epitope of an antigen (or a PD-1 and/or LAG-3 inhibitor), or a fragment or variant thereof as defined herein,
d) a poly(A) tail, preferably consisting of 10 to 200, 10 to 100, 40 to 80 or 50 to 70 adenosine nucleotides.

Preferably, the RNA as defined herein comprises at least one coding sequence as defined herein typically comprises a length of about 50 to about 20000, or 500 to about 20000 nucleotides, or about 500 to about 20000 nucleotides, or about 500 to about 10000 nucleotides, or of about 1000 to about 10000 nucleotides, or preferably of about 1000 to about 5000 nucleotides, or even more preferably of about 1000 to about 2500 nucleotides.

According to preferred embodiments, the RNA may be an mRNA, a self-replicating RNA, a circular RNA, or a replicon RNA.

In embodiments, the RNA is a circular RNA. As used herein, "circular RNA" has to be understood as a circular polynucleotide that can encode at least one antigenic peptide or protein as defined herein. Accordingly, in preferred embodiments, said circular RNA comprises at least one coding sequence encoding at least one antigenic peptide or protein derived from YFV or a fragment or variant thereof as defined herein. The production of circRNAs can be performed using various methods provided in the art. For example, US6210931 teaches a method of synthesizing circRNAs by inserting DNA fragments into a plasmid containing sequences having the capability of spontaneous cleavage and self-circularization. US5773244 teaches producing circRNAs by making a DNA construct encoding an RNA cyclase ribozyme, expressing the DNA construct as an RNA, and then allowing the RNA to self-splice, which produces a circRNA free from intron in vitro. WO1992001813 teaches a process of making single strand circular nucleic acids by synthesizing a linear polynucleotide, combining the linear nucleotide with a complementary linking oligonucleotide under hybridization conditions, and ligating the linear polynucleotide. The person skilled in the art may also use methods provided in WO2015034925 or WO2016011222 to produce circular RNA. Accordingly, methods for producing circular RNA as provided in US6210931, US5773244, WO1992001813, WO2015034925 and WO2016011222 are incorporated herewith by reference.

In embodiments, the RNA is a replicon RNA. The term "replicon RNA" will be recognized and understood by the person of ordinary skill in the art, and are for example intended to be optimized self-replicating artificial RNA constructs. Such constructs include replication elements (replicase) derived from alphaviruses and the substitution of the structural virus proteins with the artificial nucleic acid of interest (in the context of the invention, an artificial nucleic acid comprising at least one coding sequence encoding at least one antigenic peptide or protein derived from YFV. Alternatively, the replicase may be provided on an independent construct comprising a replicase RNA sequence derived from e.g. Semliki forest virus (SFV), Sindbis virus (SIN), Venezuelan equine Encephalitis virus (VEE), Ross-River virus (RRV), or other viruses belonging to the alphavirus family. Downstream of the replicase lies a sub-genomic promoter that controls replication of the artificial nucleic acid of the invention, i.e. an artificial nucleic acid comprising at least one coding sequence encoding at least one antigenic peptide or protein derived from YFV.

In preferred embodiments, the RNA of the invention is an mRNA.

The RNA, preferably the mRNA of the invention may be prepared using any method known in the art, including chemical synthesis such as e.g. solid phase RNA synthesis, as well as in vitro methods, such as RNA in vitro transcription reactions.

In a preferred embodiment, the RNA, preferably the mRNA is obtained by RNA in vitro transcription. Accordingly, the RNA of the invention is preferably an in vitro transcribed RNA.

The terms "RNA in vitro transcription" or "in vitro transcription" relate to a process wherein RNA is synthesized in a cell-free system (in vitro). RNA may be obtained by DNA-dependent in vitro transcription of an appropriate DNA template, which according to the present invention is a linearized plasmid DNA template or a PCR-amplified DNA template. The promoter for controlling RNA in vitro transcription can be any promoter for any DNA-dependent RNA polymerase. Particular examples of DNA-dependent RNA polymerases are the T7, T3, SP6, or Syn5 RNA polymerases. In a preferred embodiment of the present invention the DNA template is linearized with a suitable restriction enzyme, before it is subjected to RNA in vitro transcription.

Reagents used in RNA in vitro transcription typically include: a DNA template (linearized plasmid DNA or PCR product) with a promoter sequence that has a high binding affinity for its respective RNA polymerase such as bacteriophage-encoded RNA polymerases (T7, T3, SP6, or Syn5); ribonucleotide triphosphates (NTPs) for the four bases (adenine, cytosine, guanine and uracil); optionally, a cap analogue as defined herein (e.g. m7G(5')ppp(5')G (m7G)); optionally, further modified nucleotides as defined herein; a DNA-dependent RNA polymerase capable of binding to the promoter sequence within the DNA template (e.g. T7, T3, SP6, or Syn5 RNA polymerase); optionally, a ribonuclease (RNase) inhibitor to inactivate any potentially contaminating RNase; optionally, a pyrophosphatase to degrade pyrophosphate, which may inhibit RNA in vitro transcription; MgCl2, which supplies Mg2+ ions as a co-factor for the polymerase; a buffer (TRIS or HEPES) to maintain a suitable pH value, which can also contain antioxidants (e.g. DTT), and/or polyamines such as spermidine at optimal concentrations, e.g. a buffer system comprising TRIS-Citrate as disclosed in WO2017/109161.

In embodiments, the nucleotide mixture used in RNA in vitro transcription may additionally contain modified nucleotides as defined herein. In that context, preferred modified nucleotides comprise pseudouridine (ψ), N1- methylpseudouridine (m1ψ), 5-methylcytosine, and 5-methoxyuridine.

In preferred embodiments, the nucleotide mixture (i.e. the fraction of each nucleotide in the mixture) used for RNA in vitro transcription reactions may be optimized for the given RNA sequence, preferably as described WO2015/188933.

In embodiment where more than one different RNA as defined herein has to be produced, e.g. where 2, 3, 4, 5, 6, 7, 8, 9, 10 or even more different artificial RNAs have to be produced (e.g. encoding different YFV prME antigens), procedures as described in WO2017/109134 may be suitably used.

In the context of RNA vaccine production, it may be required to provide GMP-grade RNA. GMP-grade RNA may be produced using a manufacturing process approved by regulatory authorities. Accordingly, in a particularly preferred embodiment, RNA production is performed under current good manufacturing practice (GMP), implementing various quality control steps on DNA and RNA level, preferably according to WO2016/180430. In preferred embodiments, the RNA of the invention is a GMP-grade RNA, particularly a GMP-grade mRNA.

The obtained RNA products are preferably purified using PureMessenger^{®} (CureVac, Tübingen, Germany; RP-HPLC according to WO2008/077592) and/or tangential flow filtration (as described in WO2016/193206).

In a further preferred embodiment, the RNA, particularly the purified RNA, is lyophilized according to WO2016/165831 or WO2011/069586 to yield a temperature stable dried artificial RNA (powder) as defined herein. The RNA of the invention, particularly the purified RNA may also be dried using spray-drying or spray-freeze drying according to WO2016/184575 or WO2016184576 to yield a temperature stable RNA (powder) as defined herein. Accordingly, in the context of manufacturing and purifying RNA, the disclosures of WO2017/109161, WO2015/188933, WO2016/180430, WO2008/077592, WO2016/193206, WO2016/165831, WO2011/069586, WO2016/184575, and WO2016184576 are incorporated herewith by reference.

Accordingly, in preferred embodiments, the RNA is a dried RNA, particularly a dried mRNA.

The term "dried RNA" as used herein has to be understood as RNA that has been lyophilized, or spray-dried, or spray-freeze dried as defined above to obtain a temperature stable dried RNA (powder).

In preferred embodiments, the artificial RNA of the invention is a purified RNA, particularly purified mRNA.

The term "purified RNA" or "purified mRNA" as used herein has to be understood as RNA which has a higher purity after certain purification steps (e.g. HPLC, TFF, Oligo d(T) purification, precipitation steps) than the starting material (e.g. in vitro transcribed RNA). Typical impurities that are essentially not present in purified RNA comprise peptides or proteins (e.g. enzymes derived from DNA dependent RNA in vitro transcription, e.g. RNA polymerases, RNases, pyrophosphatase, restriction endonuclease, DNase), spermidine, BSA, abortive RNA sequences, RNA fragments (short double stranded RNA fragments, abortive sequences etc.), free nucleotides (modified nucleotides, conventional NTPs, cap analogue), template DNA fragments, buffer components (HEPES, TRIS, MgCl2) etc. Other potential impurities that may be derived from e.g. fermentation procedures comprise bacterial impurities (bioburden, bacterial DNA) or impurities derived from purification procedures (organic solvents etc.). Accordingly, it is desirable in this regard for the "degree of RNA purity" to be as close as possible to 100%. It is also desirable for the degree of RNA purity that the amount of full length RNA transcripts is as close as possible to 100%. Accordingly "purified RNA" as used herein has a degree of purity of more than 75%, 80%, 85%, very particularly 90%, 91%, 92%, 93%, 94%, 95%, 96%, 97%, 98% and most favorably 99% or more. The degree of purity may for example be determined by an analytical HPLC, wherein the percentages provided above correspond to the ratio between the area of the peak for the target RNA and the total area of all peaks representing the by-products. Alternatively, the degree of purity may for example be determined by an analytical agarose gel electrophoresis or capillary gel electrophoresis.

It has to be understood that "dried RNA" as defined herein and "purified RNA" as defined herein or "GMP-grade mRNA" as defined herein may have superior stability characteristics (in vitro, in vivo) and improved efficiency (e.g. better translatability of the mRNA in vivo) and are therefore particularly suitable in the context of the invention.

Apart from the at least one epitope-encoding RNA (as defined above), the inventive combination further comprises at least one PD-1 pathway inhibitor and at least one LAG-3 pathway inhibitor. Said pathway inhibitors are described in greater detail below.

### Inhibitors

### PD-1 pathway

Programmed Death-1 (PD-1, PDCD1) is a type I transmembrane protein belonging to the extended CD28 family of T cell regulators. PD-1 lacks the membrane-proximal cysteine residue required for homodimerization of other members of the CD28 family. Structural and biochemical analyses showed that PD-1 is monomeric in solution as well as on the cell surface (Okazaki and Honjo, 2007. Int Immunol. 19(7):813-24). PD-1 is expressed on activated T cells, B cells and monocytes. The broader expression of PD-1 contrasts with restricted expression of other CD28 family members to T cells, suggesting that PD-1 regulates a wider spectrum of immune responses compared with other CD28 family members.

PD-1 negatively regulates antigen receptor signaling by recruiting the protein tyrosine phosphatase SHP-2 upon interacting with either of two ligands, PD-L1 or PD-L2.

PD-L1 (B7-H1, CD274) and PD-L2 (B7-DC, CD273) are type I transmembrane glycoproteins composed of IgC-and IgV-type extracellular domains. PD-L1 and PD-L2 share 40% amino acid identity while human and mouse orthologs of PD-L1 and PD-L2 share 70% amino acid identity. Both PD-L1 and PD-L2 have short cytoplasmic tails with no known motif for signal transduction, suggesting that these ligands do not transduce any signal upon interaction with PD-1.

The interaction of PD-L1 and PD-1 provides a crucial negative co-stimulatory signal to T cells and functions as cell death inducer. Interaction between low concentration of PD-1 and PD-L1 leads to the transmission of an inhibitory signal that inhibits the proliferation of antigen-specific CD8+ cells. At higher concentrations this interaction does not inhibit T cell proliferation but reduces the production of multiple cytokines. Thus, binding to PD-L1 can antagonize the B7 - CD28 signal when antigenic stimulation is weak and plays a key role in downregulating T cell responses.

The role of PD-1 and PD-1 ligands in inhibiting T cell activation and proliferation suggested that these proteins may serve as therapeutic targets for treatments of inflammation, cancer or infectious diseases. Depending on the desired therapeutic outcome, an up- or down-modulation of the PD-1 pathway is required. Up-modulation of the immune system is particularly required in the treatment of cancers and chronic infections. This can be achieved for example by PD-1 blockade or inhibiting the PD-1 pathway. Inhibition of the PD-1 pathway can be achieved, for example, by an antibody directed at PD-1 or a PD-1 ligand. In this context the PD-1 pathway inhibitor may reverse T cell exhaustion resulting from PD-1 signalling and thereby restore or enhance T cell function (e.g. proliferation, cytokine production, target cell killing). In addition, anergic T cells which are unresponsive to antigen stimulation may be reactivated.

Members of the PD-1 pathway are all proteins which are associated with PD-1 signalling. On the one hand these might be proteins which induce PD-1 signalling upstream of PD-1 as e.g. the ligands of PD-1 PD-L1 and PD-L2 and the signal transduction receptor PD-1. On the other hand these might be signal transduction proteins downstream of PD-1 receptor. Particularly preferred as members of the PD-1 pathway in the context of the present invention are PD-1, PD-L1 and PD-L2.

In the context of the present invention, a "PD-1 pathway inhibitor" is preferably defined herein as a compound capable to impair the PD-1 pathway signaling, preferably signaling mediated by the PD-1 receptor. Therefore, the PD-1 pathway inhibitor may be any inhibitor directed against any member of the PD-1 pathway capable of impairing (i.e. reducing, inhibiting, preventing) PD-1 pathway signaling. PD-1 pathway inhibitors may act intra-cellularly (e.g. by interfering with intra-cellular signalling components of the PD-1 pathway that are typically involved in signalling induced by binding of PD-1 to its respective ligand(s)) or extracellularly (e.g. by interfering with PD-1 or its respective ligand(s). In this context, "interfering" in all its grammatical terms means "interacting with", "modulating" or "altering" to the extent that the respective signalling pathway is preferably impaired.

In this context, the inhibitor may be an antagonistic antibody as defined herein, targeting any member of the PD-1 pathway, preferably the PD-1 receptor, or its ligands PD-L1 or PD-L2. The PD-1 pathway inhibitor may be a nucleic acid encoding a polypeptide PD-1 pathway inhibitor, in particular an antagonistic antibody. Or, the PD-1 pathway inhibitor may be an antagonistic binding protein, e.g. a soluble PD-1 receptor or a fusion protein, or a nucleic acid encoding said antagonistic binding protein. PD-L1 or PD-L2 or fragments or derivatives thereof may be used as PD1 pathway inhibitors as well. Nucleic acids encoding PD-L1 or PD-L2 or fragments or derivatives thereof are also envisaged as PD-1 pathway inhibitors herein. Furthermore, the PD-1 pathway inhibitor may be an antagonistic nucleic acid, such as a siRNA (small interfering RNA) shRNA, miRNA or any other antisense RNA directed against a member of the PD-1 pathway, preferably PD-1, PD-L1 or PD-L2. Additionally, a PD-1 pathway inhibitor may be a small molecule inhibitor capable of inhibiting PD-1 pathway signaling, e.g. a PD-1 binding peptide or a small organic molecule.

Preferably, the PD-1 pathway inhibitor is thus selected from an antagonistic antibody as defined herein or a nucleic acid encoding said antibody, an antagonistic binding protein as defined herein or a nucleic acid encoding said antagonistic binding protein, a peptide or a nucleic acid encoding said peptide, an antagonistic nucleic acid or a small organic molecule.

### LAG-3 pathway

LAG-3 (also referred to as CD223) is a member of the immunoglobulin superfamily (IgSF) and exerts a wide variety of biologic impacts on T cell function. LAG-3 is a transmembrane protein with structural homology to CD4 and includes four extracellular IgG domains. The membrane-distal IgG domain contains a short amino acid sequence, the so-called extra loop that is not found in other IgG superfamily proteins. LAG-3 has been demonstrated to bind to Class II MHC with high affinity primarily through a small set of amino acids localized to the D1 domain- this is in sharp contrast to CD4 which interacts with Class II MHC through a rather large surface involving multiple residues. The intracellular domain of LAG-3 is relatively short and contains a unique amino acid sequence (KIEELE) that is required for LAG-3-mediated modulation of T cell function.

LAG-3 is one of the various immune-checkpoint receptors and is expressed on cell membranes of natural killer cells (NK), B cells, T cells, and dendritic cells (DC). LAG-3 is in many ways a T cell activation marker, expressed on both CD4+ and CD8+ T cells 3-4 days post activation. The LAG-3/MHC class II molecule interaction reportedly down-regulates antigen-dependent, but not antigen-independent, stimulation of CD4⁺ T lymphocytes. It has been further demonstrated to mitigate *in vitro* and *in vivo* expansion of both CD4+ and CD8+ T cells, thus confirming its role as a negative regulator. Further revealed that the KIEELE domain plays a critical role in the negative regulatory function of LAG-3; i.e., LAG-3 molecules lacking this domain could not negatively modulate T cell function *in vitro* or *in vivo* (Goldberg and Drake, Curr Top Microbiol Immunol. 2011; 344: 269-278 and He et al. Cancer Sci. 2016 Sep; 107(9): 1193-1197).

As a negative regulator of T cell mediated immune responses, LAG-3 can reduce the body"s ability to resist infection or combat cancer. Therefore, impairment of LAG-3 mediated signaling is envisaged to enhance immune responses against infectious pathogens and malignant cells. LAG-3 pathway inhibitors described herein preferably act to reverse T cell exhaustion and anergy resulting from LAG-3 signalling, thereby restoring or enhancing T cell function (e.g. proliferation, stimulation, cytokine production, target cell killing) in cancer or infectious diseases.

Members of the LAG-3 pathway are all proteins which are associated with LAG-3 signaling. On the one hand these might be proteins which induce LAG-3 signaling upstream of LAG-3 as e.g. the MHC-II as a LAG-3 ligand or LAG-3 itself. On the other hand these might be signal transduction proteins downstream of the LAG-3 receptor. Particularly preferred as members of the LAG-3 pathway in the context of the present invention is LAG-3.

In the context of the present invention, a "LAG-3 pathway inhibitor" is preferably defined herein as a compound capable to impair the LAG-3 pathway signaling, preferably signaling mediated by the LAG-3 receptor. Therefore, the LAG-3 pathway inhibitor may be any inhibitor directed against any member of the LAG-3 pathway capable of impairing (i.e. reducing, inhibiting, preventing) LAG-3 pathway signaling. LAG-3 pathway inhibitors may act intra-cellularly (e.g. by interfering with intra-cellular signalling components of the LAG-3 pathway that are typically involved in signalling induced by binding of LAG-3 to its respective ligand(s)) or extracellularly (e.g. by interfering with LAG-3 or its respective ligand(s)). In this context, "interfering" in all its grammatical terms means "interacting with", "modulating" or "altering" to the extent that the respective signalling pathway is preferably impaired.

In this context, the inhibitor may be an antagonistic antibody as defined herein, targeting any member of the LAG-3 pathway, preferably the LAG-3 receptor, or its ligand MHC-II. The LAG-3 pathway inhibitor may be a nucleic acid encoding a polypeptide LAG-3 pathway inhibitor, in particular an antagonistic antibody. Also, the LAG-3 pathway inhibitor may be an antagonistic binding protein, e.g. a soluble LAG-3 receptor or a fusion protein, or a nucleic acid encoding such an antagonistic binding protein. MHC-II or fragments or derivatives thereof may act as PD1-inhibiting ligands as well. Nucleic acids encoding MHC-II or fragments or derivatives thereof are also envisaged herein as LAG-3 pathway inhibitors. Furthermore, the LAG-3 pathway inhibitor may be an antagonistic nucleic acid, such as a siRNA (small interfering RNA) shRNA, miRNA or any other antisense RNA directed against a member of the LAG-3 pathway, preferably LAG-3 or MHC-II. Additionally, a LAG-3 pathway inhibitor may be a small molecule inhibitor capable of inhibiting LAG-3 pathway signaling, e.g. a LAG-3 binding peptide or a small organic molecule.

Preferably, the LAG-3 pathway inhibitor is thus selected from an antagonistic antibody as defined herein or a nucleic acid encoding said antibody, an antagonistic binding protein as defined herein or a nucleic acid encoding said antagonistic binding protein, a peptide or a nucleic acid encoding said peptide, an antagonistic nucleic acid or a small organic molecule.

### PD-1 and LAG-3 pathway inhibitor types

As indicated above, the PD-1 pathway inhibitor and/or the LAG-3 pathway inhibitor comprised in the inventive combination may be independently selected from an antibody (or a nucleic acid encoding said antibody), a protein (or a nucleic acid encoding said protein), a peptide (or a nucleic acid encoding said peptide), a nucleic acid, and a small organic molecule. In the following, the present disclosure may commonly refer to PD-1 pathway inhibitors and LAG-3 pathway inhibitors as "pathway inhibitors" or "inhibitors". Usually -and unless denoted otherwise- when referring to "pathway inhibitors" or "inhibitors", the disclosure relates to PD-1 pathway inhibitors, LAG-3 pathway inhibitors or both.

The PD-1 pathway inhibitor is preferably capable of impairing (i.e. reducing, inhibiting, preventing) PD-1 pathway signalling, preferably signalling mediated by PD-1. It may act as a competitive or non-competitive PD-1 antagonist. The LAG-3 pathway inhibitor is preferably capable of impairing (i.e. reducing, inhibiting, preventing) LAG-3 signalling, preferably signalling mediated by LAG-3. It may function as a competitive or non-competitive LAG-3 antagonist.

In this context, an "antagonist" is a substance that inhibits or reduces agonist-mediated biological responses by binding to a target receptor; whereas an "agonist" is a substance that binds to a target receptor and triggers a biological response. In this regard, a "Competitive antagonists" bind to but do not activate their target receptor. They typically compete with available agonists for (active) binding sites and are thus capable of displacing the agonist from said binding sites (particularly if present at sufficient amounts), resulting in a lower frequency of target receptor activation. Competitive antagonists include reversible competitive antagonist (binding to their target receptor via non-covalent interactions) or irreversible competitive antagonist (binding to their target receptor permanently via covalent interactions). "Non-competitive antagonists" bind to allosteric sites (i.e. a binding site that is different from the active site) of their target receptor. Thus, non-competitive antagonists typically do not compete with agonists for binding at the active site. Once bound, such antagonist may induce or prevent conformational changes in the target receptor resulting in impaired receptor-mediated signaling upon agonist binding.

### Antibodies and nucleic acids encoding the same

In preferred embodiments, pathway inhibitors of the inventive combination are independently selected from an antibody, or a variant, fragment or derivative thereof. Said antibody may be selected from a human, humanized, chimeric, monoclonal or polyclonal antibody, an antibody heavy chain and/or an antibody light chain. In further preferred embodiments, pathway inhibitors of the inventive combination are selected from nucleic acids encoding such antibodies, variants, fragments or derivatives.

### Antibodies

An "antibody" may be selected from any antibody, e.g. any recombinantly produced or naturally occurring antibodies, known in the art, in particular antibodies suitable for therapeutic, diagnostic or scientific purposes, particularly directed against PD-1, PD-L1 or PD-L2 (for PD-1 pathway inhibitors) or LAG-3 (for LAG-3 pathway inhibitors). Herein, the term "antibody" is used in its broadest sense and specifically covers monoclonal and polyclonal antibodies (including, antagonist, and blocking or neutralizing antibodies) and antibody species with polyepitopic specificity. According to the invention, "antibody" typically comprises any antibody known in the art (e.g. IgM, IgD, IgG, IgA and IgE antibodies), such as naturally occurring antibodies, antibodies generated by immunization in a host organism, antibodies which were isolated and identified from naturally occurring antibodies or antibodies generated by immunization in a host organism and recombinantly produced by biomolecular methods known in the art, as well as chimeric antibodies, human antibodies, humanized antibodies, bispecific antibodies, intrabodies, i.e. antibodies expressed in cells and optionally localized in specific cell compartments, and fragments and variants of the aforementioned antibodies. In general, an antibody consists of a light chain and a heavy chain both having variable and constant domains. The light chain consists of an N-terminal variable domain, VL, and a C-terminal constant domain, CL. In contrast, the heavy chain of the IgG antibody, for example, is comprised of an N-terminal variable domain, VH, and three constant domains, CH1, CH2 und CH3. Single chain antibodies may be used according to the present invention as well.

Antibodies may preferably comprise full-length antibodies, i.e. antibodies composed of the full heavy and full light chains, as described above. However, derivatives of antibodies such as antibody fragments, variants or adducts may also be used as PD-1 pathway inhibitors and/or LAG-3 pathway inhibitors according to the invention. Antibody fragments may be selected from Fab, Fab", F(ab")₂, Fc, Facb, pFc", Fd, Fd" and Fv fragments of the aforementioned (full-length) antibodies, In general, antibody fragments are known in the art. For example, a Fab ("fragment, antigen binding") fragment is composed of one constant and one variable domain of each of the heavy and the light chain. The two variable domains bind the epitope on specific antigens. The two chains are connected via a disulfide linkage. A scFv ("single chain variable fragment") fragment, for example, typically consists of the variable domains of the light and heavy chains. The domains are linked by an artificial linkage, in general a polypeptide linkage such as a peptide composed of 15-25 glycine, proline and/or serine residues.

The term "polyclonal antibody" typically refers to mixtures of antibodies directed to specific antigens or immunogens or epitopes of a protein which were generated by immunization of a host organism, such as a mammal, e.g. including goat, cattle, swine, dog, cat, donkey, monkey, ape, a rodent such as a mouse, hamster and rabbit. Polyclonal antibodies are generally not identical, and thus usually recognize different epitopes or regions from the same antigen. Thus, in such a case, typically a mixture (a composition) of different antibodies will be used, each antibody being directed to specific antigens or immunogens or epitopes of a protein, particularly directed to PD-1, PD-L1 or PD-L2 (in case of a PD-1 pathway inhibitor) or LAG-3 (in case of a LAG-3 pathway inhibitor).

The term "monoclonal antibody" herein typically refers to an antibody obtained from a population of substantially homogeneous antibodies, i.e., the individual antibodies comprising the population are identical except for possible naturally-occurring mutations that may be present in minor amounts. Monoclonal antibodies are highly specific, being directed to a single antigenic site. Furthermore, in contrast to conventional (polyclonal) antibody preparations which typically include different antibodies directed to different determinants (epitopes), each monoclonal antibody is directed to a single determinant on the antigen. For example, monoclonal antibodies as defined above may be made by the hybridoma method first described by Kohler and Milstein, Nature, 256:495 (1975), or may be made by recombinant DNA methods, e.g. as described in U.S. Pat. No. 4,816,567. "Monoclonal antibodies" may also be isolated from phage libraries generated using the techniques described in McCafferty et al., Nature, 348:552-554 (1990), for example. According to Kohler and Milstein, an immunogen (antigen) of interest is injected into a host such as a mouse and B-cell lymphocytes produced in response to the immunogen are harvested after a period of time. The B-cells are combined with myeloma cells obtained from mouse and introduced into a medium which permits the B-cells to fuse with the myeloma cells, producing hybridomas. These fused cells (hybridomas) are then placed into separate wells of microtiter plates and grown to produce monoclonal antibodies. The monoclonal antibodies are tested to determine which of them are suitable for detecting the antigen of interest. After being selected, the monoclonal antibodies can be grown in cell cultures or by injecting the hybridomas into mice. In the context of the present invention particularly preferred are monoclonal antibodies directed against PD-1, PD-L1 and PD-L2 (in case of PD-1 pathway inhibitors) or against LAG-3 (in case of LAG-3 pathway inhibitors).

"Chimeric antibodies" are preferably antibodies in which the constant domains of an antibody described above are replaced by sequences of antibodies from other organisms, preferably human sequences.

"Humanized (non-human) antibodies" are antibodies in which the constant and variable domains (except for the hypervariable domains) of an antibody are replaced by human sequences.

"Human antibodies" can be isolated from human tissues or from immunized non-human host organisms which are transgene for the human IgG gene locus. Additionally, human antibodies can be provided by the use of a phage display.

"Bispecific antibodies" in context of the invention are preferably antibodies which act as an adaptor between an effector and a respective target by two different antigen-binding domains, e.g. for the purposes of recruiting effector molecules such as toxins, drugs, cytokines etc., targeting effector cells such as CTL, NK cells, makrophages, granulocytes, etc. (see for review: Kontermann R.E., Acta Pharmacol. Sin, 2005, 26(1): 1-9). Bispecific antibodies as described herein are, in general, configured to recognize by two different antigen-binding domains, e.g., two different antigens, immunogens, epitopes, drugs, cells (or receptors on cells), or other molecules (or structures) as described herein. "Bispecific" or "bispecificity" means that the two antigen-binding regions of the antibodies are specific for two different epitopes. Thus, different antigens, immunogens or epitopes, etc. can be brought close together, what, optionally, allows a direct interaction of the two components. For example, different cells such as effector cells and target cells can be connected via a bispecific antibody. Encompassed by the present invention are bispecific antibodies or fragments thereof which bind, e.g. PD-1 and/or its ligands PD-L1 and PD-L2 (in case of PD-1 pathway inhibitors) and/or LAG-3 (in case of LAG-3 pathway inhibitors) on the surface of a cell, e.g. a tumor cell, provided that said interaction preferably results in impairment of the PD-1 or LAG-3 signaling pathway.

"Trispecific antibodies" in context of the invention are preferably antibodies which act as an adaptor between an effector and two respective targets by three different antigen-binding domains, e.g. for the purposes of recruiting effector molecules such as toxins, drugs, cytokines etc., targeting effector cells such as CTL, NK cells, makrophages, granulocytes, etc. (see for review: Kontermann R.E., Acta Pharmacol. Sin, 2005, 26(1): 1-9). Trispecific antibodies as described herein are, in general, configured to recognize by three different antigen-binding domains, e.g., three different antigens, immunogens, epitopes, drugs, cells (or receptors on cells), or other molecules (or structures) as described herein. "Trispecific" or "trispecificity" means that the three different antigen-binding regions of the antibodies are specific for three different epitopes. Thus, different antigens, immunogens or epitopes, etc. can be brought close together, what, optionally, allows a direct interaction of the two components. For example, different cells such as effector cells and target cells can be connected via a trispecific antibody. Encompassed by the present invention are bispecific antibodies or fragments thereof which bind, e.g. PD-1 and/or its ligands PD-L1 and PD-L2 (in case of PD-1 pathway inhibitors) and/or LAG-3 (in case of LAG-3 pathway inhibitors) on the surface of a cell, e.g. a tumor cell, provided that said interaction preferably results in impairment of the PD-1 or LAG-3 signaling pathway.

Particularly preferred antibodies used as PD-1 and/or LAG-3 inhibitors in the inventive combination are bi- or trispecific antibodies which recognize two or three different checkpoint molecules, e.g. PD-1, PD-L1, PD-L2, LAG-3, TIM-3, B7-H3 (also known as CD276) and B7-H4 (also known as B7-S1, B7x and VCTN1) CTLA4, A2aR, etc., reviewed *inter alia* by Pardoll DM Nat Rev Cancer. 2012 Mar 22;12(4):252-64.

Antibodies are preferably capable of specifically binding to their target epitope(s). The term "specifically binding" as used herein means that an agent (e.g. an antibody) binds more readily to its intended target (i.e. the epitope recognized by said antibody) than to a different, non-specific target. In other words, the agent (e.g. antibody) specifically binds to its target if it preferentially binds or recognizes the target even in the presence of other, non-target entities as measurable by a quantifiable assay. For instance, binding specificity can be determined by various ligand binding assays such as Radioactive Ligand Binding Assays, ELISA, fluorescence based techniques (e.g. Fluorescence Polarization (FP), Fluorescence Resonance Energy Transfer (FRET)), or surface plasmon resonance. Preferably, agents that specifically bind to a target do not (significantly) cross-react with other non-target entities.

### Nucleic acids encoding antibodies

PD-1 pathway inhibitors and/or the LAG-3 pathway inhibitors described herein may independently from each other be selected from a nucleic acid encoding an antibody as described herein, or a fragment, variant or derivative thereof.

Such nucleic acids are preferably selected from a DNA, e.g. viral DNA, plasmid DNA, a PCR product, a cDNA or an RNA, more preferably an RNA, e.g. viral RNA, replicon RNA, mRNA, and most preferably an mRNA, and comprise at least one coding sequence that encodes the respective antibody (or antibody fragment, variant or derivative) and optionally regulatory elements driving its expression. Such nucleic acids comprise a transcription initiation site, an open reading frame encoding the antibody (or antibody fragment, variant or derivative) and a transcription termination site. As indicated above, said nucleic acids, in particular RNAs, any modification, design or embodiment described in the context of the epitope-encoding RNAs are equally applicable to the antibody-encoding nucleic acids described herein, *mutatis mutandis.*

Thus, the at least one coding sequence of said encoding nucleic acid may encode an antibody or a fragment, variant or derivative thereof. Specifically, the at least one coding sequence of the nucleic acids may encode (a) an antibody as described herein, (b) an antibody variant, in particular a sequence variant of the antibodies described herein, (c) a fragment of an antibody (or variant thereof) as described herein, in particular an antigen-binding fragment such as a Fab, Fab", F(ab")2, Facb, Fab/c, Fd, Fd", Fv, heavy chain antibodies, sdAb (nanobodies) or (d) a derivative of such an antibody (or variant thereof) or fragment of said antibody (or variant thereof), in particular an antigen-binding derivative, including scFv, scFv", scFv dimers (diabodies), scFv trimers (triabodies), or minibodies.

Below, some preferred examples of antibodies suitable for use as PD-1 or LAG-3 pathway inhibitors are provided. Said antibodies are thus envisaged as PD-1 or LAG-3 pathway inhibitors in the inventive combination. Variants, in particular sequence variants or glycosylation variants, of said antibodies are also envisaged. A "sequence variant" of an antibody is an antiobdy comprising an altered amino acid sequence as compared to a "reference" (or "parent") antibody. Antibody sequence variants are envisaged to comprise or consist of an amino acid sequence which is preferably at least 75%, preferably at least 80%, preferably at least 85%, preferably at least 90%, more preferably at least 95%, more preferably at least 96%, more preferably at least 97% identical to the amino acid sequence of the parent antibody. Antibody sequence variants may comprise at least one amino acid modification, such as a deletion, substitution or insertion as compared to the amino acid sequqence of the parent antibody. Amino acid modifications may occur in the variable or constant regions of the antibody, including the complementarity determining regions (CDRs), the framework regions or the Fc part. Often, conservative amino acid substitution(s) are preferred. The term "antibody variant" further includes "glycosylation variants" comprising different glycosylation patterns as compared to the parent antibody. The term "antibody variant" further includes antibodies comprising covalent modifications as compared to the respective parent antibody. Such covalent modifications include, for instance, phosphorylation, S-nitrosylation, methylation, N-acetylation, lipidation, disulfide bond formation, sulfation, acylation and deamination of amino acids. Antibody sequence variants preferably exhibit antigen-binding properties (e.g. binding affinity, binding specificity) that are comparable or even improved as compared to the respective parent antibodies.

Further, fragments of said antibodies (or antibody variants) may be used as pathway inhibitors. An "antibody fragment" is a part or portion of said antibody. Preferably, antibody fragments used as pathway inhibitors are antigen-binding fragments, and preferably retain the antigen-binding properties of the full-length antibodies. Further, derivatives of said antibodies (or variants or fragments thereof) may be used as pathway inhibitors. Antibody derivatives comprise at least one antibody (or variant thereof) or a fragment of said antibody linked to a moiety which peferably confers a new or additional functionality, optionally via a suitable linker. Antibody derivatives may comprise two or more antibody fragments. Said derivatives preferably exhibit antigen-binding properties (e.g. binding affinity, binding specificity) that are comparable or even improved as compared to the respective parent antibodies.

### PD-1 pathway inhibitor antibodies

In preferred embodiments, the PD-1 pathway inhibitor is an antibody or a nucleic acid encoding such an antibody.

Antibodies used as PD-1 pathway inhibitors are preferably capable of specifically binding to their respective target (i.e. a member of the PD-1 pathway, including PD-1, PD-L1 or PD-L2) and inhibit PD-1 pathway signalling.

Such antibodies are thus envisaged to specifically bind to PD-1 (in particular to its extracellular domain), PD-L1 or PD-L2; for instance in a way so that receptor-ligand interactions between PD-1 and PD-L1 or PD-L2 are prevented or disrupted, thereby impairing PD-1 pathway signalling. E.g., PD-1 pathway inhibitor antibodies may bind proximal to binding sites required for receptor ligand interaction. By sterically hindering receptor ligand interaction, and/or displacing the PD1 ligands from their active binding sites at the PD-1 receptor, PD-1 mediated signalling may be prevented or disrupted. E.g., such an antagonistic antibody may bind close to the PD-L1 binding site on PD-1, thus inhibiting the binding of PD-L1 to PD-1.

Preferred antibodies used as PD-1 pathway inhibitors include
(a) the anti-PD-1 antibodies Nivolumab (also referred to as MDX-1106, BMS-936558, ONO-4538, trade name OPDIVO^{®}, CAS Number 946414-94-4) (Brahmer et al., 2010. J Clin Oncol. 28(19):3167-75),; Pidilizumab (also referred to as CT-011) (Berger et al., 2008. Clin Cancer Res. 14(10):3044-51),; Pembrolizumab (also referred to as MK-3475, trade name KEYTRUDA^{®}, CAS Number 1374853-91-4) (, Poole RM Drugs. 2014;74(16):1973-81);, PF-06801591 (ClinicalTrials.gov identifier: NCT02573259),; mDX-400 (Merck & Co), BGB-A317 (Desai, J Clin Oncol 34, 2016 (suppl; abstr 3066),; MEDI0680 (also referred to as AMP-514) (ClinicalTrials.gov Identifier: NCT02013804, ),; PDR001 (ClinicalTrials.gov Identifier: NCT02678260),; Spartalizumab (Novartis AG, CAS Number 1935694-88-4), Cemiplimab (REGN2810, CAS Number 1801342-60-8, (Falchook et al. J Immunother Cancer. 2016 Nov;4:70),; Pidilizumab (Pfizer, CAS Number 1036730-42-3), SHR-1210 (Incyte Corp, Jiangsu Hengrui Medicine Co Ltd, ClinicalTrials.gov Identifier: NCT02742935), TSR-042 (ClinicalTrials.gov Identifier: NCT02715284);, ANA011 (AnaptysBio, Inc.),; AGEN-2034 (Agenus, Inc.); AM-0001 (ARMO Biosciences);, BGB-108 (BeiGene);, AK-104 and AK-105 (Akeso Biopharma), ABBV-181 (AbbVie), BAT-1306 (Bio-Thera Solutions), AMP-224 (MedImmune), LZM-009 (Livzon Pharmaceutical Group), GLS-010 (Arcus Biosciences), Dostarlimab (Tesaro Inc, CAS Number 2022215-59-2), MGA-012 (Incyte Corp), Tislelizumab (BGB-A317, (BeiGene, CAS Number 1858168-59-8),; BI-754091 (Boehringer Ingelheim),; CBT-501 (CBT Pharmaceuticals, Inc.),; ENUM-003 ( Enumeral Biomedical Holdings Inc ),; ENUM-388D4 ( Enumeral Biomedical Holdings Inc ),; ENUM-244C8 (Enumeral Biomedical Holdings Inc), IBI-308 (Eli Lillylnnovent Biologics, Inc.),; JNJ-63723283 (Johnson & JohnsonJanssen Research & Development, LLC, ClinicalTrials.gov Identifier NCT02908906),; CS-1003 (CStone Pharmaceuticals), Sym-016 and Sym-021 (Symphogen), JS-001 (Shanghai Junshi Bioscience Co.,Ltd., ClinicalTrials.gov Identifier NCT02857166, JTX-4014 (Jounce Therapeutics, Inc.),; JY-034 (Beijing Eastern Biotech Co), SSI-361 (Lyvgen Biopharma Ltd), YBL-006 (Y-Biologics), AK-103 (Akeso Biopharma Inc),; MCLA-134 (Merus);, HAB-21 (Suzhou Stainwei Biotech Inc), CX-188 (CytomX Therapeutics Inc), PF-06801591 (Pfizer, ClinicalTrials.gov Identifier NCI-2016-00704);, HEISCOIII-003 (Sichuan Haisco Pharmaceutical Co), XmAb-20717 (Xencor Inc, bispecific, recognizing CTLA-4 and PD1), XmAb-23104 (Xencor Inc), MGD-019 (MacroGenics Inc, bispecific, recognizing CTLA4 and PD1), AK-112 (Akeso Biopharma, bispecific), AT-16201 (AIMM Therapeutics BV), BCD-100 (Biocard), TSR-075 (Tesaro Inc, bispecific, regognizing LAG3 and PD1), MGD-013 (MacroGenics; bi-specific; recognizing PD-1 and LAG-3), BH-2922 (Beijing Hanmi Pharmaceutical Co, bispecific, recognizing EGFR and PD1), BH-2941 (Beijing Hanmi Pharmaceutical Co, bispecific, recognizing PDL1 and PD1), BH-2950 (Beijing Hanmi Pharmaceutical Co, bispecific, recognizing Her2 and PD1), BH-2954 (Beijing Hanmi Pharmaceutical Co, bispecific), STIA-1110 (Les Laboratoires Servier SASSorrento Therapeutics),; 244C8 and 388D4 (cf. Scheuplein F et al. [abstract]. Proc 107th Ann Meet Am Ass Canc Res; 2016 Apr 16-20; New Orleans, LA. Philadelphia (PA): AACR; Cancer Res 2016;76(14 Suppl):Abstract nr 4871);
b) the anti-PDL1 antibodies BMS-936559 (also referred to as MDX-1105, ViiV Healthcare UK Ltd) (Brahmer et al. 2012. N Engl J Med. 366(26):2455-65), Atezolizumab (also referred to as MPDL3280A; Roche, trade name TECENTRIQ^{®}, CAS Number 1380723-44-3) (Cha et al. Semin Oncol. 2015 Jun;42(3):484-7), Durvalumab (also referred to as MEDI4736, Medlmmune, AstraZeneca, CAS Number 1428935-60-7) (Antonia et al. Lancet Oncol. 2016 Mar;17(3):299-308),; Avelumab (also referred to as MSB0010718C, Merck, CAS Number 1537032-82-8) (Boyerinas et al. Cancer Immunol Res. 2015 Oct;3(10):1148-57),; BGBA-333 (BeiGene Ltd), CX-072 (CytomX Therapeutics Inc), KD033 (Kadmon Corp, , LLC), KN-035 (AlphaMab Co), BCD-135 (Biocad),; CBA-0710 (Sorrento Therapeutics Inc), CK-301 (Checkpoint Therapeutics Inc), MSB-2311 (MabSpace Biosciences), LY-3300054 (Eli Lilly),CS-1001 (CStone Pharmaceuticals Co), FAZ-053 (Novartis AG), SHR-1316 (Jiangsu Hengrui Medicine Co), FS-118 (F-star Biotechnology Ltd, bispecific, recognizing PD-L1 and LAG-3), HLX-10 (Shanghai Henlius Biotech Co), STIA-1015 (Sorrento Therapeutics), BH-2941 (Beijing Hanmi Pharmaceutical Co, bispecific, recognizing PDL1 and PD1), CBT-502 (Chia Tai Tianqing Pharmaceutical Group Co), STT-01 (Stcube Inc), JS-003 (Shanghai Junshi Bioscience Co, bispecific), HLX-20 (Shanghai Henlius Biotech Co), YBL-007 (Y-Biologics), YBL-008 (Y-Biologics, bispecific, recognizing VEGF and PDL1), IMM-25 (ImmuneOnco Biopharmaceuticals (Shanghai) Co), KD-036 (Kadmon Corp LLC), KY-1003 (Kymab Ltd), STIA-1011 (Sorrento Therapeutics Inc), PMC-305 (PharmAbcine Inc), IKT-203 (Icell Kealex Therapeutics), AK-106 (Akeso Biopharma Inc), IKT-703 (Icell Kealex Therapeutics), MSB-002 (MabSpace Biosciences (Suzhou) Co Ltd), STIA-100X (Sorrento Therapeutics Inc), STIA-1010 (Sorrento Therapeutics Inc), STIA-1012 (Sorrento Therapeutics Inc), STIB-010X (Sorrento Therapeutics Inc), STI-A1014 (Sorrento Therapeutics),; MCLA-145 (Merus, bispecific); and SP142 (Spring Bioscience);
   and (c) the anti-PDL2 antibody rHigM12B7 (Mayo Clinic) and CA-170 (Aurigene Discovery Technologies Ltd, Curis).

In this context particularly preferred are bi- and/or trispecific antibodies directed against PD-1, PD-L1 or PD-L2 e.g. MCLA-134 (bispecific; recoginizing PD-1 and TIM-3); MGD-013 (bispecific; recognizing PD-1 and LAG-3), Sym-016 (trispecific; recognizing PD-L1, LAG-3 and TIM-3), XmAb-20717 (bispecific; recoginizing PD-1 and CTLA-4),

In preferred embodiments, the PD-1 pathway inhibitor is thus selected from (a) an antibody as described above, (b) an antibody variant, in particular a sequence variant of the antibodies described above, (c) a fragment of an antibody (or variant thereof) as described above, in particular an antigen-binding fragment such as a Fab, Fab", F(ab")2, Facb, Fab/c, Fd, Fd", Fv, heavy chain antibodies, sdAb (nanobodies) or (d) a derivative of such an antibody (or variant thereof) or fragment of said antibody (or variant thereof), in particular an antigen-binding derivative, including scFv, scFv", scFv dimers (diabodies), scFv trimers (triabodies), or minibodies, or (e) a nucleic acid (preferably a DNA or RNA, in particular mRNA) encoding (a), (b), (c) and/or (d).

In further preferred embodiments, the PD-1 pathway inhibitor antibody comprises
(a) an antibody heavy chain comprising or consisting of an amino acid sequence according to SEQ ID NO: 19 (SEQ ID NO: 4761 of PCT/EP2016/059711) or a variant or fragment thereof, and/or an antibody light chain comprising or consisting of an amino acid sequence according to SEQ ID NO: 20 (4768 of PCT/EP2016/059711) or a variant or fragment thereof; or
(b) an antibody heavy chain comprising or consisting of an amino acid sequence according to SEQ ID NO: 21 (4292 of PCT/EP2016/059711) or a variant or fragment thereof, and/or an antibody light chain comprising or consisting of an amino acid sequence according to SEQ ID NO: 22 (4299 of PCT/EP2016/059711) or a variant or fragment thereof; or
(c) an antibody heavy chain comprising or consisting of an amino acid sequence according to SEQ ID NO: 23 (5139 of PCT/EP2016/059711) or a variant or fragment thereof, and/or an antibody light chain comprising or consisting of an amino acid sequence according to SEQ ID NO: 24 (5146 of PCT/EP2016/059711) or a variant or fragment thereof; or
(d) an antibody heavy chain comprising or consisting of an amino acid sequence according to SEQ ID NO: 25 (4852 of PCT/EP2016/059711) or a variant or fragment thereof, and/or an antibody light chain comprising or consisting of an amino acid sequence according to SEQ ID NO: 26 (4859 of PCT/EP2016/059711) or a variant or fragment thereof; or
(e) an antibody heavy chain comprising or consisting of an amino acid sequence according to SEQ ID NO: 27 (1590 of PCT/EP2016/059711) or a variant or fragment thereof, and/or an antibody light chain comprising or consisting of an amino acid sequence according to SEQ ID NO: 28 (1597 of PCT/EP2016/059711) or a variant or fragment thereof; or
(f) an antibody heavy chain comprising or consisting of an amino acid sequence according to SEQ ID NO: 29 (379 of PCT/EP2016/059711) or a variant or fragment thereof, and/or an antibody light chain comprising or consisting of an amino acid sequence according to SEQ ID NO: 30 (368 of PCT/EP2016/059711) or a variant or fragment thereof; or
(g) an antibody heavy chain comprising or consisting of an amino acid sequence according to SEQ ID NO: 31 (428 of PCT/EP2016/059711) or a variant or fragment thereof, and/or an antibody light chain comprising or consisting of an amino acid sequence according to SEQ ID NO: 32 (435 of PCT/EP2016/059711) or a variant or fragment thereof.

### LAG-3 pathway inhibitor antibodies

Antibodies against members of the LAG-3 pathway (including LAG-3) may be used as LAG-3 pathway inhibitors in accordance with the present invention. Antibodies used as LAG-3 pathway inhibitors are preferably capable of specifically binding to their respective target and inhibit LAG-3 pathway signalling.

Such antibodies are thus envisaged to specifically bind to LAG-3 (in particular to its extracellular domain); for instance in a way so that receptor-ligand interactions between LAG-3 and MHC-II are prevented or disrupted, thereby impairing LAG-3 pathway signalling. E.g., LAG-3 pathway inhibitor antibodies may bind proximal to binding sites required for receptor ligand interaction. By sterically hindering receptor ligand interaction, and/or displacing the LAG-3 ligands from their active binding sites at the LAG-3 receptor, LAG-3 mediated signalling may be prevented or disrupted.

Preferred antibodies used as LAG-3 pathway inhibitors include BMS-986016, LAG525 and GSK2831781 (ClinicalTrials.gov Identifier: NCT02195349) BI-754111 (Boehringer Ingelheim), ENUM-006 ( Enumeral Biomedical Holdings Inc), FS-18, IMP-701 (CoStim Pharmaceuticals; Novartis), IMP-731 (Immutep), MGD-013 (MacroGenics; bi-specific; recognizing PD-1 and LAG-3). Sym-016 (Symphogen; tri-specific; recognizing PD-L1, LAG-3 and TIM-3), TRL-7117 (Trellis Bioscience), and TSR-033 (Creative Biolabs).

### Multispecific binding proteins

It is particularly preferred that the PD-1 pathway inhibitor and the LAG-3 pathway inhibitor are comprised by the same binding protein, particularly in the same antibody. Thus, in preferred embodiments, the PD-1 and the LAG-3 pathway inhibitor selected from a multispecific antibody, preferably a bi- or trispecific antibody specifically binding to LAG-3 and at least one of PD-1, PD-L1 and/or PD-L2, said bi- or trispecific antibody optionally being selected from MGD-013 (bispecific; recognizing PD-1 and LAG-3), Sym-016 (trispecific; recognizing PD-L1, LAG-3 and TIM-3).

In this context, a "multispecific antibody" is defined as an antibody or an antibody fragment, variant or derivative capable of specifically recognizing several (at least two) distinct epitopes (and optionally two distinct antigens comprising the same) via its antigen-binding sites. The term includes bi-specific and tri-specific antibodies as defined elsewhere herein.

In preferred embodiments, the LAG-3 pathway inhibitor is thus selected from (a) an antibody as described above, (b) an antibody variant, in particular a sequence variant of the antibodies described above, (c) a fragment of an antibody (or variant thereof) as described above, in particular an antigen-binding fragment such as a Fab, Fab", F(ab")2, Facb, Fab/c, Fd, Fd", Fv, heavy chain antibodies, sdAb (nanobodies) or (d) a derivative of such an antibody (or variant thereof) or fragment of said antibody (or variant thereof), in particular an antigen-binding derivative, including scFv, scFv", scFv dimers (diabodies), scFv trimers (triabodies), or minibodies, or a nucleic acid (preferably a DNA or RNA, in particular mRNA) encoding (a), (b), (c) and/or (d).

### Antagonistic binding proteins and nucleic acids encoding the same

In further preferred embodiments, the at least one PD-1 pathway inhibitor and/or the at least one LAG-3 pathway inhibitor are independently from each other selected from an antagonistic binding protein or a nucleic acid encoding the same.

### Antagonistic binding proteins

An "antagonistic binding protein" is a protein that acts as an antagonist (as defined above). The terms "protein" and "polypeptide" are used interchangeably herein to refer to (macro) molecules comprising at least two amino acids joined to each other by a peptide bond. As used herein, the term "antagonistic binding proteins" preferably refers to proteins capable of specifically binding to PD-1, PD-L1 or PD-L2 (in case of PD-1 pathway inhibitors) or to LAG-3 (in case of LAG-3 inhibitors) and antagonizing the action of their respective ligands, thereby impairing PD-1 or LAG-3 signalling. Thus, antagonistic binding proteins in the context of the present invention are preferably capable of binding to PD-1 or LAG-3 but impairing PD-1 pathway signaling or LAG-3 pathway signaling. The term "specifically binding" has been defined in the context of antibodies above and equally applies to the antagonistic binding proteins defined herein. The term "antagonistic binding protein" may, but preferably does not include antibodies as defined above. Antagonistic binding proteins envisaged for use in the inventive combination may be monospecific, bispecific or multispecific. Antagonistic binding proteins may be chimeric fusion proteins or may be fragments, variants or derivatives of receptors or their respective ligands discussed herein.

Preferred antagonistic binding proteins in the context of the present invention include fusion proteins and soluble receptors.

### Nucleic acids encoding antagonistic binding proteins

PD-1 pathway inhibitors and/or the LAG-3 pathway inhibitors described herein may independently from each other be selected from a nucleic acid encoding an antagonistic binding protein as described herein.

Such nucleic acids are preferably selected from a DNA, a cDNA or an RNA, more preferably an RNA, and most preferably an mRNA, and comprise at least one coding sequence that encodes the respective antagonistic binding protein and optionally regulatory elements driving its expression. Said nucleic acids, in particular RNAs, may comprise any of the modifications (i.e. chemical, lipid or sequence modifications) described in the context of the epitope-encoding RNAs above.

### Fusion proteins

"Fusion proteins" or "chimeric proteins" are proteins created through the joining of two or more "parent" genes which originally coded for separate proteins. Translation of said fusion gene results in a single polypeptide (i.e., the fusion protein). Said fusion protein may comprise the full polypeptide sequence encoded by the "parent" genes, or fragments or variants thereof, encoding a portion of the "parent" polypeptide sequence (e.g. a domain) or a derivative thereof. Fusion proteins may thus retain the biological function of the "parent" gene products; or may comprise additional or alternative biological functions. Fusion proteins may comprise a peptide linker or ("spacer") between the fused polypeptide sequences, so as to allow correct folding and/or prevent steric hindrance of the fused entities. In the context of the present invention, fusion proteins particularly encompass recombinant fusion proteins, i.e. fusion proteins created artificially by recombinant DNA technology (genetic engineering). Fusion proteins used as antagonistic binding proteins inhibiting PD-1 or LAG-3 pathway typically comprise a portion that is capable of binding to PD-1 (in case of PD-1 pathway inhibitors) or LAG-3 (in case of LAG-3 pathway inhibitors) but without triggering the respective signaling cascade. Typically, such fusion proteins may comprise PD-1 ligands or fragments thereof (in case of PD-1 pathway inhibitors) or LAG-3 ligands or fragments thereof (in case of LAG-3 pathway inhibitors), which retain the binding specificity of said ligand to its respective target, but inhibit or do not elicit the respective downstream signaling pathway which is typically triggered by binding of said ligand to its target. Fusion proteins may comprise further entities (protein fragments, protein domains or other moieties) which may mediate binding of further targets (e.g. antibodies or fragments or variants thereof, or other binding proteins), toxicity (e.g. toxins), effector functions (e.g. antibody Fc parts) or optimized pharmacokinetic properties, e.g. increased stability, bioavailability, absorption; distribution and/or reduced clearance.

### PD-1 pathway inhibitor fusion proteins

Accordingly, fusion proteins used as PD-1 pathway inhibitors in accordance with the present invention may comprise (i) a PD-L1 ligand or a fragment, variant or derivative thereof; and/or (ii) a PD-L2 ligand or a fragment, variant or derivative thereof; and optionally (iii) a further moiety optionally selected from an Fc immunoglobulin.

The term "PD-L1" preferably refers to the human "Programmed cell death 1 ligand 1" (also referred to as "B7 homolog 1" or "B7-H1", UniProt Acc. No. Q9NZQ7, entry version #144 last modified November 2, 2016, sequence version #1) encoded by the *CD274* gene. The term "PD-L2" preferably refers to the human "Programmed cell death 1 ligand 2" (also referred to as "Butyrophilin" or "B7-DC", UniProt Acc. No. Q9BQ51; entry version #128 last modified November 2, 2016, sequence version #2) encoded by the *PDCD1LG2* gene.

Fusion proteins envisaged as PD-1 inhibitors may either comprise full-length PD-1L and/or PD-2L or fragments, variants or derivatives thereof. The terms "fragment", "variant" and "derivative" have been defined in the context of antibodies. The respective definitions are applicable to the fusion proteins described herein, *mutatis mutandis.*

A preferred example of such a fusion protein is AMP-224. AMP-224 is a recombinant fusion protein composed of the extracellular domain of the PD-1 ligand programmed cell death ligand 2 (PD-L2, B7-DC) and the Fc region of human immunoglobulin (Ig) G1, with potential immune checkpoint inhibitory and antineoplastic activities. Anti-PD-1 fusion protein AMP-224 reportedly specifically binds to PD-1 on chronically stimulated (but not normal activated) T-cells and reduces their proliferation. This may restore immune function and may result in the activation of cytotoxic T-cells and cell-mediated immune responses against tumor cells (cf. Smothers et al. Ann Oncol (2013) 24 (suppl 1): i7 and Mkrtichyan et al., 2012. J Immunol. 189(5):2338-47).

The present invention further envisages nucleic acids encoding fusion proteins as described above as PD-1 pathway inhibitors.

### LAG-3 pathway inhibitor fusion proteins

Fusion proteins used as LAG-3 pathway inhibitors in accordance with the present invention may comprise (i) a LAG-3 ligand or a fragment, variant or derivative thereof; and optionally (ii) a further moiety optionally selected from an Fc immunoglobulin.

Fusion proteins envisaged as LAG-3 inhibitors may for instance comprise full-length MHC-II or fragments, variants or derivatives thereof. The terms "fragment", "variant" and "derivative" have been defined in the context of antibodies. The respective definitions are applicable to the fusion proteins described herein, mutatis mutandis.

The present invention further envisages nucleic acids encoding fusion proteins as described above as LAG-3 pathway inhibitors.

### Soluble PD-1 or LAG-3 receptors

Soluble forms of the PD-1 or LAG-3 (also referred to herein as "soluble PD-1 receptors" or "sPD-1" and "soluble LAG-3 receptors" or "sLAG-3", respectively) described herein are further preferred pathway inhibitors in the context of the present invention. Such soluble forms of PD-1 and LAG-3 preferably retain the ligand-binding ability of the membrane bound forms, and thus act as competitive inhibitors of the binding of the respective ligand (i.e. as competitive antagonists). Thereby, sPD-1 and sLAG-3 are preferably capable of inhibiting signal transduction caused by binding of the respective ligand to its membrane-bound PD-1 or LAG-3 receptor. Preferably, sPD-1 and sLAG-3 exhibit comparable binding affinities and binding specificities as their membrane-bound counterparts.

sPD-1 and sLAG-3 in the context of the present invention preferably retain the extracellular domains of their membrane-bound counterparts (whose sequences are depicted below), but lack the all or part of the signal peptide, transmembrane domain and cytoplasmic domain.

In the context of the present invention, soluble PD-1 and LAG-3 receptors are not limited to PD-1 or LAG-3 receptors comprising or consisting of extracellular domains exhibiting 100% identity to (part of) the PD-1 sequence (SEQ ID NO: 33) and LAG-3 sequence (SEQ ID NO: 34) depicted below. Rather, variants, includings sequence variants (e.g. comprising amino acid substitutions (particularly conservative substitutions), deletions and/or insertions), and modified variants including chemical and/or post-translational modifications are also encompassed by the terms "sPD-1" and "sLAG-3". Said terms further include derivatives of sPD-1 and sLAG3, respectively, which are modified to include an additional functionality, e.g. optimized manufacturing properties (including moieties which confer an increased solubility or enhanced excretion, or allow for purification), or pharmacokinetics/pharmacodynamics for *in vivo* use (including moieties which confer increased stability, bioavailability, absorption; distribution and/or reduced clearance). For instance, such soluble PD-1 or LAG-3 receptor derivatives may comprise further moieties, typically at the amino and/or carboxyl terminal residues, e.g. serving as purification tags or stabilizers.

Soluble receptors described herein may in particular be truncated receptors lacking the transmembrane and cytosolic domain or any other part of the sequence which is not required for ligand binding), or truncated receptor derivatives comprising further moieties or domains which preferably implicate an additional functionality as described above.

Soluble forms of PD-1 and LAG-3, including variants and derivatives thereof can be prepared using well-known *in vitro* recombinant DNA techniques, using nucleotide sequences encoding the appropriate polypeptides or peptides. Methods well-known to those skilled in the art can be used to construct expression vectors containing relevant coding sequences and appropriate transcriptional/translational control signals. See, for example, the techniques described in Sambrook, et al., Molecular Cloning: A Laboratory Manual (3rd Ed.) [Cold Spring Harbor Laboratory, N.Y., 2000].

### Soluble PD-1 receptors

In preferred embodiments, the PD-1 pathway inhibitor is a soluble PD-1 (receptor) or a variant or derivative thereof, or a nucleic acid encoding the same.

The term "soluble PD-1 (receptor)" or "sPD-1" is used herein to refer to PD-1 polypeptides which are substantially free of transmembrane and intracellular (cytoplasmic) polypeptide domains, i.e. lack sufficient portions of these segments to provide membrane anchoring or signal transduction, respectively.

As used herein, the term "PD-1" or "PD-1 protein" or "PD-1 polypeptide" or "PD-1 receptor" preferably refers to the human "Programmed cell death protein 1" (UniProt Acc. No. Q15116, entry version #152 last modified November 2, 2016, sequence version #3) encoded by the *PDCD1* gene or an allelic variant or ortholog thereof. Soluble PD-1 receptors in the context of the present invention may thus comprise the (part of) the following amino acid sequence as depicted in SEQ ID NO: 33 below

SEQ ID NO: 33

10 20 30 40 50
MQIPQAPWPV VWAVLQLGWR PGWFLDSPDR PWNPPTFSPA LLVVTEGDNA
60 70 80 90 100
TFTCSFSNTS ESFVLNWYRM SPSNQTDKLA AFPEDRSQPG QDCRFRVTQL
110 120 130 140 150
PNGRDFHMSV VRARRNDSGT YLCGAISLAP KAQIKESLRA ELRVTERRAE
160 170 180 190 200
VPTAHPSPSP RPAGQFQTLV VGVVGGLLGS LVLLVWVLAV ICSRAARGTI
210 220 230 240 250
GARRTGQPLK EDPSAVPVFS VDYGELDFQW REKTPEPPVP CVPEQTEYAT
260 270 280
IVFPSGMGTS SPARRGSADG PRSAQPLRPE DGHCSWPL

As indicated above, soluble receptors comprising isoforms, variants and derivatives of the polypeptide sequence depicted above (and in particular the extracellular domain) are also envisaged as long as they are soluble and retain the ligand binding capabilities of the PD-1 receptor.

Accordingly, in preferred embodiments, the PD-1 pathway inhibitor is a "soluble PD-1 receptor" comprising a portion of the amino acid sequence depicted in SEQ ID NO: 33 or comprising an amino acid sequence which is at least 75%, preferably at least 80%, preferably at least 85%, preferably at least 90%, more preferably at least 95%, more preferably at least 96%, more preferably at least 97%, more preferably at least 98%, most preferably at least 99% identical to a portion of the amino acid sequence depicted in SEQ ID NO: 33. Soluble forms of PD-1 preferably comprise the extracellular PD-1 domain comprising amino acid residues corresponding to amino acid residues 21 to 170 of SEQ ID NO: 33 depicted above (underlined) and/or may preferably lack all or part of the PD-1 transmembrane domain comprising amino acid residues corresponding to amino acid residues 171 to 191 of SEQ ID NO: 33 depicted above and/or the PD-1 cytoplasmic domain comprising amino acid residues corresponding to amino acid residues 192 to 288 of SEQ ID NO: 33 depicted above.

In further preferred embodiments, the PD-1 pathway inhibitor is a nucleic acid, preferably a DNA or RNA, in particular a mRNA, comrpsing at least one coding sequence encoding a soluble PD-1 as defined above.

### Soluble LAG-3 receptors

In preferred embodiments, the LAG-3 pathway inhibitor is a soluble LAG-3 (receptor) or a variant or derivative thereof, or a nucleic acid encoding the same.

As used herein, the term "LAG-3" or "LAG-3 protein" or "LAG-3 polypeptide" or "LAG-3 receptor" preferably refers to the human "Lymphocyte activation gene 3 protein" (UniProt Acc. No. P18627, entry version #150 last modified November 30, 2016, sequence version #5) encoded by the LAG3 gene or an allelic variant or ortholog thereof. Soluble LAG-3 receptors in the context of the present invention may thus comprise the (part of) the following amino acid sequence as depicted in SEQ ID NO: 34 below

    10         20         30         40         50
MWEAQFLGLL FLQPLWVAPV KPLQPGAEVP VVWAQEGAPA QLPCSPTIPL
    60         70         80         90        100
QDLSLLRRAG VTWQHQPDSG PPAAAPGHPL APGPHPAAPS SWGPRPRRYT
    110        120        130        140        150
VLSVGPGGLR SGRLPLQPRV QLDERGRQRG DFSLWLRPAR RADAGEYRAA
    160        170        180        190        200
VHLRDRALSC RLRLRLGQAS MTASPPGSLR ASDWVILNCS FSRPDRPASV
    210        220        230        240        250
HWFRNRGQGR VPVRESPHHH LAESFLFLPQ VSPMDSGPWG CILTYRDGFN
    260        270        280        290        300
VSIMYNLTVL GLEPPTPLTV YAGAGSRVGL PCRLPAGVGT RSFLTAKWTP
    310        320        330        340        350
PGGGPDLLVT GDNGDFTLRL EDVSQAQAGT YTCHIHLQEQ QLNATVTLAI
    360        370        380        390        400
ITVTPKSFGS PGSLGKLLCE VTPVSGQERF VWSSLDTPSQ RSFSGPWLEA
    410        420        430        440        450
QEAQLLSQPW QCQLYQGERL LGAAVYFTEL SSPGAQRSGR APGALPAGHL
    460        470        480        490        500
LLFLILGVLS LLLLVTGAFG FHLWRRQWRP RRFSALEQGI HPPQAQSKIE
    510        520
ELEQEPEPEP EPEPEPEPEP EPEQL

As indicated above, soluble receptors comprising variants, fragments and derivatives of the polypeptide sequence depicted above (and in particular the extracellular domain) are also envisaged herein as long as they are soluble and retain the ligand binding capabilities of the LAG-3 receptor.

Accordingly, in preferred embodiments, the LAG-3 inhibitor is a "soluble LAG-3" which comprises a portion of the amino acid sequence depicted in SEQ ID NO: 34 or comprising an amino acid sequence which is at least 75%, preferably at least 80%, preferably at least 85%, preferably at least 90%, more preferably at least 95%, more preferably at least 96%, more preferably at least 97%, more preferably at least 98%, most preferably at least 99% identical to SEQ ID NO: 34 or a portion thereof. Soluble forms of LAG-3 preferably comprise the extracellular LAG-3 domain comprising amino acid residues corresponding to amino acid residues 29 to 450 of SEQ ID NO: 34 depicted above (underlined) and/or may preferably lack all or part of the LAG-3 transmembrane domain comprising amino acid residues corresponding to amino acid residues 451 to 471 of SEQ ID NO: 34 depicted above and/or the LAG-3 cytoplasmic domain comprising amino acid residues corresponding to amino acid residues 472 to 525 of SEQ ID NO: 34 depicted above.

A preferred example of a soluble LAG-3 receptor derivative is IMP321. IMP231 is a soluble LAG-3 derived receptor that consists of an extracellular portion of human LAG-3 fused to the Fc fraction of human IgG1. IMP321 reportedly antagonizes normal LAG-3 signaling by binding to MHC II on the surface of APCs and has been shown to have strong immunostimulatory effects (Brignone C et al. J Immunol. 2007;179(6):4202-11).

In further preferred embodiments, the LAG-3 inhibitor is a nucleic acid, preferably a DNA or RNA, in particular a mRNA, comripsing at least one coding sequence encoding a soluble LAG-3 as defined above.

### Antagonistic nucleic acids

According to further preferred embodiments, PD-1 pathway inhibitors and/or LAG-3 pathway inhibitors of the inventive combination are selected independently from each other from antagonistic nucleic acids, optionally selected from a microRNA, a siRNA, a shRNA, an antisense RNA, or an aptamer.

As used herein, the term "antagonistic nucleic acid" refers to nucleic acids (as defined above) which are capable of inhibiting or reducing agonist-mediated biological receptor signalling by binding to a target. In this context, a target may be a nucleic acid (e.g. a DNA or RNA) coding for the respective receptor agonist or its ligand. For instance, antagonistic nucleic acids may bind to DNA or RNA sequences encoding PD-1, PD-L1 or PD-L2 (in case of PD-1 pathway inhibitors); or DNA or RNA sequences encoding LAG-3 (in case of LAG-3 inhibitors). Antagonistic nucleic acids of interest include antisense RNAs, in particular microRNAs, siRNAs or shRNAs.

"Antisense RNAs" or "asRNAs" are single or double-stranded RNA molecules exhibiting preferably at least 90%, more preferably 95% and especially 100% (of the nucleotides of a dsRNA) sequence identity to a section of a naturally occurring mRNA sequence. In the context of the present invention, such naturally occurring mRNA sequence may be coding for PD-1, PD-L1 or PD-L2 (in case of PD-1 pathway inhibitors) or LAG-3 (in case of LAG-3 pathway inhibitors). Antisense RNAs typically exhibit complementarity either to a coding or a non-coding section, however, in some cases wobble base (G:U) pairing, nucleotide bulges and/or mismatches may occur as long as they do not abolish the capability of the antisense RNA to bind to its target and impair the targeted signalling pathway.

"MicroRNAs" or "miRNAs" are small (~20-24 nucleotide) non-coding double-stranded RNAs (dsRNAs) capable of recruiting the AGO-2 RISC complex to a complementary target transcript, thereby preferably inducing the miRNA-mediated RNAi pathway. MicroRNAs are typically processed from pri-microRNA to short stem-loop structures called pre-microRNA and finally to mature miRNA. Both strands of the stem of the pre-microRNA may be processed to a mature microRNA. After processing, the mature single-stranded microRNAs, associated with Argonaute 2 (AGO2) in the RNA-induced silencing complex (RISC), typically bind to the 3' UTRs of their cytosolic mRNA targets, resulting in either reduced translation or deadenylation and degradation of the mRNA transcript. The predominant function of microRNAs is thus to (negatively) regulate protein translation by binding to complementary sequences of target mRNAs. The term "microRNA" includes miRNAs, mature single stranded miRNAs, precursor miRNAs (pre-miRNA), primary miRNA transcripts (pri-miRNA), duplex miRNAs and variants thereof. MicroRNAs are particularly envisaged to be capable of binding to a target site within a 3 , untranslated region of a target nucleic acid.

"Small interfering RNAs" or "siRNAs" are small (~12-35 nucleotide) non-coding RNA molecules capable of inducing RNAi. siRNAs comprise an RNA duplex (double-stranded region) formed by complement base pairing with phosphorylated 5'-ends and hydroxylated 3'-ends, optionally with one or two single-stranded overhanging nucleotides. The duplex portion typically comprises between 17 and 29 nucleotides. siRNA may be generated from two RNA molecules that hybridize together or may alternatively be generated from a single RNA molecule that includes a self-hybridizing portion (shRNA). The duplex portion of an siRNA may, but typically does not, include one or more bulges containing one or more unpaired and/or mismatched nucleotides in one or both strands of the duplex or may contain one or more non-complementary nucleotide pairs. One strand of a siRNA (referred to as the antisense strand) includes a portion that hybridizes with a target transcript (e.g. a target mRNA). The antisense strand may be precisely complementary with a complementary region of the target transcript (i.e. the siRNA antisense strand may hybridize to the target sequence without a single mismatch, wobble base pairing or nucleotide bulge) or one or more mismatches, wobble (G:U) base pairings and/or nucleotide bulges between the siRNA antisense strand and the complementary region of the target transcript may exist.

According to preferred embodiments, siRNAs directed against PD-1, PD-L1 or PD-L2 (in case of PD-1 pathway inhibitors) or directed against LAG-3 (in case of LAG-3 inhibitors) are double-stranded RNAs (dsRNAs) having a length of from 17 to 29, preferably from 19 to 25, and preferably is at least 90%, more preferably 95% and especially 100% (of the nucleotides of a dsRNA) complementary to a section of the naturally occurring mRNA sequence coding for PD-1, PD-L1 or PD-L2 (in case of PD-1 pathway inhibitors) or LAG-3 (in case of LAG-3 pathway inhibitors) either to a coding or a non-coding section, preferably a coding section. Such a section of the naturally occurring mRNA sequence may be termed herein a "target sequence" and may be any section of the naturally occurring mRNA coding for PD-1, PD-L1 or PD-L2 (in case of PD-1 pathway inhibitors) or LAG-3 (in case of LAG-3 pathway inhibitors). The sequence of the double-stranded siRNA used according to the invention is, however, preferably wholly complementary in its general structure with a section of the target sequence. In this context the nucleic acid molecule of the complex may be a dsRNA having the general structure 5'-(N17-29)-3', preferably having the general structure 5'-(N19-25)-3', more preferably having the general structure 5'-(N19-24)-3', or yet more preferably having the general structure 5'-(N21-23)-3', wherein for each general structure each N is a (preferably different) nucleotide of a section of the target sequence, preferably being selected from a continuous number of 17 to 29 nucleotides of a section of the target sequence, and being present in the general structure 5'-(N17-29)-3' in their natural order. In principle, all the sections having a length of from 17 to 29, preferably from 19 to 25, base pairs that occur in the target sequence can serve for preparation of a dsRNA as defined herein. Equally, dsRNAs used as siRNAs can also be directed against mRNA sequences that do not lie in the coding sequence, in particular in the 5' non-coding sequence of the target sequence, for example, therefore, against non-coding sequences of the target sequence having a regulatory function. The target sequence of the dsRNA used as siRNA can therefore lie in the translated and untranslated region of the target sequence and/or in the region of the control elements of the mRNA sequence. The target sequence for a dsRNA used as siRNA directed against PD-1, PD-L1 or PD-L2 (in case of PD-1 pathway inhibitors) or LAG-3 (in case of LAG-3 pathway inhibitors) can also lie in the overlapping region of untranslated and translated sequence; in particular, the target sequence can comprise at least one nucleotide upstream of the start triplet of the coding sequence of the mRNA sequence.

"Short hairpin RNAs" or "shRNAs" are single-strand RNA molecules comprising at least two complementary portions hybridized or capable of hybridizing to form a double-stranded (duplex) structure sufficiently long to mediate RNAi. These complementary portions are generally between 17~29 nucleotides in length, typically at least 19 base pairs in length. shRNAs further comprise at least one single-stranded portion, typically between 1~10 nucleotides in length that forms a loop connecting the complementary strands forming the duplex portion. The duplex portion may, but typically does not, contain one or more bulges consisting of one or more unpaired nucleotides. As described above, shRNAs are thought to be processed into siRNAs (see above) by the RNAi machinery. shRNAs are therefore siRNA precursors and are thought to induce gene silencing via the siRNA-mediated RNAi pathway.

"Aptamers" or "oligonucleotide aptamers" are small nucleic acid ligands composed of RNA or single-stranded DNA oligonucleotides with high specificity and affinity for their targets. These short, chemically synthesized oligonucleotides fold into specific three-dimensional (3D) structures. In contrast to other nucleic acid molecular probes, aptamers interact with and bind to their targets through structural recognition, a process similar to that of an antigen-antibody reaction. Aptamers may be used to target any member of the PD-1 signaling pathway (in case of PD-1 pathway inhibitors) or LAG-3 signaling pathway (in case of LAG-3 pathway inhibitors) in order to antagonize PD-1 or LAG-3 action. The term "aptamer" as used herein includes mono-, bi- and polyvalent aptamers, mono-, bi- and multispecifc aptamers, aptamer-drug conjugates (ApDC) comprising aptamers covalently coupled to a drug (e.g. a chemotherapeutic agent), optionally via a suitable linker, aptamers coupled to high molecular weight polymers (e.g. PEG), aptamer-tethered DNA nanotrains (aptNTrs), aptamers associated with carriers (e.g. copolymers, liposomes metal nanoparticles or virus-like particules), aptamer-Fc conjugates and aptamer-siRNA or aptamer-miRNA chimeras. (cf. Sun et al. Molecular Therapy Nucleic Acids (2014) 3, e182 for review).

### Small molecules

According to further preferred embodiments, PD-1 pathway inhibitors and/or LAG-3 pathway inhibitors of the inventive combination are selected independently from each other from small molecules inhibiting PD-1 and/or LAG-3 pathway signalling.

Particularly preferred examples in this context are: CA-170 (oral small molecule; PD-L1/PD-L2/VISTA antagonist), CA-327 (oral small molecule; PD-L1/ TIM3 antagonist); and XCE853 (synthetic compound; PD-1 antagonist).

### (Pharmaceutical) composition

In a further aspect, the present invention provides a composition comprising the combination according to the invention, and at least one pharmaceutically acceptable carrier. The composition according to the invention is preferably provided as a pharmaceutical composition or as a vaccine.

A "vaccine" is typically understood to be a prophylactic or therapeutic material providing at least one epitope of an antigen, preferably an immunogen. "Providing at least on epitope" means, for example, that the vaccine comprises the epitope or that the vaccine comprises a molecule that, e.g., codes for the epitope or a molecule comprising the epitope. The vaccine according to the invention comprises at least one epitope-encoding RNA comprising at least one coding sequence encoding an epitope (or an antigen or variant or fragment thereof comprising said epitope). Said epitope (or antigen or variant or fragment thereof comprising said epitope) is derived from an infectious pathogen or from a tumor or cancer cell, and triggers an adaptive immune response which preferably eliminates said infectious pathogen or tumor or cancer cell.

The (pharmaceutical) composition or vaccine provided herein may further comprise at least one pharmaceutically acceptable excipient, adjuvant or further component (e.g. additives, auxiliary substances, and the like).

According to some preferred embodiments, the (pharmaceutical) composition or the vaccine according to the invention comprises the combination of the present invention, wherein the at least one coding sequence of the at least one epitope-encoding RNA encodes at least one epitope of an antigen, preferably at least one epitope of a tumor antigen as defined in List 1, or a fragment or variant of any one of these antigens as defined herein. The (pharmaceutical) composition or vaccine according to the invention may thus comprise at least one epitope-encoding RNA of the inventive combination, wherein the RNA encodes one specific epitope (or an antigen as defined herein or a variant or fragment thereof comprising said epitope), together with a PD-1 and a LAG-3 pathway inhibitor. In such embodiments, the (pharmaceutical) composition or vaccine preferably comprises at least one epitope-encoding RNA comprising the at least one coding sequence as defined herein encoding said epitope (or an antigen as defined herein or a variant or fragment thereof comprising said epitope).

Alternatively, the (pharmaceutical) composition or vaccine of the present invention may comprise, in addition to the at least one PD-1 pathway inhibitor and the at least one LAG-3 pathway inhibitor, at least one epitope-encoding RNA as defined herein, wherein said at least one epitope-encoding RNA encodes at least two, three, four, five, six, seven, eight, nine, ten, eleven or twelve distinct epitopes (or antigens or variants or fragments thereof comprising said epitopes) as defined herein. In such embodiments, the (pharmaceutical) composition or vaccine preferably comprises several classes of the epitope-encoding RNA in combination with the PD-1 and LAG-3 pathway inhibtors, wherein each epitope-encoding RNA species encodes a distinct epitope (or an antigen or a variant or fragment thereof comprising said epitope) as defined herein.

In other embodiments, the epitope-encoding RNA comprised in the (pharmaceutical) composition or vaccine is a bi- or multicistronic RNA as defined herein, which encodes the at least two, three, four, five, six, seven, eight, nine, ten, eleven or twelve distinct epitopes (or antigens or variants or fragments thereof comprising said epitopes).

Mixtures between these embodiments are also envisaged, such as compositions comprising more than one epitope-encoding RNA species, wherein at least one epitope-encoding RNA species may be monocistronic, while at least one other epitope-encoding RNA species may be bi- or multicistronic.

### Complexation

In preferred embodiments, the at least one epitope-encoding RNA of the inventive combination, (pharmaceutical) composition or vaccine (or any other nucleic acid, in particular RNA, as defined herein) is provided in a complexed form, i.e. complexed or associated with one or more (poly-)cationic compounds, preferably with (poly-)cationic polymers, (poly-)cationic peptides or proteins, e.g. protamine, (poly-)cationic polysaccharides and/or (poly-)cationic lipids. In this context, the terms "complexed" or "associated" refer to the essentially stable combination of the at least one epitope-encoding RNA (or said other nucleic acid, in particular RNA) with one or more of the aforementioned compounds into larger complexes or assemblies without covalent binding.

### Lipids

According to preferred embodiments, the epitope-encoding RNA of the inventive combination, (pharmaceutical) composition or vaccine (or any other nucleic acid, in particular RNA, as defined herein) is complexed or associated with lipids (in particular cationic and/or neutral lipids) to form one or more liposomes, lipoplexes, lipid nanoparticles, or nanoliposomes.

Therefore, in some embodiments, the epitope-encoding RNA of the inventive combination, (pharmaceutical) composition or vaccine (or any other nucleic acid, in particular RNA, as defined herein) is provided in the form of a lipid-based formulation, in particular in the form of liposomes, lipoplexes, and/or lipid nanoparticles comprising said epitope-encoding RNA (or said other nucleic acid, in particular RNA) . It is also conceivable to provide the PD-1 and/or LAG-3 pathway inhibitors complexed or associated with lipids to form one or more liposomes, lipoplexes, or lipid nanoparticles.

### Lipid nanoparticles

According to some prefrerred embodiments, the epitope-encoding RNA of the inventive combination, (pharmaceutical) composition or vaccine (or any other nucleic acid, in particular RNA, as defined herein), is complexed or associated with lipids (in particular cationic and/or neutral lipids) to form one or more lipid nanoparticles.

Preferably, lipid nanoparticles (LNPs) comprise: (a) at least one epitope-encoding RNA of the inventive combination, (pharmaceutical) composition or vaccine (or any other nucleic acid, in particular RNA, as defined herein), (b) a cationic lipid, (c) an aggregation reducing agent (such as polyethylene glycol (PEG) lipid or PEG-modified lipid), (d) optionally a non-cationic lipid (such as a neutral lipid), and (e) optionally, a sterol.

In some embodiments, LNPs comprise, in addition to the at least one epitope-encoding RNA of the inventive combination, (pharmaceutical) composition or vaccine (or any other nucleic acid, in particular RNA, as defined herein), (i) at least one cationic lipid; (ii) a neutral lipid; (iii) a sterol, e.g. , cholesterol; and (iv) a PEG-lipid, in a molar ratio of about 20-60% cationic lipid: 5-25% neutral lipid: 25-55% sterol; 0.5-15% PEG-lipid.

In some embodiments, the epitope-encoding RNA of the inventive combination, (pharmaceutical) composition or vaccine (or any other nucleic acid, in particular RNA, as defined herein), may be formulated in an aminoalcohol lipidoid. Aminoalcohol lipidoids which may be used in the present invention may be prepared by the methods described in U.S. Patent No. 8,450,298, herein incorporated by reference in its entirety.

### (i) Cationic lipids

LNPs may include any cationic lipid suitable for forming a lipid nanoparticle. Preferably, the cationic lipid carries a net positive charge at about physiological pH.

The cationic lipid may be an amino lipid. As used herein, the term "amino lipid" is meant to include those lipids having one or two fatty acid or fatty alkyl chains and an amino head group (including an alkylamino or dialkylamino group) that may be protonated to form a cationic lipid at physiological pH.

The cationic lipid may be, for example, N,N-dioleyl-N,N-dimethylammonium chloride (DODAC), N,N-distearyl-N,N-dimethylammonium bromide (DDAB), 1,2- dioleoyltrimethyl ammonium propane chloride (DOTAP) (also known as N-(2,3-dioleoyloxy)propyl)-N,N,N- trimethylammonium chloride and 1,2-Dioleyloxy-3-trimethylaminopropane chloride salt), N-(1-(2,3-dioleyloxy)propyl)-N,N,N-trimethylammonium chloride (DOTMA), N,N-dimethyl-2,3-dioleyloxy)propylamine (DODMA), 1,2-DiLinoleyloxy-N,N-dimethylaminopropane (DLinDMA), 1,2-Dilinolenyloxy-N,N-dimethylaminopropane (DLenDMA), 1,2-di-y-linolenyloxy-N,N-dimethylaminopropane (y-DLenDMA), 1,2-Dilinoleylcarbamoyloxy-3-dimethylaminopropane (DLin-C-DAP), 1,2-Dilinoleyoxy-3-(dimethylamino)acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S- DMA), 1-Linoleoyl-2-linoleyloxy-3-dimethylaminopropane (DLin-2-DMAP), 1,2-Dilinoleyloxy-3-trimethylaminopropane chloride salt (DLin-TMA.Cl), 1,2-Dilinoleoyl-3-trimethylaminopropane chloride salt (DLin-TAP.Cl), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DMA) , 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][I,3]dioxol-5-amine, (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethyl-azanediyl)didodecan-2-ol (C12-200), 2,2-dilinoleyl-4-(2-dimethylaminoethyl)-[1,3]-dioxolane (DLin-K-C2-DMA), 2,2-dilinoleyl-4-dimethylaminomethyl-[1,3]-dioxolane (DLin-K-DMA), (6Z,9Z,28Z,31Z)-heptatriaconta-6,9,28,31-tetraen-19-yl 4-(dimethylamino) butanoate (DLin-M-C3-DMA), 3-((6Z,9Z,28Z,31Z)-heptatriaconta-6,9,28,3-1-tetraen-19-yloxy)-N,N-dimethylpropan-1-amine (MC3 Ether), 4-((6Z,9Z,28Z,31 Z)-heptatriaconta-6,9,28,31-tetraen-19--yloxy)-N,N-dimethylbutan-1-amine (MC4 Ether), or any combination of any of the foregoing.

Other cationic lipids include, but are not limited to, N,N-distearyl-N,N- dimethylammonium bromide (DDAB), 3P-(N-(N',N"-dimethylaminoethane)-carbamoyl)cholesterol (DC-Chol), N-(1-(2,3-dioleyloxy)propyl)-N-2-(spermine-carboxamido)ethyl)-N,N-dimethylammonium trifluoracetate (DOSPA), dioctadecylamidoglycyl carboxyspermine (DOGS), 1,2-dileoyl-sn-3-phosphoethanolamine (DOPE), 1,2-dioleoyl-3-dimethylammonium propane (DODAP), N-(1,2-dimyristyloxyprop-3-yl)-N,N-dimethyl-N-hydroxyethyl ammonium bromide (DMRIE), and 2,2-Dilinoleyl-4-dimethylaminoethyl-[1,3]-dioxolane (XTC). Additionally, commercial preparations of cationic lipids can be used, such as, e.g., LIPOFECTIN (including DOTMA and DOPE, available from GIBCO/BRL), and LIPOFECTAMINE (comprising DOSPA and DOPE, available from GIBCO/BRL).

Other suitable cationic lipids are disclosed in International Publication Nos. WO 09/086558, WO 09/127060, WO 10/048536, WO 10/054406, WO 10/088537, WO 10/129709, and WO 2011/153493; U.S. Patent Publication Nos. 2011/0256175, 2012/0128760, and 2012/0027803; U.S. Patent Nos. 8,158,601; and Love et al, PNAS, 107(5), 1864-69, 2010.

Other suitable amino lipids include those having alternative fatty acid groups and other dialkylamino groups, including those in which the alkyl substituents are different (e.g., N-ethyl- N-methylamino-, and N-propyl-N-ethylamino-). In general, amino lipids having less saturated acyl chains are more easily sized, particularly when the complexes must be sized below about 0.3 microns, for purposes of filter sterilization. Amino lipids containing unsaturated fatty acids with carbon chain lengths in the range of C14 to C22 may be used. Other scaffolds can also be used to separate the amino group and the fatty acid or fatty alkyl portion of the amino lipid.

In some embodiments, amino or cationic lipids have at least one protonatable or deprotonatable group, such that the lipid is positively charged at a pH at or below physiological pH (e.g. pH 7.4), and neutral at a second pH, preferably at or above physiological pH. It will, of course, be understood that the addition or removal of protons as a function of pH is an equilibrium process, and that the reference to a charged or a neutral lipid refers to the nature of the predominant species and does not require that all of the lipid be present in the charged or neutral form. Lipids that have more than one protonatable or deprotonatable group, or which are zwitterionic, are not excluded from use in the invention.

In some embodiments, the protonatable lipids have a pKa of the protonatable group in the range of about 4 to about 11, e.g., a pKa of about 5 to about 7.

LNPs can include two or more cationic lipids. The cationic lipids can be selected to contribute different advantageous properties. For example, cationic lipids that differ in properties such as amine pKa, chemical stability, half-life in circulation, half-life in tissue, net accumulation in tissue, or toxicity can be used in the LNP. In particular, the cationic lipids can be chosen so that the properties of the mixed-LNP are more desirable than the properties of a single-LNP of individual lipids.

In some embodiments, the cationic lipid is present in a ratio of from about 20 mol % to about 70 or 75 mol % or from about 45 to about 65 mol % or about 20, 25, 30, 35, 40, 45, 50, 55, 60, 65, or about 70 mol % of the total lipid present in the LNP. In further embodiments, the LNPs comprise from about 25% to about 75% on a molar basis of cationic lipid, e.g., from about 20 to about 70%, from about 35 to about 65%, from about 45 to about 65%, about 60%, about 50% or about 40% on a molar basis (based upon 100% total moles of lipid in the lipid nanoparticle). In some embodiments, the ratio of cationic lipid to nucleic acid is from about 3 to about 15, such as from about 5 to about 13 or from about 7 to about 11.

Other suitable (cationic) lipids are disclosed in WO2009/086558, WO2009/127060, WO2010/048536, WO2010/054406, WO2010/088537, WO2010/129709, WO2011/153493, US2011/0256175, US2012/0128760, US2012/0027803, US8158601, WO2016118724, WO2016118725, WO2017070613, WO2017070620, WO2017099823, and WO2017112865. In that context, the disclosures of WO2009/086558, WO2009/127060, WO2010/048536, WO2010/054406, WO2010/088537, WO2010/129709, WO2011/153493, US2011/0256175, US2012/0128760, US2012/0027803, US8158601, WO2016118724, WO2016118725, WO2017070613, WO2017070620, WO2017099823, and WO2017112865 specifically relating to (cationic) lipids suitable for LNPs are incorporated herewith by reference.

### (ii) Neutral and non-cationic lipids

The non-cationic lipid can be a neutral lipid, an anionic lipid, or an amphipathic lipid. Neutral lipids, when present, can be any of a number of lipid species which exist either in an uncharged or neutral zwitterionic form at physiological pH. Such lipids include, for example, diacylphosphatidylcholine, diacylphosphatidylethanolamine, ceramide, sphingomyelin, dihydrosphingomyelin, cephalin, and cerebrosides. The selection of neutral lipids for use in the particles described herein is generally guided by consideration of, e.g., LNP size and stability of the LNP in the bloodstream. Preferably, the neutral lipid is a lipid having two acyl groups (e.g., diacylphosphatidylcholine and diacylphosphatidylethanolamine).

In some embodiments, the neutral lipids contain saturated fatty acids with carbon chain lengths in the range of C10 to C20. In other embodiments, neutral lipids with mono or diunsaturated fatty acids with carbon chain lengths in the range of C10 to C20 are used. Additionally, neutral lipids having mixtures of saturated and unsaturated fatty acid chains can be used.

Suitable neutral lipids include, but are not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl- phosphatidylethanolamine (DOPE), palmitoyloleoylphosphatidylcholine (POPC), palmitoyloleoylphosphatidylethanolamine (POPE), dioleoyl-phosphatidylethanolamine 4-(N- maleimidomethyl)-cyclohexane-I- carboxylate (DOPE-mal), dipalmitoyl phosphatidyl ethanolamine (DPPE), dimyristoylphosphoethanolamine (DMPE), dimyristoyl phosphatidylcholine (DMPC), distearoyl-phosphatidyl-ethanolamine (DSPE), SM, 16-0- monomethyl PE, 16-O-dimethyl PE, 18-1 -trans PE, I-stearoyl-2-oleoyl-phosphatidyethanolamine (SOPE), cholesterol, or a mixture thereof. Anionic lipids suitable for use in LNPs include, but are not limited to, phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, lysylphosphatidylglycerol, and other anionic modifying groups joined to neutral lipids.

Amphipathic lipids refer to any suitable material, wherein the hydrophobic portion of the lipid material orients into a hydrophobic phase, while the hydrophilic portion orients toward the aqueous phase. Such compounds include, but are not limited to, phospholipids, aminolipids, and sphingolipids. Representative phospholipids include sphingomyelin, phosphatidylcholine, phosphatidylethanolamine, phosphatidylserine, phosphatidylinositol, phosphatidic acid, palmitoyloleoyl phosphatdylcholine, lysophosphatidylcholine, lysophosphatidylethanolamine, dipalmitoylphosphatidylcholine, dioleoylphosphatidylcholine, distearoylphosphatidylcholine, or dilinoleoylphosphatidylcholine. Other phosphorus-lacking compounds, such as sphingolipids, glycosphingolipid families, diacylglycerols, and beta-acyloxyacids, can also be used.

In some embodiments, the non-cationic lipid is present in a ratio of from about 5 mol % to about 90 mol %, about 5 mol % to about 10 mol %, about 5, 10, 15, 20, 25, 30, 35, 40, 45, 50, 55, 60, 65, 70, 75, 80, 85, or about 90 mol % of the total lipid present in the LNP.

In some embodiments, LNPs comprise from about 0% to about 15 or 45% on a molar basis of neutral lipid, e.g., from about 3 to about 12% or from about 5 to about 10%. For instance, LNPs may include about 15%, about 10%, about 7.5%, or about 7.1% of neutral lipid on a molar basis (based upon 100% total moles of lipid in the LNP).

### (iii) Sterols

The sterol is preferably cholesterol.

The sterol can be present in a ratio of about 10 mol % to about 60 mol % or about 25 mol % to about 40 mol % of the LNP. In some embodiments, the sterol is present in a ratio of about 10, 15, 20, 25, 30, 35, 40, 45, 50, 55, or about 60 mol % of the total lipid present in the LNP. In other embodiments, LNPs comprise from about 5% to about 50% on a molar basis of the sterol, e.g., about 15% to about 45%, about 20% to about 40%, about 48%, about 40%, about 38.5%, about 35%, about 34.4%, about 31.5% or about 31% on a molar basis (based upon 100% total moles of lipid in the LNP).

### (iv) Aggregation Reducing Agents

The aggregation reducing agent can be a lipid capable of reducing aggregation.

Examples of such lipids include, but are not limited to, polyethylene glycol (PEG)-modified lipids, monosialoganglioside Gml, and polyamide oligomers (PAO) such as those described in U.S. Patent No. 6,320,017, which is incorporated by reference in its entirety. Other compounds with uncharged, hydrophilic, steric-barrier moieties, which prevent aggregation during formulation, like PEG, Gml or ATTA, can also be coupled to lipids. ATTA-lipids are described, e.g., in U.S. Patent No. 6,320,017, and PEG-lipid conjugates are described, e.g. , in U.S. Patent Nos. 5,820,873, 5,534,499 and 5,885,613, each of which is incorporated by reference in its entirety.

The aggregation reducing agent may be, for example, selected from a polyethyleneglycol (PEG)-lipid including, without limitation, a PEG-diacylglycerol (DAG), a PEG-dialkylglycerol, a PEG-dialkyloxypropyl (DAA), a PEG-phospholipid, a PEG-ceramide (Cer), or a mixture thereof (such as PEG-Cer14 or PEG-Cer20). The PEG-DAA conjugate may be, for example, a PEG- dilauryloxypropyl (C12), a PEG-dimyristyloxypropyl (C14), a PEG-dipalmityloxypropyl (C16), or a PEG- distearyloxypropyl (C18). Other pegylated-lipids include, but are not limited to, polyethylene glycol-didimyristoyl glycerol (C14-PEG or PEG-C14, where PEG has an average molecular weight of 2000 Da) (PEG-DMG); (R)-2,3-bis(octadecyloxy)propyl-1-(methoxy poly(ethylene glycol)2000)propylcarbamate) (PEG-DSG); PEG-carbamoyl-1,2-dimyristyloxypropylamine, in which PEG has an average molecular weight of 2000 Da (PEG-cDMA); N-Acetylgalactosamine-((R)-2,3-bis(octadecyloxy)propyl-1-(methoxypoly(ethylene glycol)2000)propylcarbamate)) (GalNAc-PEG-DSG); mPEG (mw2000)-diastearoylphosphatidyl-ethanolamine (PEG-DSPE); and polyethylene glycol-dipalmitoylglycerol (PEG-DPG).

In some embodiments, the aggregation reducing agent is PEG-DMG. In other embodiments, the aggregation reducing agent is PEG-c-DMA.

Further examples of PEG-lipids suitable in that context are provided in US20150376115A1 and WO2015199952, each of which is incorporated by reference in its entirety.

### LNP composition

The composition of LNPs may be influenced by, *inter alia,* the selection of the cationic lipid component, the degree of cationic lipid saturation, the nature of the PEGylation, the ratio of all components and biophysical parameters such as its size. In one example by Semple et al. (Semple et al. Nature Biotech. 2010 28: 172-176; herein incorporated by reference in its entirety), the LNP composition was composed of 57.1 % cationic lipid, 7.1% dipalmitoylphosphatidylcholine, 34.3 % cholesterol, and 1.4% PEG-c-DMA (Basha et al. Mol Ther. 2011 19:2186-2200; herein incorporated by reference in its entirety).

In some embodiments, LNPs may comprise from about 35 to about 45% cationic lipid, from about 40% to about 50% cationic lipid, from about 50% to about 60% cationic lipid and/or from about 55% to about 65% cationic lipid. In some embodiments, the ratio of lipid to (epitope-encoding) RNA (or nucleic acid)may range from about 5: 1 to about 20: 1, from about 10: 1 to about 25: 1, from about 15: 1 to about 30: 1 and/or at least 30: 1.

The average molecular weight of the PEG moiety in the PEG-modified lipids can range from about 500 to about 8,000 Daltons (e.g., from about 1,000 to about 4,000 Daltons). In one preferred embodiment, the average molecular weight of the PEG moiety is about 2,000 Daltons.

The concentration of the aggregation reducing agent may range from about 0.1 to about 15 mol %, per 100% total moles of lipid in the LNP. In some embodiments, LNPs include less than about 3, 2, or 1 mole percent of PEG or PEG-modified lipid, based on the total moles of lipid in the LNP. In further embodiments, LNPs comprise from about 0.1% to about 20% of the PEG-modified lipid on a molar basis, e.g., about 0.5 to about 10%, about 0.5 to about 5%, about 10%, about 5%, about 3.5%, about 1.5%, about 0.5%, or about 0.3% on a molar basis (based on 100% total moles of lipids in the LNP).

Different LNPs having varying molar ratios of cationic lipid, non-cationic (or neutral) lipid, sterol (e.g., cholesterol), and aggregation reducing agent (such as a PEG- modified lipid) on a molar basis (based upon the total moles of lipid in the lipid nanoparticles) as depicted in Table 2 below:.

**Table 2: Lipid-based formulations**

| Formulation No. | Molar Ratio of Lipids (Based upon 100% total moles of lipid in the lipid nanoparticle) | | | |
|---|---|---|---|---|
| | Cationic Lipid | Non-Cationic (or Neutral) Lipid | Sterol | Aggregation Reducing Agent (e.g., PEG-lipid) |
| 1 | from about 35 to about 65% | from about 3 to about 12 or 15% | from about 15 to about 45% | from about 0.1 to about 10% (preferably from about 0.5 to about 2 or 3%) |
| 2 | from about 20 to about 70% | from about 5 to about 45% | from about 20 to about 55% | from about 0.1 to about 10% (preferably from about 0.5 to about 2 or 3%) |
| 3 | from about 45 to about 65% | from about 5 to about 10% | from about 25 to about 40% | from about 0.1 to about 3% |
| 4 | from about 20 to about 60% | from about 5 to about 25% | from about 25 to about 55% | from about 0.1 to about 5% (preferably from about 0.1 to about 3%) |
| 5 | about 40% | about 10% | about 40% | about 10% |
| 6 | about 35% | about 15% | about 40% | about 10% |
| 7 | about 52% | about 13% | about 30% | about 5% |
| 8 | about 50% | about 10% | about 38.5% | about 1.5% |

In some embodiments, LNPs occur as liposomes or lipoplexes as described in further detail below.

### LNP size

In some embodiments, LNPs have a median diameter size of from about 50 nm to about 300 nm, such as from about 50 nm to about 250 nm, for example, from about 50 nm to about 200 nm.

In some embodiments, smaller LNPs may be used. Such particles may comprise a diameter from below 0.1 um up to 100 nm such as, but not limited to, less than 0.1 um, less than 1.0 um, less than 5 um, less than 10 um, less than 15 um, less than 20 um, less than 25 um, less than 30 um, less than 35 um, less than 40 um, less than 50 um, less than 55 um, less than 60 um, less than 65 um, less than 70 um, less than 75 um, less than 80 um, less than 85 um, less than 90 um, less than 95 um, less than 100 um, less than 125 um, less than 150 um, less than 175 um, less than 200 um, less than 225 um, less than 250 um, less than 275 um, less than 300 um, less than 325 um, less than 350 um, less than 375 um, less than 400 um, less than 425 um, less than 450 um, less than 475 um, less than 500 um, less than 525 um, less than 550 um, less than 575 um, less than 600 um, less than 625 um, less than 650 um, less than 675 um, less than 700 um, less than 725 um, less than 750 um, less than 775 um, less than 800 um, less than 825 um, less than 850 um, less than 875 um, less than 900 um, less than 925 um, less than 950 um, less than 975 um, In another embodiment, nucleic acids may be delivered using smaller LNPs which may comprise a diameter from about 1 nm to about 100 nm, from about 1 nm to about 10 nm, about 1 nm to about 20 nm, from about 1 nm to about 30 nm, from about 1 nm to about 40 nm, from about 1 nm to about 50 nm, from about 1 nm to about 60 nm, from about 1 nm to about 70 nm, from about 1 nm to about 80 nm, from about 1 nm to about 90 nm, from about 5 nm to about from 100 nm, from about 5 nm to about 10 nm, about 5 nm to about 20 nm, from about 5 nm to about 30 nm, from about 5 nm to about 40 nm, from about 5 nm to about 50 nm, from about 5 nm to about 60 nm, from about 5 nm to about 70 nm, from about 5 nm to about 80 nm, from about 5 nm to about 90 nm, about 10 to about 50 nM, from about 20 to about 50 nm, from about 30 to about 50 nm, from about 40 to about 50 nm, from about 20 to about 60 nm, from about 30 to about 60 nm, from about 40 to about 60 nm, from about 20 to about 70 nm, from about 30 to about 70 nm, from about 40 to about 70 nm, from about 50 to about 70 nm, from about 60 to about 70 nm, from about 20 to about 80 nm, from about 30 to about 80 nm, from about 40 to about 80 nm, from about 50 to about 80 nm, from about 60 to about 80 nm, from about 20 to about 90 nm, from about 30 to about 90 nm, from about 40 to about 90 nm, from about 50 to about 90 nm, from about 60 to about 90 nm and/or from about 70 to about 90 nm.

In some embodiments, the LNP may have a diameter greater than 100 nm, greater than 150 nm, greater than 200 nm, greater than 250 nm, greater than 300 nm, greater than 350 nm, greater than 400 nm, greater than 450 nm, greater than 500 nm, greater than 550 nm, greater than 600 nm, greater than 650 nm, greater than 700 nm, greater than 750 nm, greater than 800 nm, greater than 850 nm, greater than 900 nm, greater than 950 nm or greater than 1000 nm.

In other embodiments, LNPs have a single mode particle size distribution (i.e., they are not bi- or poly-modal).

### Other components

LNPs may further comprise one or more lipids and/or other components in addition to those mentioned above.

Other lipids may be included in the liposome compositions for a variety of purposes, such as to prevent lipid oxidation or to attach ligands onto the liposome surface. Any of a number of lipids may be present in LNPs, including amphipathic, neutral, cationic, and anionic lipids. Such lipids can be used alone or in combination.

Additional components that may be present in a LNP include bilayer stabilizing components such as polyamide oligomers (see, e.g., U.S. Patent No. 6,320,017, which is incorporated by reference in its entirety), peptides, proteins, and detergents.

### Liposomes

In some embodiments, epitope-encoding RNAs of the inventive combination, (pharmaceutical) composition or vaccine (or any other nucleic acid, in particular RNA, as defined herein) are formulated as liposomes.

Cationic lipid-based liposomes are able to complex with negatively charged nucleic acids (e.g. RNAs) via electrostatic interactions, resulting in complexes that offer biocompatibility, low toxicity, and the possibility of the large-scale production required for in vivo clinical applications. Liposomes can fuse with the plasma membrane for uptake; once inside the cell, the liposomes are processed via the endocytic pathway and the nucleic acid is then released from the endosome/carrier into the cytoplasm. Liposomes have long been perceived as drug delivery vehicles because of their superior biocompatibility, given that liposomes are basically analogs of biological membranes, and can be prepared from both natural and synthetic phospholipids (Int J Nanomedicine. 2014; 9: 1833-1843).

Liposomes typically consist of a lipid bilayer that can be composed of cationic, anionic, or neutral (phospho)lipids and cholesterol, which encloses an aqueous core. Both the lipid bilayer and the aqueous space can incorporate hydrophobic or hydrophilic compounds, respectively. Liposomes may have one or more lipid membranes. Liposomes can be single-layered, referred to as unilamellar, or multi-layered, referred to as multilamellar.

Liposome characteristics and behaviour *in vivo* can be modified by addition of a hydrophilic polymer coating, e.g. polyethylene glycol (PEG), to the liposome surface to confer steric stabilization. Furthermore, liposomes can be used for specific targeting by attaching ligands (e.g., antibodies, peptides, and carbohydrates) to its surface or to the terminal end of the attached PEG chains (Front Pharmacol. 2015 Dec 1;6:286).

Liposomes are typically present as spherical vesicles and can range in size from 20 nm to a few microns.

Liposomes can be of different sizes such as, but not limited to, a multilamellar vesicle (MLV) which may be hundreds of nanometers in diameter and may contain a series of concentric bilayers separated by narrow aqueous compartments, a small unicellular vesicle (SUV) which may be smaller than 50 nm in diameter, and a large unilamellar vesicle (LUV) which may be between 50 and 500 nm in diameter. Liposome design may include, but is not limited to, opsonins or ligands in order to improve the attachment of liposomes to unhealthy tissue or to activate events such as, but not limited to, endocytosis. Liposomes may contain a low or a high pH in order to improve the delivery of the pharmaceutical formulations.

As a non-limiting example, liposomes such as synthetic membrane vesicles may be prepared by the methods, apparatus and devices described in US Patent Publication No. US20130177638, US20130177637, US20130177636, US20130177635, US20130177634, US20130177633, US20130183375, US20130183373 and US20130183372, the contents of each of which are herein incorporated by reference in its entirety. The epitope-encoding RNA of the inventive combination, (pharmaceutical) composition or vaccine (or any other nucleic acid, in particular RNA, as defined herein), may be encapsulated by the liposome and/or it may be contained in an aqueous core which may then be encapsulated by the liposome (see International Pub. Nos. WO2012031046, WO2012031043, WO2012030901 and WO2012006378 and US Patent Publication No. US20130189351, US20130195969 and US20130202684; the contents of each of which are herein incorporated by reference in their entirety).

In some embodiments, the epitope-encoding RNA of the inventive combination, (pharmaceutical) composition or vaccine (or any other nucleic acid, in particular RNA, as defined herein), may be formulated in liposomes such as, but not limited to, DiLa2 liposomes (Marina Biotech, Bothell, WA), SMARTICLES^{®} (Marina Biotech, Bothell, WA), neutral DOPC (I,2-dioleoyl-sn-glycero-3-phosphocholine) based liposomes (e.g., siRNA delivery for ovarian cancer (Landen et al. Cancer Biology & Therapy 2006 5(12)1708-1713); herein incorporated by reference in its entirety) and hyaluronan-coated liposomes (Quiet Therapeutics, Israel).

### Lipoplexes

In some embodiments, epitope-encoding RNAs of the inventive combination, (pharmaceutical) composition or vaccine (or any other nucleic acid, in particular RNA, as defined herein) are formulated as lipoplexes, i.e. cationic lipid bilayers sandwiched between nucleic acid (e.g. (epitope-encoding) RNA or DNA) layers.

Cationic lipids, such as DOTAP, (1,2-dioleoyl-3-trimethylammonium-propane) and DOTMA (N-[1-(2,3-dioleoyloxy)propyl]-N,N,N-trimethyl-ammonium methyl sulfate) can form complexes or lipoplexes with negatively charged nucleic acids to form nanoparticles by electrostatic interaction, providing high *in vitro* transfection efficiency .

### Nanoliposomes

In some embodiments, epitope-encoding RNAs of the inventive combination, (pharmaceutical) composition or vaccine (or any other nucleic acid, in particular RNA, as defined herein) are formulated as neutral lipid-based nanoliposomes such as 1,2-dioleoyl-sn-glycero-3- phosphatidylcholine (DOPC)-based nanoliposomes (Adv Drug Deliv Rev. 2014 Feb; 66: 110-116.).

### Emulsions

In some embodiments, epitope-encoding RNAs of the inventive (pharmaceutical) composition or vaccine (or any other nucleic acid, in particular RNA, as defined herein) are formulated as emulsions. In another embodiment, said epitope-encoding RNAs (and optionally inhibitors) are formulated in a cationic oil-in-water emulsion where the emulsion particle comprises an oil core and a cationic lipid which can interact with the nucleic acid(s) anchoring the molecule to the emulsion particle (see International Pub. No. WO2012006380; herein incorporated by reference in its entirety). In some embodiments, said RNA is formulated in a water-in-oil emulsion comprising a continuous hydrophobic phase in which the hydrophilic phase is dispersed. As a non-limiting example, the emulsion may be made by the methods described in International Publication No. WO201087791, the contents of which are herein incorporated by reference in its entirety.

### (Poly-)cationic compounds and carriers

In preferred embodiments, the epitope-encoding RNA of the inventive combination, (pharmaceutical) composition or vaccine (or any other nucleic acid, in particular RNA, as defined herein) is is complexed or associated with a cationic or polycationic compound ("(poly-)cationic compound") and/or a polymeric carrier.

The term "(poly-)cationic compound" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4.

Accordingly, a "(poly-)cationic compound" may be any positively charged compound or polymer, preferably a cationic peptide or protein, which is positively charged under physiological conditions, particularly under physiological conditions in vivo. A "(poly-)cationic peptide or protein" may contain at least one positively charged amino acid, or more than one positively charged amino acid, e.g. selected from Arg, His, Lys or Orn.

### (Poly-)cationic amino acids, peptides and proteins

(Poly-)cationic compounds being particularly preferred agents for complexation or association of the epitope-encoding RNA of the inventive combination, (pharmaceutical) composition or vaccine (or any other nucleic acid, in particular RNA, as defined herein) include protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia),* pAntp, plsl, FGF, Lactoferrin, Transportan, Buforin-2, Bac715-24, SynB, SynB(1), pVEC, hCT-derived peptides, SAP, or histones.

More preferably, the epitope-encoding RNA of the inventive combination, (pharmaceutical) composition or vaccine (or any other nucleic acid, in particular RNA, as defined herein) is complexed with one or more polycations, preferably with protamine or oligofectamine (discussed below), most preferably with protamine. In this context protamine is particularly preferred.

Additionally, preferred (poly-)cationic proteins or peptides may be selected from the following proteins or peptides having the following total formula (III):

(Arg)ₗ;(Lys)ₘ;(His)ₙ;(Orn)ₒ;(Xaa)ₓ, formula (III)

wherein I + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred cationic peptides in this context are e.g. Arg₇, Arg₈, Arg₉, H₃R₉, R₉H₃, H₃R₉H₃, YSSR₉SSY, (RKH)₄, Y(RKH)₂R, etc. In this context the disclosure of WO 2009/030481 is incorporated herewith by reference.

### (Poly-)cationic polysaccharides

Further preferred (poly-)cationic compounds for complexation of or association with the epitope-encoding RNA of the inventive combination, (pharmaceutical) composition or vaccine (or any other nucleic acid, in particular RNA, as defined herein) include (poly-)cationic polysaccharides, e.g. chitosan, polybrene, cationic polymers, e.g. polyethyleneimine (PEI).

### (Poly-)cationic lipids

Further preferred (poly-)cationic compounds for complexation of or association with the epitope-encoding RNA of the inventive combination, (pharmaceutical) composition or vaccine (or any other nucleic acid, in particular RNA, as defined herein) include (poly-)cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(alpha-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, or oligofectamine.

### (Poly-)cationic polymers

Further preferred (poly-)cationic compounds for complexation of or association with the epitope-encoding RNA of the inventive combination, (pharmaceutical) composition or vaccine (or any other nucleic acid, in particular RNA, as defined herein) include (poly-)cationic polymers, e.g. modified polyaminoacids, such as beta-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g. polyethyleneglycole); etc.

### Polymeric carriers

According to preferred embodiments, epitope-encoding RNA of the inventive combination, (pharmaceutical) composition or vaccine (or any other nucleic acid, in particular RNA, as defined herein) is complexed or associated with a polymeric carrier.

A "polymeric carrier" used according to the invention might be a polymeric carrier formed by disulfide-crosslinked cationic components. The disulfide-crosslinked cationic components may be the same or different from each other. The polymeric carrier can also contain further components.

It is also particularly preferred that the polymeric carrier used according to the present invention comprises mixtures of cationic peptides, proteins or polymers and optionally further components as defined herein, which are crosslinked by disulfide bonds as described herein. In this context, the disclosure of WO 2012/013326 is incorporated herewith by reference.

In this context, the cationic components, which form basis for the polymeric carrier by disulfide-crosslinkage, are typically selected from any suitable (poly-)cationic peptide, protein or polymer suitable for this purpose, particular any (poly-)cationic peptide, protein or polymer capable of complexing, and thereby preferably condensing, the epitope-encoding RNA of the inventive combination, (pharmaceutical) composition or vaccine (or any other nucleic acid, in particular RNA, as defined herein). The (poly-)cationic peptide, protein or polymer, is preferably a linear molecule, however, branched (poly-)cationic peptides, proteins or polymers may also be used.

Every disulfide-crosslinking (poly-)cationic protein, peptide or polymer of the polymeric carrier, which may be used to complex the epitope-encoding RNA of the (pharmaceutical) composition or vaccine (or any other nucleic acid, in particular RNA, as defined herein) contains at least one -SH moiety, most preferably at least one cysteine residue or any further chemical group exhibiting an -SH moiety, capable of forming a disulfide linkage upon condensation with at least one further (poly-)cationic protein, peptide or polymer as cationic component of the polymeric carrier as mentioned herein.

As defined above, the polymeric carrier, which may be used to complex the epitope-encoding RNA of the inventive combination, (pharmaceutical) composition or vaccine (or any other nucleic acid, in particular RNA, as defined herein) may be formed by disulfide-crosslinked cationic (or polycationic) components. Preferably, such (poly-)cationic peptides or proteins or polymers of the polymeric carrier, which comprise or are additionally modified to comprise at least one - SH moiety, are selected from, proteins, peptides and polymers as defined herein.

In some embodiments, the polymeric carrier may be selected from a polymeric carrier molecule according to generic formula (IV):

L-P¹-S-[S-P²-S]ₙ-S-P³-L formula (IV)

wherein,
P¹ and P³ are different or identical to each other and represent a linear or branched hydrophilic polymer chain, each P¹ and P³ exhibiting at least one -SH-moiety, capable to form a disulfide linkage upon condensation with component P², or alternatively with (AA), (AA)ₓ, or [(AA)ₓ]_{z} if such components are used as a linker between P¹ and P² or P³ and P²) and/or with further components (e.g. (AA), (AA)ₓ, [(AA)ₓ]_{z} or L), the linear or branched hydrophilic polymer chain selected independent from each other from polyethylene glycol (PEG), poly-N-(2-hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine), wherein the hydrophilic polymer chain exhibits a molecular weight of about 1 kDa to about 100 kDa, preferably of about 2 kDa to about 25 kDa; or more preferably of about 2 kDa to about 10 kDa, e.g. about 5 kDa to about 25 kDa or 5 kDa to about 10 kDa;
P² is a (poly-)cationic peptide or protein, e.g. as defined above for the polymeric carrier formed by disulfide-crosslinked cationic components, and preferably having a length of about 3 to about 100 amino acids, more preferably having a length of about 3 to about 50 amino acids, even more preferably having a length of about 3 to about 25 amino acids, e.g. a length of about 3 to 10, 5 to 15, 10 to 20 or 15 to 25 amino acids, more preferably a length of about 5 to about 20 and even more preferably a length of about 10 to about 20; or
is a (poly-)cationic polymer, e.g. as defined above for the polymeric carrier formed by disulfide-crosslinked cationic components, typically having a molecular weight of about 0.5 kDa to about 30 kDa, including a molecular weight of about 1 kDa to about 20 kDa, even more preferably of about 1.5 kDa to about 10 kDa, or having a molecular weight of about 0.5 kDa to about 100 kDa, including a molecular weight of about 10 kDa to about 50 kDa, even more preferably of about 10 kDa to about 30 kDa;
each P² exhibiting at least two -SH-moieties, capable to form a disulfide linkage upon condensation with further components P² or component(s) P¹ and/or P² or alternatively with further components (e.g. (AA), (AA)ₓ, or [(AA)ₓ]_{z}); -S-S- is a (reversible) disulfide bond (the brackets are omitted for better readability), wherein S preferably represents sulphur or a -SH carrying moiety, which has formed a (reversible) disulfide bond. The (reversible) disulfide bond is preferably formed by condensation of -SH-moieties of either components P¹ and P², P² and P², or P² and P², or optionally of further components as defined herein (e.g. L, (AA), (AA)ₓ, [(AA)ₓ]_{z}, etc); The -SH-moiety may be part of the structure of these components or added by a modification as defined below;
L is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, (e.g. TAT or KALA), a ligand of a receptor (e.g. cytokines, hormones, growth factors etc), small molecules (e.g. carbohydrates like mannose or galactose or synthetic ligands), small molecule agonists, inhibitors or antagonists of receptors (e.g. RGD peptidomimetic analogues), or any further protein as defined herein, etc.;
n is an integer, typically selected from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 4 to 9, 4 to 10, 3 to 20, 4 to 20, 5 to 20, or 10 to 20, or a range of about 3 to 15, 4 to 15, 5 to 15, or 10 to 15, or a range of about 6 to 11 or 7 to 10. Most preferably, n is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

In this context, the disclosure of WO 2011/026641 is incorporated herewith by reference. Each of hydrophilic polymers P¹ and P³ typically exhibits at least one -SH-moiety, wherein the at least one -SH-moiety is capable to form a disulfide linkage upon reaction with component P² or with component (AA) or (AA)ₓ, if used as linker between P¹ and P² or P³ and P² as defined below and optionally with a further component, e.g. L and/or (AA) or (AA)ₓ, e.g. if two or more -SH-moieties are contained. The following subformulae "P¹-S-S-P²" and "P²-S-S-P³" within generic formula (IV) above (the brackets are omitted for better readability), wherein any of S, P¹ and P³ are as defined herein, typically represent a situation, wherein one-SH-moiety of hydrophilic polymers P¹ and P³ was condensed with one -SH-moiety of component P² of generic formula (IV) above, wherein both sulphurs of these -SH-moieties form a disulfide bond -S-S-as defined herein in formula (IV). These -SH-moieties are typically provided by each of the hydrophilic polymers P¹ and P², e.g. via an internal cysteine or any further (modified) amino acid or compound which carries a -SH moiety. Accordingly, the subformulae "P¹-S-S-P²" and "P²-S-S-P³" may also be written as "P¹-Cys-Cys-P²" and "P²-Cys-Cys-P³", if the -SH- moiety is provided by a cysteine, wherein the term Cys-Cys represents two cysteines coupled via a disulfide bond, not via a peptide bond. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH-moieties to form a disulfide bond. Accordingly, the term "-Cys-Cys-" also may be understood generally as "-(Cys-S)-(S-Cys)-", wherein in this specific case S indicates the sulphur of the -SH-moiety of cysteine. Likewise, the terms "-S-Cys" and "-Cys-S" indicate a disulfide bond between a -SH containing moiety and a cysteine, which may also be written as "-S-(S-Cys)" and "-(Cys-S)-S". Alternatively, the hydrophilic polymers P¹ and P³ may be modified with a -SH moiety, preferably via a chemical reaction with a compound carrying a -SH moiety, such that each of the hydrophilic polymers P¹ and P³ carries at least one such -SH moiety. Such a compound carrying a -SH moiety may be e.g. an (additional) cysteine or any further (modified) amino acid, which carries a -SH moiety. Such a compound may also be any non-amino compound or moiety, which contains or allows to introduce a -SH moiety into hydrophilic polymers P¹ and P³ as defined herein. Such non-amino compounds may be attached to the hydrophilic polymers P¹ and P³ of formula (IV) of the polymeric carrier according to the present invention via chemical reactions or binding of compounds, e.g. by binding of a 3-thio propionic acid or thioimolane, by amide formation (e.g. carboxylic acids, sulphonic acids, amines, etc), by Michael addition (e.g maleinimide moieties, α,β-unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components. A particularly preferred PEG derivate in this context is alpha-Methoxy-omega-mercapto poly(ethylene glycol). In each case, the SH-moiety, e.g. of a cysteine or of any further (modified) amino acid or compound, may be present at the terminal ends or internally at any position of hydrophilic polymers P¹ and P³. As defined herein, each of hydrophilic polymers P¹ and P³ typically exhibits at least one -SH-moiety preferably at one terminal end, but may also contain two or even more -SH-moieties, which may be used to additionally attach further components as defined herein, preferably further functional peptides or proteins e.g. a ligand, an amino acid component (AA) or (AA)ₓ, antibodies, cell penetrating peptides or enhancer peptides (e.g. TAT, KALA), etc.

### Weight ratio and N/P ratio

In preferred embodiments of the invention, the epitope-encoding RNA (or said other nucleic acid, in particular RNA) is associated with or complexed with a (poly-)cationic compound or a polymeric carrier, optionally in a weight ratio selected from a range of about 6:1 *(w*/*w)* to about 0.25:1 (w/w), more preferably from about 5:1 (w/w) to about 0.5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w) of epitope-encoding (or other) RNA to (poly-)cationic compound and/or polymeric carrier; or optionally in a nitrogen/phosphate (N/P) ratio of epitope-encoding (or other) RNA to (poly-)cationic compound and/or polymeric carrier in the range of about 0.1-10, preferably in a range of about 0.3-4 or 0.3-1, and most preferably in a range of about 0.5-1 or 0.7-1, and even most preferably in a range of about 0.3-0.9 or 0.5-0.9. More preferably, the N/P ratio of the at least one epitope-encoding (or other) RNA to the one or more polycations is in the range of about 0.1 to 10, including a range of about 0.3 to 4, of about 0.5 to 2, of about 0.7 to 2 and of about 0.7 to 1.5.

The epitope-encoding RNA of the inventive combination, (pharmaceutical) composition or vaccine (or any other nucleic acid, in particular RNA, as defined herein) can also be associated with a vehicle, transfection or complexation agent for increasing the transfection efficiency and/or the immunostimulatory properties of said epitope-encoding RNA.

The inventive combination, (pharmaceutical) composition or vaccine may comprise, in addition to the at least one PD-1 pathway inhibitor and at least one LAG-3 pathway inhibitor as defined herein, at least one epitope-encoding RNA which is complexed with one or more (poly-)cationic compounds and/or polymeric carriers, and at least one "free" epitope-encoding RNA, wherein the at least one complexed RNA is preferably identical to the at least one "free" RNA.

In this context, it is particularly preferred that the inventive combination, (pharmaceutical) composition or vaccine comprises the epitope-encoding RNA that is complexed at least partially with a (poly-)cationic compound and/or a polymeric carrier, preferably cationic proteins or peptides. In this context, the disclosure of WO 2010/037539 and WO 2012/113513 is incorporated herewith by reference. "Partially" means that only a part of said epitope-encoding RNA is complexed with a (poly-)cationic compound and/or polymeric carrier, while the rest of said epitope-encoding RNA is present in uncomplexed form ("free").

Preferably, the molar ratio of the complexed epitope-encoding RNA to the free epitope-encoding RNA is selected from a molar ratio of about 0.001:1 to about 1:0.001, including a ratio of about 1:1. More preferably the ratio of complexed epitope-encoding RNA to free epitope-encoding RNA is selected from a range of about 5:1 (w/w) to about 1:10 (w/w), more preferably from a range of about 4:1 (w/w) to about 1:8 (w/w), even more preferably from a range of about 3:1 (w/w) to about 1:5 (w/w) or 1:3 (w/w), and most preferably the ratio of complexed epitope-encoding RNA to free epitope-encoding RNA is selected from a ratio of about 1:1 (w/w).

The complexed epitope-encoding RNA of the inventive combination, (pharmaceutical) composition or vaccine is preferably prepared according to a first step by complexing the epitope-encoding RNA with a (poly-)cationic compound and/or with a polymeric carrier, preferably as defined herein, in a specific ratio to form a stable complex. In this context, it is highly preferable, that no free (poly-)cationic compound or polymeric carrier or only a negligibly small amount thereof remains in the fraction of the complexed epitope-encoding RNA after complexing said epitope-encoding RNA. Accordingly, the ratio of the epitope-encoding RNA and the (poly-)cationic compound and/or the polymeric carrier in the fraction of the complexed RNA is typically selected in a range so that the epitope-encoding RNA is entirely complexed and no free (poly-)cationic compound or polymeric carrier or only a negligibly small amount thereof remains in said fraction.

Preferably, the ratio of the epitope-encoding RNA as defined herein to the (poly-)cationic compound and/or the polymeric carrier, preferably as defined herein, is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w).

Alternatively, the ratio of the epitope-encoding RNA as defined herein to the (poly-)cationic compound and/or the polymeric carrier may also be calculated on the basis of the nitrogen/phosphate ratio (N/P-ratio) of the entire complex. In the context of the present invention, an N/P-ratio is preferably in the range of about 0.1-10, preferably in a range of about 0.3-4 and most preferably in a range of about 0.5-2 or 0.7-2 regarding the ratio of epitope-encoding RNA : (poly-)cationic compound and/or polymeric carrier, preferably as defined herein, in the complex, and most preferably in a range of about 0.7-1,5, 0.5-1 or 0.7-1, and even most preferably in a range of about 0.3-0.9 or 0.5-0.9, preferably provided that the (poly-)cationic compound in the complex is a (poly-)cationic protein or peptide and/or the polymeric carrier as defined above.

In other embodiments, epitope-encoding RNA of the inventive combination, (pharmaceutical) composition or vaccine as defined herein may be used in free or naked form without being associated with any further vehicle, transfection or complexation agent.

It has to be understood and recognized, that according to the present invention, the inventive combination, (pharmaceutical) composition or vaccine may comprise, in addition to the at least one PD-1 pathway inhibitor and the at least one LAG-3 pathway inhibitor, at least one free epitope-encoding RNA as defined herein, preferably an mRNA, and/or at least one complexed epitope-encoding RNA as defined herein, preferably an mRNA, wherein every agent disclosed herein may be used for complexation.

### Adjuvants

According to further embodiments, the inventive combination, (pharmaceutical) composition or vaccine may comprise an adjuvant, which is preferably added in order to enhance the immunostimulatory properties of said combination, (pharmaceutical) composition or vaccine.

An adjuvant or an adjuvant component in the broadest sense is typically a pharmacological and/or immunological agent that may modify, e.g. enhance, the effect of other agents, e.g. therapeutic agents or vaccines. In this context, an adjuvant may be understood as any compound, which is suitable to support administration and delivery of the composition according to the invention.

Furthermore, such an adjuvant may, without being bound thereto, initiate or increase an immune response of the innate immune system, i.e. a non-specific immune response."Adjuvants" typically do not elicit an adaptive immune response. Insofar, "adjuvants" do not qualify as antigens. In other words, when administered, the inventive combination, (pharmaceutical) composition or vaccine typically initiates an adaptive immune response due to an epitope, which is encoded by the at least one coding sequence of the epitope-encoding RNA contained in said combination, (pharmaceutical) composition or vaccine. Additionally, an adjuvant present in the combination, (pharmaceutical) composition or vaccine according to the invention may generate an (supportive) innate immune response. In addition, the PD-1 and LAG-3 pathway inhibitors of the inventive combination, (pharmaceutical) composition or vaccine preferably support adaptive immune responses by antagonizing the inhibitory action of the immune checkpoints PD-1 and LAG-3.

Such an adjuvant may be selected from any adjuvant known to a skilled person and suitable for the present case, i.e. supporting the induction of an immune response in a mammal. Preferably, the adjuvant may be selected from the group consisting of, without being limited thereto, TDM, MDP, muramyl dipeptide, pluronics, alum solution, aluminium hydroxide, ADJUMER^{™} (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINE^{™} (propanediamine); BAY R1005^{™} ((N-(2-deoxy-2-L-leucylamino-b-D-glucopyranosyl)-N-octadecyl-dodecanoyl-amide hydroacetate); CALCITRIOL^{™} (1-alpha,25-dihydroxy-vitamin D3); calcium phosphate gel; CAP^{™} (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund"s complete adjuvant; Freund"s incomplete adjuvant; gamma inulin; Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4,5-c]quinoline-4-amine); ImmTher^{™} (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-1beta; interleukin-2; interleukin-7; interleukin-12; ISCOMS^{™}; ISCOPREP 7.0.3.^{™}; liposomes; LOXORIBINE^{™} (7-allyl-8-oxoguanosine); LT oral adjuvant (E.coli labile enterotoxin-protoxin); microspheres and microparticles of any composition; MF59^{™}; (squalene-water emulsion); MONTANIDE ISA 51^{™} (purified incomplete Freund"s adjuvant); MONTANIDE ISA 720^{™} (metabolisable oil adjuvant); MPL^{™} (3-Q-desacyl-4"-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE^{™} (Nac-Mur-L-Ala-D-Gln-OCH3); MURAPALMITINE^{™} and D-MURAPALMITINE^{™} (Nac-Mur-L-Thr-D-isoGln-sn-glyceroldipalmitoyl); NAGO (neuraminidase-galactose oxidase); nanospheres or nanoparticles of any composition; NISVs (non-ionic surfactant vesicles); PLEURAN^{™} (β-glucan); PLGA, PGA and PLA (homo- and co-polymers of lactic acid and glycolic acid; microspheres/nanospheres); PLURONIC L121^{™}; PMMA (polymethyl methacrylate); PODDS^{™} (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON^{™} (QS-21); Quil-A (Quil-A saponin); S-28463 (4-amino-otec-dimethyl-2-ethoxymethyl-1H-imidazo[4,5 c]quinoline-1-ethanol); SAF-1^{™} ("Syntex adjuvant formulation"); Sendai proteoliposomes and Sendai-containing lipid matrices; Span-85 (sorbitan trioleate); Specol (emulsion of Marcol 52, Span 85 and Tween 85); squalene or Robane^{®} (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid^{®} (N-acetylglucosaminyl-N-acetylmuramyl-L-Ala-D-isoGlu-L-Aladipalmitoxypropylamide); Theronyl-MDP (Termurtide^{™} or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Particularly preferred, an adjuvant may be selected from adjuvants, which support induction of a Th1-immune response or maturation of naïve T-cells, such as GM-CSF, IL-12, IFNγ, any immunostimulatory nucleic acid as defined above, preferably an immunostimulatory RNA, CpG DNA, etc.

### (Poly-)cationic compounds

Suitable adjuvants may also be selected from (poly-)cationic compounds as described herein for complexation of the epitope-encoding RNA (or other nucleic acids, in particular RNAs as defined herein). Associating or complexing the epitope-encoding RNA of the (pharmaceutical) composition or vaccine with (poly-)cationic compounds as defined herein preferably provides adjuvant properties and confers a stabilizing effect to the RNA of the composition.

In particular, preferred (poly-)cationic compounds used as adjuvants are selected from (poly-)cationic peptides or proteins as disclosed in the section headed "(Poly-)cationic amino acids, peptides and proteins" in the context of carriers above. Further preferred (poly-)cationic compounds used as adjuvants may include (poly-)cationic polysaccharides as disclosed in the section headed "(Poly-)cationic polysaccharides" in the context of carriers above. Further preferred (poly-)cationic compounds used as adjuvants may include (poly-)cationic lipids as disclosed in the section headed "(Poly-)cationic lipids" in the context of carriers above. Further preferred (poly-)cationic compounds used as adjuvants may include (poly-)cationic polymers as disclosed in the section headed "(Poly-)cationic polymers" in the context of carriers above.

The ratio of the epitope-encoding RNA to the (poly-)cationic compound in the adjuvant component may be calculated on the basis of the nitrogen/phosphate ratio (N/P-ratio) of the entire complex, i.e. the ratio of positively charged (nitrogen) atoms of the (poly-)cationic compound to the negatively charged phosphate atoms of the epitope-encoding RNA. For example, 1 µg of epitope-encoding RNA typically contains about 3 nmol phosphate residues, provided said RNA exhibits a statistical distribution of bases. Additionally, 1 µg of peptide typically contains about x nmol nitrogen residues, dependent on the molecular weight and the number of basic amino acids. When exemplarily calculated for (Arg)₉ (molecular weight 1424 g/mol, 9 nitrogen atoms), 1 µg (Arg)₉ contains about 700 pmol (Arg)₉ and thus 700 x 9=6300 pmol basic amino acids = 6.3 nmol nitrogen atoms. For a mass ratio of about 1:1 RNA/(Arg)9 an N/P ratio of about 2 can be calculated. When exemplarily calculated for protamine (molecular weight about 4250 g/mol, 21 nitrogen atoms, when protamine from salmon is used) with a mass ratio of about 2:1 with 2 µg RNA, 6 nmol phosphate are to be calulated for the RNA; 1 µg protamine contains about 235 pmol protamine molecues and thus 235 x 21 = 4935 pmol basic nitrogen atoms = 4.9 nmol nitrogen atoms. For a mass ratio of about 2:1 RNA/protamine an N/P ratio of about 0.81 can be calculated. For a mass ratio of about 8:1 RNA/protamine an N/P ratio of about 0.2 can be calculated. In the context of the present invention, an N/P-ratio is preferably in the range of about 0.1-10, preferably in a range of about 0.3-4 and most preferably in a range of about 0.5-2 or 0.7-2 regarding the ratio of RNA : peptide in the complex, and most preferably in the range of about 0.7-1.5.

### Preparation

In preferred embodiments, the combination, (pharmaceutical) composition or vaccine of the present invention is obtained in two separate steps in order to obtain both, an efficient immunostimulatory effect and efficient translation of the epitope-encoding RNA comprised in said combination, (pharmaceutical) composition or vaccine.

In a first step, an RNA is complexed with a (poly-)cationic compound in a specific ratio to form a stable complex ("complexed (RNA"). Said RNA can be an epitope-encoding RNA as defined herein, or a different RNA. In this context, it is important, that no free (poly-)cationic compound or only a neglibly small amount remains in the fraction of the complexed RNA. Accordingly, the ratio of the (epitope-encoding) RNA and the (poly-)cationic compound is typically selected in a range that the (epitope-encoding) RNA is entirely complexed and no free (poly-)cationic compound or only a neglectably small amount remains in the composition. Preferably the ratio of the (epitope-encoding) RNA to the (poly-)cationic compound is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w).

According to preferred embodiments, in a second step, an epitope-encoding RNA is added to the complexed (epitope-encoding) RNA in order to form the (immunostimulatory) composition of the invention. Therein, said added epitope-encoding RNA is present as free RNA, preferably as free mRNA, which is not complexed by other compounds. Prior to addition, the free RNA is not complexed and will preferably not undergo any detectable or significant complexation reaction upon the addition to the complexed (epitope-encoding) RNA. This is due to the strong binding of the (poly-)cationic compound to the complexed (epitope-encoding) RNA. In other words, when the free epitope-encoding RNA is added to the complexed (epitope-encoding) RNA, preferably no free or substantially no free (poly-)cationic compound is present, which could form a complex with said free epitope-encoding RNA. Accordingly, the free epitope-encoding RNA of the inventive (pharmaceutical) composition or vaccine can efficiently be transcribed *in vivo.* Therein, the free epitope-encoding RNA , preferably an mRNA, may occur as a mono-, di-, or multicistronic (m)RNA, i.e. an RNA which carries the coding sequences of one or more epitopes (or antigens). Such coding sequences in di-, or even multicistronic mRNA may be separated by at least one IRES sequence, e.g. as defined herein.

In particularly preferred embodiments, the free epitope-encoding RNA as defined herein, which is comprised in the inventive combination, (pharmaceutical) composition or vaccine, may be identical or different to the complexed RNA, depending on the specific requirements of therapy. Even more preferably, the free epitope-encoding RNA, which is comprised in the inventive combination, (pharmaceutical) composition or vaccine, is identical to the complexed epitope-encoding RNA, in other words, the combination, (pharmaceutical) composition or vaccine comprises the epitope-encoding RNA in both free and complexed form.

In particularly preferred embodiments, the inventive combination, (pharmaceutical) composition or vaccine thus comprises the epitope-encoding RNA as defined herein, wherein said epitope-encoding RNA is present in the said combination, (pharmaceutical) composition or vaccine partially as free RNA and partially as complexed RNA. Preferably, the epitope-encoding RNA as defined herein, preferably an mRNA, is complexed as described above and the same epitope-encoding (m)RNA is then added in the form of free RNA, wherein preferably the compound, which is used for complexing the epitope-encoding RNA is not present in free form in the composition at the moment of addition of the free epitope-encoding RNA.

The ratio of the complexed (epitope-encoding) RNA and the free epitope-encoding RNA may be selected depending on the specific requirements of a particular therapy. Typically, the ratio of the complexed (epitope-encoding) RNA and the free epitope-encoding RNA is selected such that a significant stimulation of the innate immune system is elicited due to the presence of the complexed (epitope-encoding) RNA. In parallel, the ratio is selected such that a significant amount of the free epitope-encoding RNA can be provided *in vivo* leading to an efficient translation and concentration of the expressed epitope (or antigen comprising the same) *in vivo.* Preferably the ratio of the complexed (epitope-encoding) RNA to free epitope-encoding RNA in the inventive (pharmaceutical) composition or vaccine is selected from a range of about 5:1 (w/w) to about 1:10 (w/w), more preferably from a range of about 4:1 (w/w) to about 1:8 (w/w), even more preferably from a range of about 3:1 (w/w) to about 1:5 (w/w) or 1:3 (w/w), and most preferably about 1:1 (w/w).

Additionally or alternatively, the ratio of the complexed (epitope-encoding) RNA and the free (epitope-encoding) RNA may be calculated on the basis of the nitrogen/phosphate ratio (N/P-ratio) of the entire RNA complex. In the context of the present invention, an N/P-ratio is preferably in the range of about 0.1-10, preferably in a range of about 0.3-4 and most preferably in a range of about 0.5-2 or 0.7-2 regarding the ratio of RNA : peptide in the complex, and most preferably in the range of about 0.7-1.5.

Additionally or alternatively, the ratio of the complexed (epitope-encoding) RNA and the free epitope-encoding RNA may also be selected on the basis of the molar ratio of both RNAs to each other. Typically, the molar ratio of the complexed (epitope-encoding) RNA to the free epitope-encoding RNA may be selected such, that the molar ratio suffices the above (w/w) and/or N/P-definitions. More preferably, the molar ratio of the complexed (epitope-encoding) RNA to the free epitope-encoding RNA may be selected e.g. from a molar ratio of about 0.001:1, 0.01:1, 0.1:1, 0.2:1, 0.3:1, 0.4:1, 0.5:1, 0.6:1, 0.7:1, 0.8:1, 0.9:1, 1:1, 1:0.9, 1:0.8, 1:0.7, 1:0.6, 1:0.5, 1:0.4, 1:0.3, 1:0.2, 1:0.1, 1:0.01, 1:0.001, etc. or from any range formed by any two of the above values, e.g. a range selected from about 0.001:1 to 1:0.001, including a range of about 0.01:1 to 1:0.001, 0.1:1 to 1:0.001, 0.2:1 to 1:0.001, 0.3:1 to 1:0.001, 0.4:1 to 1:0.001, 0.5:1 to 1:0.001, 0.6:1 to 1:0.001, 0.7:1 to 1:0.001, 0.8:1 to 1:0.001, 0.9:1 to 1:0.001, 1:1 to 1:0.001, 1:0.9 to 1:0.001, 1:0.8 to 1:0.001, 1:0.7 to 1:0.001, 1:0.6 to 1:0.001, 1:0.5 to 1:0.001, 1:0.4 to 1:0.001, 1:0.3 to 1:0.001, 1:0.2 to 1:0.001, 1:0.1 to 1:0.001, 1:0.01 to 1:0.001, or a range of about 0.01:1 to 1:0.01, 0.1:1 to 1:0.01, 0.2:1 to 1:0.01, 0.3:1 to 1:0.01, 0.4:1 to 1:0.01, 0.5:1 to 1:0.01, 0.6:1 to 1:0.01, 0.7:1 to 1:0.01, 0.8:1 to 1:0.01, 0.9:1 to 1:0.01, 1:1 to 1:0.01, 1:0.9 to 1:0.01, 1:0.8 to 1:0.01, 1:0.7 to 1:0.01, 1:0.6 to 1:0.01, 1:0.5 to 1:0.01, 1:0.4 to 1:0.01, 1:0.3 to 1:0.01, 1:0.2 to 1:0.01, 1:0.1 to 1:0.01, 1:0.01 to 1:0.01, or including a range of about 0.001:1 to 1:0.01, 0.001:1 to 1:0.1, 0.001:1 to 1:0.2, 0.001:1 to 1:0.3, 0.001:1 to 1:0.4, 0.001:1 to 1:0.5, 0.001:1 to 1:0.6, 0.001:1 to 1:0.7, 0.001:1 to 1:0.8, 0.001:1 to 1:0.9, 0.001:1 to 1:1, 0.001 to 0.9:1, 0.001 to 0.8:1, 0.001 to 0.7:1, 0.001 to 0.6:1, 0.001 to 0.5:1, 0.001 to 0.4:1, 0.001 to 0.3:1, 0.001 to 0.2:1, 0.001 to 0.1:1, or a range of about 0.01:1 to 1:0.01, 0.01:1 to 1:0.1, 0.01:1 to 1:0.2, 0.01:1 to 1:0.3, 0.01:1 to 1:0.4, 0.01:1 to 1:0.5, 0.01:1 to 1:0.6, 0.01:1 to 1:0.7, 0.01:1 to 1:0.8, 0.01:1 to 1:0.9, 0.01:1 to 1:1, 0.001 to 0.9:1, 0.001 to 0.8:1, 0.001 to 0.7:1, 0.001 to 0.6:1, 0.001 to 0.5:1, 0.001 to 0.4:1, 0.001 to 0.3:1, 0.001 to 0.2:1, 0.001 to 0.1:1, etc.

Even more preferably, the molar ratio of the complexed (epitope-encoding) RNA to the free epitope-encoding RNA may be selected e.g. from a range of about 0.01:1 to 1:0.01. Most preferably, the molar ratio of the complexed (epitope-encoding) RNA to the free epitope-encoding RNA may be selected e.g. from a molar ratio of about 1:1. Any of the above definitions with regard to (w/w) and/or N/P ratio may also apply.

### Adjuvant nucleic acids

Nucleic acids can be used as adjuvants in accordance with the present invention. Such nucleic acids may also be referred to as "immune-stimulatory" or "is" nucleic acids or RNAs. According to a particularly preferred embodiment, the adjuvant nucleic acid comprises a nucleic acid of the following formula (V) or (VI):

GₗXₘGₙ formula (V)

wherein:
- G: is a nucleotide comprising guanine, uracil or an analogue of guanine or uracil;
- X: is a nucleotide comprising guanine, uracil, adenine, thymine, cytosine or an analogue thereof;
- I: is an integer from 1 to 40,
wherein
when I = 1 G is a nucleotide comprising guanine or an analogue thereof,
when l > 1 at least 50% of the nucleotides comprise guanine or an analogue thereof;
- m: is an integer and is at least 3;
wherein
when m = 3, X is a nucleotide comprising uracil or an analogue thereof,
when m > 3, at least 3 successive nucleotides comprising uracils or analogues of uracil occur;
- n: is an integer from 1 to 40,
wherein
when n = 1, G is a nucleotide comprising guanine or an analogue thereof,
when n > 1, at least 50% of the nucleotides comprise guanine or an analogue thereof;

   CₗXₘCₙ formula (VI)

   wherein:
- C: is a nucleotide comprising cytosine, uracil or an analogue of cytosine or uracil;
- X: is a nucleotide comprising guanine, uracil, adenine, thymine, cytosine or an analogue thereof;
- I: is an integer from 1 to 40,
wherein
when l = 1, C is a nucleotide comprising cytosine or an analogue thereof,
when l > 1, at least 50% of the nucleotides comprise cytosine or an analogue thereof;
- m: is an integer and is at least 3;
wherein
when m = 3, X comprises uracil or an analogue thereof,
when m > 3, at least 3 successive nucleotides comprise uracils or analogues of uracil occur;
- n: is an integer from 1 to 40,
wherein
when n = 1, C is a nucleotide comprising cytosine or an analogue thereof,
when n > 1, at least 50% of the nucleotides comprise cytosine or an analogue thereof.

The nucleic acids of formula (V) or (VI), which may be used as isRNA may be relatively short nucleic acid molecules with a typical length of approximately from 5 to 100 (but may also be longer than 100 nucleotides for specific embodiments, e.g. up to 200 nucleotides), from 5 to 90 or from 5 to 80 nucleotides, preferably a length of approximately from 5 to 70, more preferably a length of approximately from 8 to 60 and, more preferably a length of approximately from 15 to 60 nucleotides, more preferably from 20 to 60, most preferably from 30 to 60 nucleotides. If the epitope-encoding RNA (or any other nucleic acid, in particular RNA, as disclosed herein) has a maximum length of, for example, 100 nucleotides, m will typically be ≤ 98.

The number of nucleotides "G" in the nucleic acid of formula (V) is determined by l or n. l and n, independently of one another, are each an integer from 1 to 40, wherein when l or n = 1 G is a nucleotide comprising guanine or an analogue thereof, and when I or n > 1 at least 50% of the nucleotides comprise guanine, or an analogue thereof.

For example, without implying any limitation, when I or n = 4 Gl or Gn can be, for example, a GUGU, GGUU, UGUG, UUGG, GUUG, GGGU, GGUG, GUGG, UGGG or GGGG, etc.; when I or n = 5 Gl or Gn can be, for example, a GGGUU, GGUGU, GUGGU, UGGGU, UGGUG, UGUGG, UUGGG, GUGUG, GGGGU, GGGUG, GGUGG, GUGGG, UGGGG, or GGGGG, etc.; etc.

A nucleotide adjacent to Xₘ in the nucleic acid of formula (V) preferably does not comprise uracil.

Similarly, the number of nucleotides "C" in the nucleic acid of formula (VI) is determined by l or n. I and n, independently of one another, are each an integer from 1 to 40, wherein when l or n = 1 C is a nucleotide comprising cytosine or an analogue thereof, and when l or n > 1 at least 50% of the nucleotides comprise cytosine or an analogue thereof.

For example, without implying any limitation, when l or n = 4, Cl or Cn can be, for example, a CUCU, CCUU, UCUC, UUCC, CUUC, CCCU, CCUC, CUCC, UCCC or CCCC, etc.; when l or n = 5 Cl or Cn can be, for example, a CCCUU, CCUCU, CUCCU, UCCCU, UCCUC, UCUCC, UUCCC, CUCUC, CCCCU, CCCUC, CCUCC, CUCCC, UCCCC, or CCCCC, etc..

A nucleotide adjacent to Xₘ in the nucleic acid of formula (VI) preferably does not comprise uracil. Preferably, for formula (V), when l or n > 1, at least 60%, 70%, 80%, 90% or even 100% of the nucleotides comprise guanine or an analogue thereof, as defined above.

The remaining nucleotides to 100% (when nucleotides comprising guanine constitutes less than 100% of the nucleotides) in the flanking sequences G1 and/or Gn are uridine or an analogue thereof, as defined hereinbefore. Also preferably, l and n, independently of one another, are each an integer from 2 to 30, more preferably an integer from 2 to 20 and yet more preferably an integer from 2 to 15. The lower limit of l or n can be varied if necessary and is at least 1, preferably at least 2, more preferably at least 3, 4, 5, 6, 7, 8, 9 or 10. This definition applies correspondingly to formula (VI).

According to a further preferred embodiment, the isRNA as described herein consists of or comprises a nucleic acid of formula (VII) or (VIII):

(NᵤGₗXₘGₙNᵥ)ₐ formula (VII)

wherein:
- G: is a nucleotide comprising guanine, uracil or an analogue of guanine or uracil, preferably comprising guanine or an analogue thereof;
- X: is a nucleotide comprising guanine, uracil, adenine, thymine, cytosine, or an analogue thereof, preferably comprising uracil or an analogue thereof;
- N: is a nucleic acid sequence having a length of about 4 to 50, preferably of about 4 to 40, more preferably of about 4 to 30 or 4 to 20 nucleic acids, each N independently being selected from a nucleotide comprising guanine, uracil, adenine, thymine, cytosine or an analogue thereof;
- a: is an integer from 1 to 20, preferably from 1 to 15, most preferably from 1 to 10;
- l: is an integer from 1 to 40,
wherein when l = 1, G is a nucleotide comprising guanine or an analogue thereof,
when l > 1, at least 50% of these nucleotides comprise guanine or an analogue thereof;
- m: is an integer and is at least 3;
wherein when m = 3, X is a nucleotide comprising uracil or an analogue thereof, and
when m > 3, at least 3 successive nucleotides comprising uracils or analogues of uracils occur;
- n: is an integer from 1 to 40,
wherein when n = 1, G is a nucleotide comprising guanine or an analogue thereof,
when n > 1, at least 50% of these nucleotides comprise guanine or an analogue thereof;
- u,v: may be independently from each other an integer from 0 to 50,
preferably wherein when u = 0, v ≥ 1, or when v = 0, u ≥ 1;

wherein the nucleic acid molecule of formula (VII) has a length of at least 50 nucleotides, preferably of at least 100 nucleotides, more preferably of at least 150 nucleotides, even more preferably of at least 200 nucleotides and most preferably of at least 250 nucleotides.

   (NᵤCₗXₘCₙNᵥ)ₐ formula (VIII)
wherein:
   - C: is a nucleotide comprising cytosine, uracil or an analogue of cytosine or uracil, preferably cytosine or an analogue thereof;
   - X: is a nucleotide comprising guanine, uracil, adenine, thymine, cytosine or an analogue thereof, preferably comprising uracil or an analogue thereof;
   - N: is each a nucleic acid sequence having independent from each other a length of about 4 to 50, preferably of about 4 to 40, more preferably of about 4 to 30 or 4 to 20 nucleic acids, each N independently being selected from a nucleotide comprising guanine, uracil, adenine, thymine, cytosine or an analogue thereof;
   - a: is an integer from 1 to 20, preferably from 1 to 15, most preferably from 1 to 10;
   - l: is an integer from 1 to 40,
   wherein when l = 1, C is a nucleotide comprising cytosine or an analogue thereof,
   when l > 1, at least 50% of these nucleotides comprise cytosine or an analogue thereof;
   - m: is an integer and is at least 3;
   wherein when m = 3, X is a nucleotide comprising uracil or an analogue thereof,
   when m > 3, at least 3 successive nucleotides comprising uracils or analogues of uracil occur;
   - n: is an integer from 1 to 40,
   wherein when n = 1, C is a nucleotide comprising cytosine or an analogue thereof,
   when n > 1, at least 50% of these nucleotides comprise cytosine or an analogue thereof.
   - u, v: may be independently from each other an integer from 0 to 50,
   preferably wherein when u = 0, v ≥ 1, or when v = 0, u ≥ 1;
wherein the nucleic acid molecule of formula (VIII) according to the invention has a length of at least 50 nucleotides, preferably of at least 100 nucleotides, more preferably of at least 150 nucleotides, even more preferably of at least 200 nucleotides and most preferably of at least 250 nucleotides.

For formula (VIII), any of the definitions given above for elements N (i.e. Nᵤ and Nᵥ) and X (Xₘ), particularly the core structure as defined above, as well as for integers a, l, m, n, u and v, similarly apply to elements of formula (IV) correspondingly, wherein in formula (VIII) the core structure is defined by CₗXₘCₙ. The definition of bordering elements Nᵤ and Nᵥ is identical to the definitions given above for Nᵤ and Nᵥ.

In particular in the context of formulas (V)-(VIII) above, a "nucleotide" is understood as a molecule comprising or preferably consisting of a nitrogenous base (preferably selected from adenine (A), cytosine (C), guanine (G), thymine (T), or uracil (U), a pentose sugar (ribose or deoxyribose), and at least one phosphate group. "Nucleosides" consist of a nucleobase and a pentose sugar (i.e. could be referred to as "nucleotides without phosphate groups"). Thus, a "nucleotide" comprising a specific base (A, C, G, T or U) preferably also comprises the respective nucleoside (adenosine, cytidine, guanosine, thymidine or uridine, respectively) in addition to one (two, three or more) phosphate groups

That is, the term "nucleotides" includes nucleoside monophosphates (AMP, CMP, GMP, TMP and UMP), nucleoside diphosphates (ADP, CDP, GDP, TDP and UDP), nucleoside triphosphates (ATP, CTP, GTP, TTP and UTP). In the context of formulas (V)-(VIII) above, nucleoside monophosphates are particularly preferred. The expression "a nucleotide comprising (...) or an analogue thereof" refers to modified nucleotides comprising a modified (phosphate) backbone, pentose sugar(s), or nucleobases. In this context, modifications of the nucleobases are particularly preferred. By way of example, when referring "to a nucleotide comprising guanine, uracil, adenine, thymine, cytosine or an analogue thereof', the term "analogue thereof" refers to both the nucleotide and the recited nucleobases, preferably to the recited nucleobases.

### Further agents

In further preferred embodiments it is also possible that the inventive combination, (pharmaceutical) composition or vaccine contains, in addition to the at least one (i) epitope-encoding RNA, (ii) PD-1 pathway inhibitor and (iii) LAG-3 pathway inhibitor, further agents or components which are selected from the group comprising: further antigens (e.g. in the form of a peptide or protein) or further epitope-encoding nucleic acids; a further immunotherapeutic agent; one or more auxiliary substances; or any further compound, which is known to be immunostimulating due to its binding affinity (as ligands) to human Toll-like receptors; and/or an adjuvant nucleic acid, preferably an immunostimulatory RNA (isRNA).

### Auxiliary substances

The inventive combination, (pharmaceutical) composition or vaccine may thus additionally contain one or more auxiliary substances in order to increase its immunogenicity or immunostimulatory capacity, if desired. A synergistic action of the epitope-encoding RNA and the inhibitors as defined herein and of an auxiliary substance, which may be optionally contained in the inventive composition, is preferably achieved thereby.

In general, it is possible to use as auxiliary substance any agent that influences the immune system in the manner of a "danger signal" (LPS, GP96, etc.) or cytokines, such as GM-CFS, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

The inventive (pharmaceutical) composition or vaccine may also additionally contain any further compound, which is known to be immune-stimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Another class of compounds, which may be added to an inventive (pharmaceutical) composition or vaccine may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences, that is to say the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

Another class of compounds, which may be added to an inventive (pharmaceutical) composition or vaccine may thus be immunostimulatory RNAs (isRNAs), i.e. RNAs that are able to induce an innate immune response. Such immunostimulatory RNAs may optionally comprise or consist of a formula (I)-(IV) as depicted above. isRNAs usually do not comprise an open reading frame and thus do not provide an epitope or antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

### Pharmaceutically acceptable excipients and carriers

Preferably, the (pharmaceutical) composition or vaccine according to the invention comprises at least one pharmaceutically acceptable excipient, in particular at least one pharmaceutically acceptable carrier. The term "pharmaceutically acceptable" refers to a compound or agent that is compatible with the one or more active agent(s) (here: epitope-encoding RNA, PD-1 and/or LAG-3 pathway inhibitor) and does not interfere with and/or substantially reduce their pharmaceutical activities. Pharmaceutically acceptable carriers preferably have sufficiently high purity and sufficiently low toxicity to make them suitable for administration to a subject to be treated.

Pharmaceutically acceptable excipients can exhibit different functional roles and include, without limitation, diluents, fillers, bulking agents, carriers, disintegrants, binders, lubricants, glidants, coatings, solvents and co-solvents, buffering agents, preservatives, adjuvants, anti-oxidants, wetting agents, anti-foaming agents, thickening agents, sweetening agents, flavouring agents and humectants.

Suitable pharmaceutically acceptable carriers are typically chosen based on the formulation of the (pharmaceutical) composition or vaccine .

For (pharmaceutical) compositions or vaccines in liquid form, useful pharmaceutically acceptable excipients in general include solvents, diluents or carriers such as (pyrogen-free) water, (isotonic) saline solutions such phosphate or citrate buffered saline, fixed oils, vegetable oils, such as, for example, groundnut oil, cottonseed oil, sesame oil, olive oil, corn oil, ethanol, polyols (for example, glycerol, propylene glycol, polyetheylene glycol, and the like); lecithin; surfactants; preservatives such as benzyl alcohol, parabens, chlorobutanol, phenol, ascorbic acid, thimerosal, and the like; isotonic agents such as sugars, polyalcohols such as manitol, sorbitol, or sodium chloride; aluminum monostearate or gelatin; antioxidants such as ascorbic acid or sodium bisulfite; chelating agents such as ethylenediaminetetraacetic acid (EDTA); buffers such as acetates, citrates or phosphates and agents for the adjustment of tonicity such as sodium chloride or dextrose. pH can be adjusted with acids or bases, such as hydrochloric acid or sodium hydroxide. Buffers may be hypertonic, isotonic or hypotonic with reference to the specific reference medium, i.e. the buffer may have a higher, identical or lower salt content with reference to the specific reference medium, wherein preferably such concentrations of the aforementioned salts may be used, which do not lead to damage of cells due to osmosis or other concentration effects. Reference media are e.g. liquids occurring in "in vivo" methods, such as blood, lymph, cytosolic liquids, or other body liquids, or e.g. liquids, which may be used as reference media in "in vitro" methods, such as common buffers or liquids. Such common buffers or liquids are known to a skilled person. Ringer-Lactate solution is particularly preferred as a liquid basis.

### Carriers

Suitable pharmaceutically acceptable carriers are typically chosen based on the formulation of the (pharmaceutical) composition or vaccine.

Liquid (pharmaceutical) compositions or vaccines administered via injection and in particular via i.v. injection should be sterile and stable under the conditions of manufacture and storage. Such compositions are typically formulated as parenterally acceptable aqueous solutions that are pyrogen-free, have suitable pH, are isotonic and maintain stability of the active ingredient(s).

Particularly useful pharmaceutically acceptable carriers for liquid (pharmaceutical) compositions or vaccines according to the invention include water, typically pyrogen-free water; isotonic saline or buffered (aqueous) solutions, e.g phosphate, citrate etc. buffered solutions. Particularly for injection of the inventive (pharmaceutical) compositions or vaccines, water or preferably a buffer, more preferably an aqueous buffer, may be used, containing a sodium salt, preferably at least 50 mM of a sodium salt, a calcium salt, preferably at least 0,01 mM of a calcium salt, and optionally a potassium salt, preferably at least 3 mM of a potassium salt.

According to preferred embodiments, the sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCI, KI, KBr, K₂CO₃, KHCO₃, K₂SO₄, and examples of calcium salts include e.g. CaCl₂, Cal₂, CaBr₂, CaCO₃, CaSO₄, Ca(OH)₂. Furthermore, organic anions of the aforementioned cations may be contained in the buffer.

According to more preferred embodiments, the buffer suitable for injection purposes as defined above, may contain salts selected from sodium chloride (NaCl), calcium chloride (CaCl₂) and optionally potassium chloride (KCI), wherein further anions may be present additional to the chlorides. CaCl₂ can also be replaced by another salt like KCI. Typically, the salts in the injection buffer are present in a concentration of at least 50 mM sodium chloride (NaCl), at least 3 mM potassium chloride (KCl) and at least 0,01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. in *"in vivo"* methods occurring liquids such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

For (pharmaceutical) compositions in (semi-)solid form, useful pharmaceutically acceptable excipients include binders such as microcrystalline cellulose, gum tragacanth or gelatin; starch or lactose; sugars, such as, for example, lactose, glucose and sucrose; starches, such as, for example, corn starch or potato starch; cellulose and its derivatives, such as, for example, sodium carboxymethylcellulose, ethylcellulose, cellulose acetate; disintegrants such as alginic acid; lubricants such as magnesium stearate; glidants such as stearic acid, magnesium stearate; calcium sulphate, colloidal silicon dioxide and the like; sweetening agents such as sucrose or saccharin; and/or flavoring agents such as peppermint, methyl salicylate, or orange flavoring.

### Formulation

(Pharmaceutical) compositions for topical administration can be formulated as creams, ointments, gels, pastes or powders. (Pharmaceutical) compositions for oral administration can be formulated as tablets, capsules, liquids, powders or in a sustained release format.

According to preferred embodiments, the inventive (pharmaceutical) composition or vaccine is administered parenterally, in particular via intradermal or intramuscular injection. Accordingly, (pharmaceutical) compositions or vaccines of the invention are preferably formulated for for parenteral administration (in particular injection) and are thus typically provided in liquid form (e.g. lipid based or saline based) or lyophilized form. Parenteral formulations are typically stored in vials, IV bags, ampoules, cartridges, or prefilled syringes and can be administered as injections, inhalants, or aerosols, with injections being preferred. Preferred parenteral formulations for injection include sterile solutions of water, physiological saline or mixtures thereof, with a physiological pH of about 7.4

### Lyophilized formulations

In preferred embodiments, the (pharmaceutical) composition or vaccine is provided in lyophilized form. Preferably, the lyophilized (pharmaceutical) composition or vaccine is reconstituted in a suitable buffer, advantageously based on an aqueous carrier, prior to administration, e.g. Ringer-Lactate solution, which is preferred, Ringer solution, a phosphate buffer solution. In some embodiments, the (pharmaceutical) composition or vaccine according to the invention contains at least two, three, four, five, six or more epitope-encoding RNAs, preferably mRNAs which are provided separately in lyophilized form (optionally together with at least one further additive) and which are preferably reconstituted separately in a suitable buffer (such as Ringer-Lactate solution) prior to their use so as to allow individual administration of each of said epitope-encoding RNAs.

### Liquid formulations

In further preferred embodiments, the (pharmaceutical) composition or vaccine is provided in the form of a saline or a lipid-based formulation. Lipid-based formulations may be selected from, but not limited to, liposomes, lipoplexes, nanoliposomes and lipid nanoparticles which are described above in the section headed "Complexation".

### Dose

The (pharmaceutical) composition or vaccine typically comprises a safe and effective amount of the epitope-encoding RNA, PD-1 and LAG-3 pathway inhibitor.

As used herein, "safe and effective amount" means an amount of the active agent(s) that is sufficient to significantly induce a positive modification of the disease to be treated. At the same time, however, a "safe and effective amount" is small enough to avoid serious side-effects, that is to say to permit a sensible relationship between advantage and risk.

In the context of the present invention, the expression "safe and effective amount" preferably means an amount of the active agent(s) that is suitable eliciting the desired therapeutic effect, e.g. in case of the inventive (pharmaceutical) composition or vaccine, stimulating the adaptive immune system in such a manner that no excessive or damaging immune reactions are achieved but, preferably, also no such immune reactions below a measurable level.

A "safe and effective amount" will furthermore vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the accompanying doctor.

Specifically, a "safe and effective amount" of the epitope-encoding RNA may furthermore be selected depending on the type of epitope-encoding RNA, e.g. monocistronic, bi- or even multicistronic RNA, since a bi- or even multicistronic RNA may lead to a significantly higher expression of the encoded antigen(s) than the use of an equal amount of a monocistronic RNA.

### Kit

In a further aspect, the present invention relates to a kit or kit-of-parts comprising the inventive combination, (pharmaceutical) composition or vaccine. In other words, the kit-of-parts typically comprises (i) at least one epitope-encoding RNA as defined herein, (ii) at least one PD-1 pathway inhibitor as defined herein and (iii) at least one LAG-3 pathway inhibitor as defined herein as its components.

The aforementioned components may each be provided in the form of a pharmaceutical composition in the kit-of-parts. Insofar, the definitions and explanations provided above for the (pharmaceutical) composition or vaccine are equally applicable to the individual components of the kit-of-parts, *mutatis mutandis.*

For instance, the (i) at least one epitope-encoding RNA, (ii) PD-1 pathway inhibitor and (iii) LAG-3 pathway inhibitor may be provided -independently from each other- in lyophilized or liquid form, optionally together with one or more pharmaceutically acceptable carrier(s), excipients, adjuvants or further agents as described above in the context of the pharmaceutical composition.

Optionally, the kit-of-parts may comprise at least one further agent as defined herein in the context of the pharmaceutical composition, antimicrobial agents, RNAse inhibitors, solubilizing agents or the like.

The kit-of-parts may be a kit of two or more parts and typically comprises the components in suitable containers. For example, each container may be in the form of vials, bottles, squeeze bottles, jars, sealed sleeves, envelopes or pouches, tubes or blister packages or any other suitable form provided the container is configured so as to prevent premature mixing of components. Each of the different components may be provided separately, or some of the different components may be provided together (i.e. in the same container).

A container may also be a compartment or a chamber within a vial, a tube, a jar, or an envelope, or a sleeve, or a blister package or a bottle, provided that the contents of one compartment are not able to associate physically with the contents of another compartment prior to their deliberate mixing by a pharmacist or physician.

The kit-of-parts may furthermore contain technical instructions with information on the administration and dosage of any of its components.

### Medical use and treatment

The inventive combination, the (pharmaceutical composition), vaccine or kit-of-parts defined herein may be used for human and also for veterinary medical purposes, preferably for human medical purposes.

According to a further aspect, the invention thus relates to the inventive combination, (pharmaceutical composition), vaccine or kit-of-parts for use as a medicament. Accordingly, the present invention encompasses a PD-1 pathway inhibitor and/or a LAG-3 pathway inhibitor as defined herein for use in therapy in combination with an epitope-encoding RNA as defined herein. Further, the invention features an epitope-encoding RNA as defined herein for use in therapy in combination with a PD-1 pathway inhibitor and a LAG-3 pathway inhibitor as defined herein.

The inventive combination, (pharmaceutical) composition, vaccine or kit-of-parts are *inter alia* useful for treatment and/or prophylaxis of diseases which would benefit from stimulation or restoration of T cell function and T cell mediated immune responses (e.g. proliferation, cytokine release, target cell killing, effector cell activation) in a subject in need thereof.

According to a further aspect, the invention thus relates to the inventive combination, (pharmaceutical) composition, vaccine or kit-of-parts for use in a method of prophylaxis or treatment of a tumor or cancer disease, an infectious disease, an allergy or an autoimmune disease.

The term "treatment" or "treating" of a disease includes preventing or protecting against the disease (that is, causing the clinical symptoms not to develop); inhibiting the disease (i.e., arresting or suppressing the development of clinical symptoms; and/or relieving the disease (i.e., causing the regression of clinical symptoms). As will be appreciated, it is not always possible to distinguish between "preventing" and "suppressing" a disease or disorder since the ultimate inductive event or events may be unknown or latent. Accordingly, the term "prophylaxis" will be understood to constitute a type of "treatment" that encompasses both "preventing" and "suppressing." The term "treatment" thus includes "prophylaxis".

The term "subject", "patient" or "individual" as used herein generally includes humans and non-human animals and preferably mammals (e.g., non-human primates, including marmosets, tamarins, spider monkeys, owl monkeys, vervet monkeys, squirrel monkeys, and baboons, macaques, chimpanzees, orangutans, gorillas; cows; horses; sheep; pigs; chicken; cats; dogs; mice; rat; rabbits; guinea pigs; etc.), including chimeric and transgenic animals and disease models. In the context of the present invention, the term "subject" preferably refers a non-human primate or a human, most preferably a human.

Accordingly, the present invention further provides methods of treating or preventing cancer, infectious diseases, autoimmune diseases or allergies, by administering to a subject in need thereof a pharmaceutically effective amount of the inventive combination, (pharmaceutical) composition, vaccine or kit-of-parts. Such methods may comprise an optional first step of preparing the inventive combination, (pharmaceutical) composition, vaccine, or kit-of-parts, and a second step, comprising administering (a pharmaceutically and/or therapeutically effective amount of) said combination, (pharmaceutical) composition, vaccine, or kit-of-parts to a patient/subject in need thereof.

The invention also relates to the use of the inventive combination, (pharmaceutical) composition, vaccine or kit-of-parts according to the invention, preferably for modulating, preferably for inducing or enhancing, an immune response in a subject, more preferably for the treatment or prophylaxis of cancer, an infectious disease, an allergy or an autoimmune disease as defined herein.

### Administration

The inventive combination, (pharmaceutical) composition, vaccine or kit-of-parts (or their components) can be administered, for example, systemically or locally.

Routes for systemic administration in general include, for example, transdermal, oral, parenteral routes, including subcutaneous, intravenous, intramuscular, intraarterial, intradermal and intraperitoneal injections and/or intranasal administration routes.

Routes for local administration in general include, for example, topical administration routes but also intradermal, transdermal, subcutaneous, or intramuscular injections or intralesional, intratumoral, intracranial, intrapulmonal, intracardial, and sublingual injections.

It is further conceivable to use different administration routes for different components of the inventive (pharmaceutical) composition, vaccine or kit-of-parts, for instance in case said (pharmaceutical) composition, vaccine or kit-of-parts comprises several epitope-encoding RNA species.

According to preferred embodiments, the inventive combination, (pharmaceutical) composition, vaccine or kit-of-parts (or their components) is/are administered by a parenteral route, preferably via intradermal, subcutaneous, or intramuscular routes. Preferably, said combination, (pharmaceutical) composition, vaccine or kit-of-parts is administered by injection, e.g. subcutaneous, intramuscular or intradermal injection, which may be needle-free and/or needle injection. Accordingly, in preferred embodiments, the medical use and/or method of treatment according to the present invention involves administration of said combination, (pharmaceutical) composition, vaccine or kit-of-parts (or their components) by subcutaneous, intramuscular or intradermal injection, preferably by intramuscular or intradermal injection, more preferably by intradermal injection. Such injection may be carried out by using conventional needle injection or jet injection, preferably by using jet injection.

### Administration regimen

The inventive combination, (pharmaceutical) composition, vaccine or kit-of-parts and the respective components (i.e. epitope-encoding RNA, PD-1 pathway inhibitor and LAG-3 pathway inhibitor) may be administered to a subject in need thereof several times a day, daily, every other day, weekly, or monthly.

The components may be administered simultaneously (i.e. at the same time via the same or different administrations routes) or separately (i.e. sequentially at different time points and/or via different administrations routes). A sequential administration scheme is also referred to as "time-staggered" administration. A time-staggered administration of the several components of the inventive combination, (pharmaceutical) composition, vaccine or kit-of-parts may ensure that the separate mechanisms elicited by said components do not negatively influence each other. Time-staggered administration may for instance mean that one epitope-encoding RNA species is administrated e.g. prior, concurrent or subsequent to the PD-1 and/or LAG-3 pathway inhibitors, and/or prior, concurrent or subsequent to different epitope-encoding RNA species. This procedure preferably allows immune cells such as antigen-presenting cells and T cells to encounter the RNA-encoded epitope, before the immune system is stimulated by inhibition of the PD-1 and LAG-3 pathways, even though a concurrent administration or an administration, wherein the PD-1 pathway inhibitor and/or LAG-3 pathway inhibitor is administered prior to the epitope-encoding RNA, may lead to the same or at least comparable results.

According to some preferred embodiments, the components of the inventive combination, (pharmaceutical) composition, vaccine or kit-of-parts (i.e. at least one epitope-encoding RNA, PD-1 pathway inhibitor and LAG-3 pathway inhibitor) are administered simultaneously (i.e. at the same time via the same or different administrations routes).

According to some preferred embodiments, the components of the inventive combination, (pharmaceutical) composition, vaccine or kit-of-parts (i.e. at least one epitope-encoding RNA, PD-1 pathway inhibitor and LAG-3 pathway inhibitor) are administered separately (i.e. sequentially at different time points and/or via different administrations routes).

### Dosage

The inventive combination, (pharmaceutical) composition, vaccine or kit-of-parts and the respective components are preferably administered to the subject in need thereof in a "pharmaceutically effective" amount.

A "pharmaceutically effective amount" in the context of the invention is typically understood as an amount that is sufficient to induce a desired pharmaceutical effect, such as an immune response. Preferably, the administered amount of the inventive combination, (pharmaceutical) composition, vaccine or kit-of-parts is also "therapeutically effective", i.e. sufficient for the alleviation of the symptoms of the disease or condition being treated and/or for prophylaxis of the symptoms of the disease or condition being prevented. In other words, a "therapeutically effective amount" means an amount of the inventive combination, (pharmaceutical) composition, vaccine or kit-of-parts that is sufficient to significantly induce a positive modification of a disease or disorder, i.e. an amount of the active ingredient that elicits the biological or medicinal response in a tissue, system, animal or human that is being sought.

Therapeutic efficacy and toxicity of inventive combination, (pharmaceutical) composition, vaccine or kit-of-parts or the respective components can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and can be expressed as the ratio LD50/ED50. Combinations, (pharmaceutical) composition, vaccine or kit-of-parts which exhibit large therapeutic indices are generally preferred. The data obtained from the cell culture assays and animal studies can be used in formulating a range of dosage for use in humans. The dosage of such compounds lies preferably within a range of circulating concentrations that include the ED50 with little or no toxicity.

The term also includes an amount of the inventive combination, (pharmaceutical) composition, vaccine or kit-of-parts sufficient to reduce the progression of the disease, for instance to reduce or inhibit tumor growth. At the same time, however, a "therapeutically effective amount" is preferably small enough to avoid serious side-effects, i.e. to permit a sensible relationship between advantage and risk. The determination of these limits typically lies within the scope of sensible medical judgment. A "therapeutically effective amount" of the inventive combination, (pharmaceutical) composition, vaccine or kit-of-parts will furthermore vary in connection with the particular disease or condition to be treated, characteristics of the patient (including age, physical condition, body weight, sex and diet), concurrent treatments, pharmacokinetic properties of the active agent(s), treatment regime and the desired effect (amelioration vs. complete remission), etc.

For instance, therapeutically effective doses of the inventive combination, (pharmaceutical) composition, vaccine or kit-of-parts or the respective components described herein may range from about 0.001 mg to 10 mg, preferably from about 0.01 mg to 5 mg, more preferably from about 0.1 mg to 2 mg per dosage unit or from about 0.01 nmol to 1 mmol per dosage unit, in particular from 1 nmol to 1 mmol per dosage unit, preferably from 1 µmol to 1 mmol per dosage unit. It is also envisaged that the therapeutically effective dose of the inventive combination, (pharmaceutical) composition, vaccine or kit-of-parts (or their components) may range (per kg body weight) from about 0.01 mg/kg to 10 g/kg, preferably from about 0.05 mg/kg to 5 g/kg, more preferably from about 0.1 mg/kg to 2.5 g/kg.

The "pharmaceutically effective amount" of the inventive combination, (pharmaceutical) composition, vaccine or kit-of-parts according to the invention or their components can be determined by routine experiments, e.g. by using animal models. Such models include, without implying any limitation, rabbit, sheep, mouse, rat, dog and non-human primate models.

### Combination therapy

The inventive combination, (pharmaceutical) composition, vaccine or kit-of-parts may also be used in combination therapy. Any other therapy useful for treating or preventing the diseases and disorders defined herein may be combined with the uses and methods disclosed herein.

For instance, the subject receiving the inventive combination, (pharmaceutical) composition or vaccine according to the invention may be a patient with cancer, preferably as defined herein, or a related condition, receiving chemotherapy (e.g. first-line or second-line chemotherapy), radiotherapy, chemoradiation (combination of chemotherapy and radiotherapy), tyrosine kinase inhibitors (e.g. EGFR tyrosine kinase inhibitors), antibody therapy and/or inhibitory and/or stimulatory checkpoint molecules (e.g. CTLA4 inhibitors), or a patient, who has achieved partial response or stable disease after having received one or more of the treatments specified above. Or, the subject receiving the inventive combination, (pharmaceutical) composition or vaccine according to the invention may be a patient with an infectious disease, preferably as defined herein, receiving antibiotic, antifungal or antiviral therapy.

In a further aspect, the present invention thus also relates to the use of the inventive combination, (pharmaceutical) composition or vaccine for supporting another therapy of cancer, an infectious disease, an allergy or an autoimmune disease.

"Support" of the treatment or prophylaxis of cancer may be any combination of a conventional cancer therapy method of such as surgery, radiation therapy, chemotherapy (e.g. first-line or second-line chemotherapy), chemoradiation, treatment with tyrosine kinase inhibitors, treatment with inhibitory and/or stimulatory checkpoint molecules, preferably CTLA4 inhibitors, antibody therapy or any combination of these, and a therapy using the inventive combination, (pharmaceutical) composition or vaccine as defined herein.

Administration of the inventive combination, (pharmaceutical) composition or vaccine according to the invention may be accomplished prior to, simultaneously and/or subsequently to administering another therapeutic or subjecting the patient to another therapy that is useful for treatment of the particular disease or condition to be treated.

### Cancer

In preferred embodiments, the inventive combination, (pharmaceutical) composition or vaccine is used for treatment or prophylaxis of cancer.

As used herein, the term "cancer" refers to a neoplasm characterized by the uncontrolled and usually rapid proliferation of cells that tend to invade surrounding tissue and to metastasize to distant body sites. The term encompasses benign and malignant neoplasms. Malignancy in cancers is typically characterized by anaplasia, invasiveness, and metastasis; whereas benign malignancies typically have none of those properties. The terms includes neoplasms characterized by tumor growth as well as cancers of blood and lymphatic system. Specific examples of cancer that can be treated with the inventive combination, (pharmaceutical) composition or vaccine include Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing"s Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin"s Lymphoma, Adult; Hodgkin"s Lymphoma, Childhood; Hodgkin"s Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi"s Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS- Related; Lymphoma, Central Nervous System (Primary); Lymphoma, Cutaneous T-Cell; Lymphoma, Hodgkin"s, Adult; Lymphoma, Hodgkin"s; Childhood; Lymphoma, Hodgkin"s During Pregnancy; Lymphoma, Non-Hodgkin"s, Adult; Lymphoma, Non-Hodgkin"s, Childhood; Lymphoma, Non-Hodgkin"s During Pregnancy; Lymphoma, Primary Central Nervous System; Macroglobulinemia, Waldenstrom"s; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplasia Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin"s Lymphoma, Adult; Non- Hodgkin"s Lymphoma, Childhood; Non-Hodgkin"s Lymphoma During Pregnancy; Non- Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood", Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin"s Lymphoma; Pregnancy and Non-Hodgkin"s Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland"Cancer, Childhood; Sarcoma, Ewing"s Family of Tumors; Sarcoma, Kaposi"s; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom"s Macro globulinemia; and Wilms" Tumor.

### Infectious diseases

The inventive combination, pharmaceutical composition or kit may also be used for treating infectious diseases. The term "infection" or "infectious disease" relates to the invasion and multiplication of microorganisms such as bacteria, viruses, and parasites that are not normally present within the body. An infection may cause no symptoms and be subclinical, or it may cause symptoms and be clinically apparent. An infection may remain localized, or it may spread through the blood or lymphatic system to become systemic. Infectious diseases in this context, preferably include viral, bacterial, fungal or protozoological infectious diseases. Specific examples of infectious diseases that can be treated with the inventive combination, (pharmaceutical) composition or vaccine include Acinetobacter infections, African sleeping sickness (African trypanosomiasis), AIDS (Acquired immunodeficiency syndrome), Amoebiasis, Anaplasmosis, Anthrax, Appendicitis, Arcanobacterium haemolyticum infections, Argentine hemorrhagic fever, Ascariasis, Aspergillosis, Astrovirus infections, Athlete"s foot, Babesiosis, Bacillus cereus infections, Bacterial meningitis, Bacterial pneumonia, Bacterial vaginosis (BV), Bacteroides infections, Balantidiasis, Baylisascaris infections, Bilharziosis, BK virus infections, Black piedra, Blastocystis hominis infections, Blastomycosis, Bolivian hemorrhagic fever, Borrelia infectionss (Borreliosis), Botulism (and Infant botulism), Bovine tapeworm, Brazilian hemorrhagic fever, Brucellosis, Burkholderia infections, Buruli ulcer, Calicivirus infections (Norovirus and Sapovirus), Campylobacteriosis, Candidiasis (Candidosis), Canine tapeworm infections, Cat-scratch disease, Chagas Disease (American trypanosomiasis), Chancroid, Chickenpox, Chlamydia infections, Chlamydia trachomatis infections, Chlamydophila pneumoniae infections, Cholera, Chromoblastomycosis, Climatic bubo, Clonorchiasis, Clostridium difficile infections, Coccidioidomycosis, Cold, Colorado tick fever (CTF), Common cold (Acute viral rhinopharyngitis; Acute coryza), Condyloma acuminata, Conjunctivitis, Creutzfeldt-Jakob disease (CJD), Crimean-Congo hemorrhagic fever (CCHF), Cryptococcosis, Cryptosporidiosis, Cutaneous larva migrans (CLM), Cutaneous Leishmaniosis, Cyclosporiasis, Cysticercosis, Cytomegalovirus infections, Dengue fever, Dermatophytosis, Dientamoebiasis, Diphtheria, Diphyllobothriasis, Donavanosis, Dracunculiasis, Early summer meningoencephalitis (FSME), Ebola hemorrhagic fever, Echinococcosis, Ehrlichiosis, Enterobiasis (Pinworm infections), Enterococcus infections, Enterovirus infections, Epidemic typhus, Epiglottitis, Epstein-Barr Virus Infectious Mononucleosis, Erythema infectiosum (Fifth disease), Exanthem subitum, Fasciolopsiasis, Fasciolosis, Fatal familial insomnia (FFI), Fifth disease, Filariasis, Fish poisoning (Ciguatera), Fish tapeworm, Flu, Food poisoning by Clostridium perfringens, Fox tapeworm, Free-living amebic infections, Fusobacterium infections, Gas gangrene, Geotrichosis, Gerstmann-Straussler-Scheinker syndrome (GSS), Giardiasis, Glanders, Gnathostomiasis, Gonorrhea, Granuloma inguinale (Donovanosis), Group A streptococcal infections, Group B streptococcal infections, Haemophilus influenzae infections, Hand foot and mouth disease (HFMD), Hantavirus Pulmonary Syndrome (HPS), Helicobacter pylori infections, Hemolytic-uremic syndrome (HUS), Hemorrhagic fever with renal syndrome (HFRS), Henipavirus infections, Hepatitis A, Hepatitis B, Hepatitis C, Hepatitis D, Hepatitis E, Herpes simplex, Herpes simplex type I, Herpes simplex type II, Herpes zoster, Histoplasmosis, Hollow warts, Hookworm infections, Human bocavirus infections, Human ewingii ehrlichiosis, Human granulocytic anaplasmosis (HGA), Human metapneumovirus infections, Human monocytic ehrlichiosis, Human papillomavirus (HPV) infections, Human parainfluenza virus infections, Hymenolepiasis, Influenza, Isosporiasis, Japanese encephalitis, Kawasaki disease, Keratitis, Kingella kingae infections, Kuru, Lambliasis (Giardiasis), Lassa fever, Legionellosis (Legionnaires" disease, Pontiac fever), Leishmaniasis, Leprosy, Leptospirosis, Lice, Listeriosis, Lyme borreliosis, Lyme disease, Lymphatic filariasis (Elephantiasis), Lymphocytic choriomeningitis, Malaria, Marburg hemorrhagic fever (MHF), Marburg virus, Measles, Melioidosis (Whitmore"s disease), Meningitis, Meningococcal disease, Metagonimiasis, Microsporidiosis, Miniature tapeworm, Miscarriage (prostate inflammation), Molluscum contagiosum (MC), Mononucleosis, Mumps, Murine typhus (Endemic typhus), Mycetoma, Mycoplasma hominis, Mycoplasma pneumonia, Myiasis, Nappy/diaper dermatitis, Neonatal conjunctivitis (Ophthalmia neonatorum), Neonatal sepsis (Chorioamnionitis), Nocardiosis, Noma, Norwalk virus infections, Onchocerciasis (River blindness), Osteomyelitis, Otitis media, Paracoccidioidomycosis (South American blastomycosis), Paragonimiasis, Paratyphus, Pasteurellosis, Pediculosis capitis (Head lice), Pediculosis corporis (Body lice), Pediculosis pubis (Pubic lice, Crab lice), Pelvic inflammatory disease (PID), Pertussis (Whooping cough), Pfeiffer"s glandular fever, Plague, Pneumococcal infections, Pneumocystis pneumonia (PCP), Pneumonia, Polio (childhood lameness), Poliomyelitis, Porcine tapeworm, Prevotella infections, Primary amoebic meningoencephalitis (PAM), Progressive multifocal leukoencephalopathy, Pseudo-croup, Psittacosis, Q fever, Rabbit fever, Rabies, Rat-bite fever, Reiter's syndrome, Respiratory syncytial virus infections (RSV), Rhinosporidiosis, Rhinovirus infections, Rickettsial infections, Rickettsialpox, Rift Valley fever (RVF), Rocky mountain spotted fever (RMSF), Rotavirus infections, Rubella, Salmonella paratyphus, Salmonella typhus, Salmonellosis, SARS (Severe Acute Respiratory Syndrome), Scabies, Scarlet fever, Schistosomiasis (Bilharziosis), Scrub typhus, Sepsis, Shigellosis (Bacillary dysentery), Shingles, Smallpox (Variola), Soft chancre, Sporotrichosis, Staphylococcal food poisoning, Staphylococcal infections, Strongyloidiasis, Syphilis, Taeniasis, Tetanus, Three-day fever, Tick-borne encephalitis, Tinea barbae (Barber"s itch), Tinea capitis (Ringworm of the Scalp), Tinea corporis (Ringworm of the Body), Tinea cruris (Jock itch), Tinea manuum (Ringworm of the Hand), Tinea nigra, Tinea pedis (Athlete"s foot), Tinea unguium (Onychomycosis), Tinea versicolor (Pityriasis versicolor), Toxocariasis (Ocular Larva Migrans (OLM) and Visceral Larva Migrans (VLM)), Toxoplasmosis, Trichinellosis, Trichomoniasis, Trichuriasis (Whipworm infections), Tripper, Trypanosomiasis (sleeping sickness), Tsutsugamushi disease, Tuberculosis, Tularemia, Typhus, Typhus fever, Ureaplasma urealyticum infections, Vaginitis (Colpitis), Variant Creutzfeldt-Jakob disease (vCJD, nvCJD), Venezuelan equine encephalitis, Venezuelan hemorrhagic fever, Viral pneumonia, Visceral Leishmaniosis, Warts, West Nile Fever, Western equine encephalitis, White piedra (Tinea blanca), Whooping cough, Yeast fungus spots, Yellow fever, Yersinia pseudotuberculosis infections, Yersiniosis, and Zygomycosis.

Use of the inventive combination, (pharmaceutical) composition or vaccine for treatment and/or prophylaxis of the diseases or conditions described herein may include administration of a pharmaceutically effective amount of said combination, (pharmaceutical) composition or vaccine to a subject in need thereof. Methods of treating diseases described herein may include administration of a pharmaceutically effective amount of said combination, (pharmaceutical) composition or vaccine to a subject in need thereof. Suitable ways of administration are described above.

### DESCRIPTION OF THE FIGURES

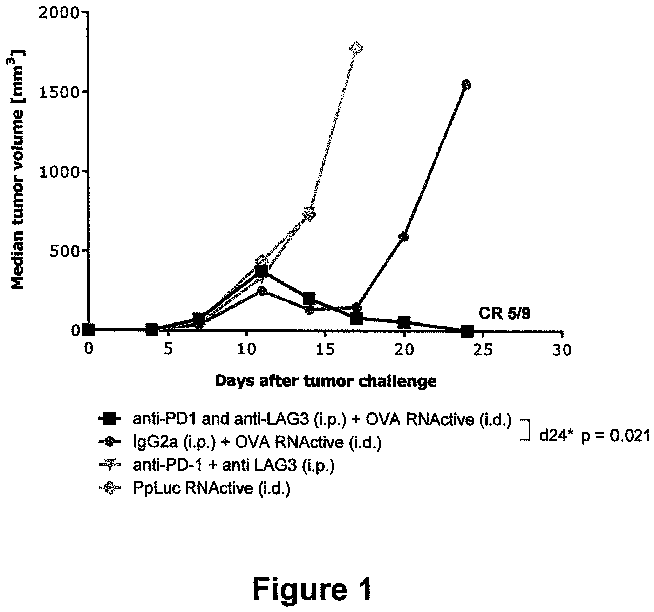

**Figure 1** **: RNA vaccination in combination with anti-PD-1 and anti-LAG-3 treatment decreases tumor growth in an E.G7-OVA tumor model.** C57BL/6 mice (n ≥ 9 per group) were challenged subcutaneously on the right flank with 3 x 10⁵ syngeneic E.G7-OVA lymphoma cells (ovalbumin expressing EL4 lymphoma cell line). Four days after tumor cell inoculation mice were vaccinated intradermally with OVA-encoding RNA ("RNActive") and treated intraperitoneally with 200 µg anti-PD-1 (BioXcell) and 200 µg anti-LAG-3 antibody (BioXcell) twice a week for three to four weeks. Mice treated with unspecific RNA vaccination served as controls. Median tumor volume of each group is depicted until single mice had to be excluded from the experiment due to meeting ethical endpoint of the study. * unpaired two-tailed Mann-Whitney test

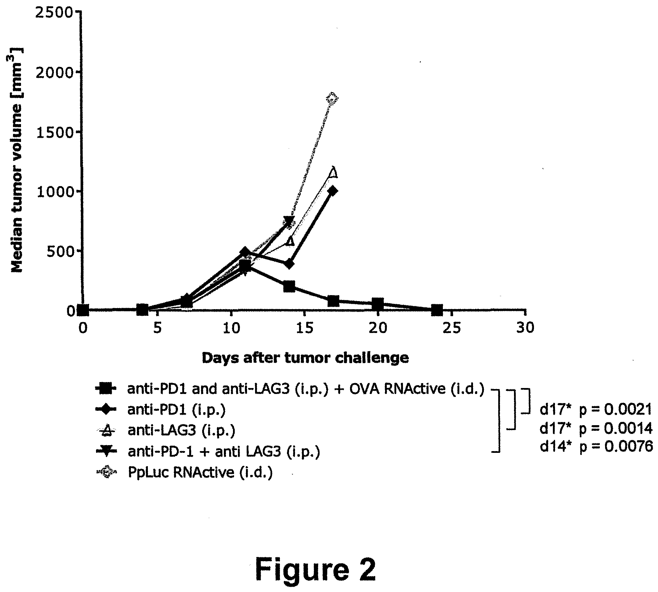

**Figure 2** **PD-1 and LAG-3 blockade alone reduce tumor growth significantly less efficient as the combination of PD-1 and LAG-3 inhibition with RNA vaccine in an E.G7-OVA tumor model.** C57BL/6 mice (n ≥ 9 per group) were challenged subcutaneously on the right flank with 3 x 10⁵ syngenic E.G7-OVA lymphoma cells (ovalbumin expressing EL4 lymphoma cell line). Four days after tumor cell inoculation mice were vaccinated intradermally with OVA-encoding RNA (RNActive) and treated intraperitoneally with 200 µg anti-PD-1 (BioXcell) and 200 µg anti-LAG-3 antibody (BioXcell) twice a week for three to four weeks. Mice treated only with anti-PD1, anti-LAG-3 or a combination of both and mice treated with unspecific RNA vaccination served as controls. Median tumor volume of each group is depicted until single mice had to be excluded from the experiment due to meeting ethical endpoint of the study. * unpaired two-tailed Mann-Whitney test.

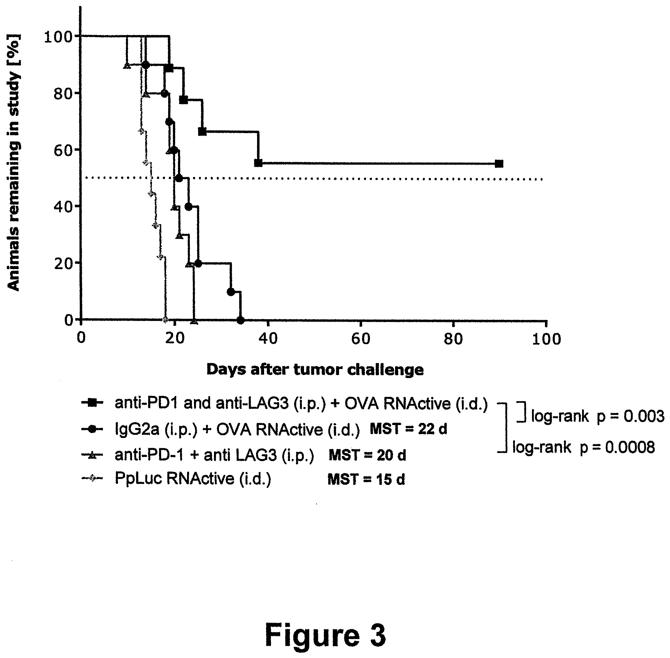

**Figure 3** **RNA vaccination in combination with anti-PD-1 and anti-LAG-3 treatment increases survival in an E.G7-OVA tumor model.** C57BL/6 mice (n ≥ 9 per group) were challenged subcutaneously on the right flank with 3 x 10⁵ syngenic E.G7-OVA lymphoma cells (ovalbumin expressing EL4 lymphoma cell line). Four days after tumor cell inoculation mice were vaccinated intradermally with OVA-encoding RNA (RNActive) and treated intraperitoneally with 200 µg anti-PD-1 (BioXcell) and 200 µg anti-LAG-3 antibody (BioXcell) twice a week for three to four weeks. Mice treated with unspecific RNActive vaccination served as controls. Kaplan-Meier plot of mice remaining in study until exclusion due to meeting ethical endpoint is presented. * log-rank comparison of survival curves.

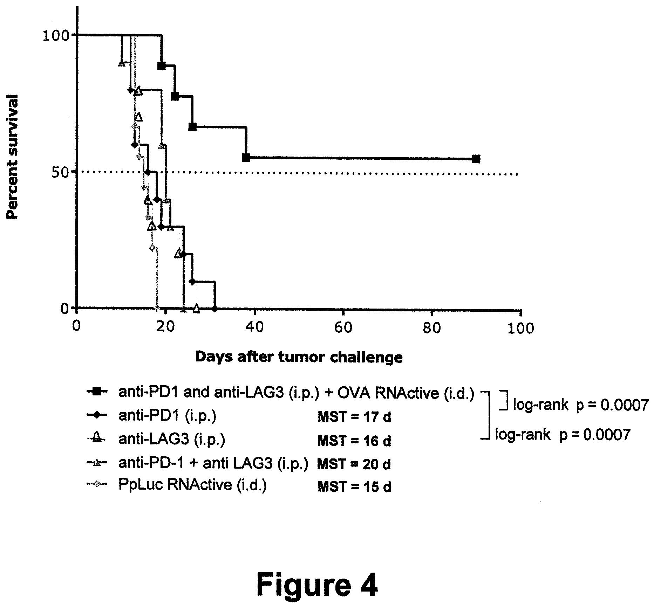

**Figure 4** **PD-1 and LAG-3 immune checkpoint inhibition had no significant effect on overall survival in an E.G7-OVA tumor model.** C57BL/6 mice (n ≥ 9 per group) were challenged subcutaneously on the right flank with 3 x 105 syngenic E.G7-OVA lymphoma cells (ovalbumin expressing EL4 lymphoma cell line). Four days after tumor cell inoculation mice were vaccinated intradermally with OVA-encoding RNA (RNActive) and treated intraperitoneally with 200 µg anti-PD-1 (BioXcell) and 200 µg anti-LAG-3 antibody (BioXcell) twice a week for three to four weeks. Mice treated only with anti-PD1, anti-LAG-3 or a combination of both and mice treated with unspecific RNA vaccination served as controls. Kaplan-Meier plot of mice remaining in study until exclusion due to meeting ethical endpoint is presented. * log-rank comparison of survival curves.

### List of Items:

Item 1: A combination comprising:
(i) at least one RNA, said RNA comprising at least one coding sequence encoding at least one epitope of an antigen; and
(ii) at least one PD-1 pathway inhibitor; and
(iii) at least one LAG-3 pathway inhibitor.

Item 2: The combination according to any one of the preceding items, wherein said PD-1 pathway inhibitor and/or said LAG-3 pathway inhibitor are selected from an antibody or a nucleic acid encoding said antibody, a protein or a nucleic acid encoding said protein, a peptide or a nucleic acid encoding said peptide, an antagonistic nucleic acid, and a small organic molecule.

Item 3: The combination according to any one of the preceding items, wherein
(a) said PD-1 pathway inhibitor is a competitive or non-competitive PD-1 antagonist; and/or
(b) said LAG-3 pathway inhibitor is a competitive or non-competitive LAG-3 antagonist.

Item 4: The combination according to any one of the preceding items, wherein
(a) said PD-1 pathway inhibitor binds to PD-1, PD-L1 and/or PD-L2; and/or
(b) said LAG-3 pathway inhibitor binds to LAG-3 and/or MHC-II.

Item 5: The combination according to any one of the preceding items, wherein said PD-1 pathway inhibitor is an antibody or a variant, fragment or derivative thereof, in particular an antigen-binding variant, fragment or derivative thereof, or a nucleic acid encoding said antibody or a variant, fragment or derivative thereof.

Item 6: The combination according to item 5, wherein said PD-1 pathway inhibitor is an anti-PD1 antibody selected from Nivolumab; Pembrolizumab; Pidilizumab; BGB-A317; MEDI0680; PDR001; REGN2810; TSR-042; AGEN-2034; AM-0001; BGB-108; BI-754091; CBT-501; ENUM-003; ENUM-388D4; IBI-308; JNJ-63723283; JS-001; JTX-4014; JY-034; MCLA-134; PF-06801591; STIA-1110; 244C8 and 388D4; an anti-PDL1 antibody selected from BMS-936559, Atezolizumab, Durvalumab, Avelumab, KD033, STI-A1014, MCLA-145, and SP142; or an anti-PDL2 antibody selected from rHigM12B7.

Item 7: The combination according to any one of items 1 to 4, wherein said PD-1 pathway inhibitor is an antagonistic binding protein, optionally selected from a fusion protein and a soluble receptor, or a nucleic acid encoding an antagonistic binding protein, optionally selected from a fusion protein and a soluble receptor.

Item 8: The combination according to item 7, wherein said fusion protein comprises (i) a PD-L1 ligand or a domain, fragment or variant thereof; and/or (ii) a PD-L2 ligand or a domain, or a fragment or variant thereof; and optionally (iii) a further entity optionally selected from an Fc immunoglobulin.

Item 9: The combination according to item 8, wherein said fusion protein is AMP-224.

Item 10: The combination according to item 7, wherein said soluble receptor is a soluble PD-1 receptor or fragment or variant thereof.

Item 11: The combination according to any one of items 1 to 5, wherein said PD-1 pathway inhibitor is a nucleic acid, preferably an RNA and more preferably an mRNA, encoding a PD-1 pathway inhibitor according to any one of items 7 to 11 or fragment or variant thereof.

Item12: The combination according to any one of items 1 to 4, wherein said PD-1 pathway inhibitor is an antagonistic nucleic acid, optionally selected from a microRNA, an siRNA, an shRNA, an antisense RNA or an aptamer.

Item 13: The combination according to any one of items 1 to 4, wherein said LAG-3 pathway inhibitor is an antibody or a variant, fragment or derivative thereof, in particular an antigen-binding variant, fragment or derivative thereof, or a nucleic acid encoding an antibody or a variant, fragment or derivative thereof, in particular an antigen-binding variant, fragment or derivative thereof.

Item 14: The combination according to item 13, wherein said antibody is an anti-LAG-3 antibody selected from BMS-986016, LAG525, GSK2831781, BI-754111, ENUM-006, FS-18, IMP-701, IMP-731, TRL-7117, and TSR-033.

Item 15: The combination according to item or 5 and/or 13, wherein said antibody is a multispecific antibody, preferably a bi- or trispecific antibody specifically binding to LAG-3 and at least one of PD-1, PD-L1 and/or PD-L2, optionally selected from MGD-013 and Sym-016.

Item 16: The combination according to any one of items 1 to 4, wherein said LAG-3 pathway inhibitor is an antagonistic binding protein or a nucleic acid encoding an antagonistic binding protein.

Item 17: The combination according to any one of items 1 to 4, wherein said LAG-3 pathway inhibitor is a nucleic acid, preferably an RNA and more preferably an mRNA, encoding a LAG-3 inhibitor according to any one of items 13 to 15 or fragment or variant thereof.

Item 18: The combination according to any one of items 1 to 4, wherein said LAG-3 pathway inhibitor is an antagonistic nucleic acid, optionally selected from a microRNA, a siRNA, a shRNA, an antisense RNA, or an aptamer.

Item 19: The combination according to any one of the preceding items, wherein said RNA encoding at least one epitope of an antigen, and/or said RNA encoding a PD-1 pathway inhibitor and/or said RNA encoding a LAG-3 pathway inhibitoris an isolated RNA.

Item 20: The combination according any one of the preceding items, wherein said RNA encoding at least one epitope of an antigen, and/or said RNA encoding a PD-1 pathway inhibitor and/or said RNA encoding a LAG-3 pathway inhibitor, is a stabilized RNA.

Item 21. The combination according to any one of the preceding items, wherein said RNA encoding at least one epitope of an antigen, and/or said RNA encoding a PD-1 pathway inhibitor and/or said RNA encoding a LAG-3 pathway inhibitor, comprises a modified RNA sequence, wherein in said modified RNA sequence
(a) the G/C content of the at least one open reading frame of said RNA sequence is increased compared to the G/C content of the corresponding coding sequence of the wild-type RNA; and/or
(b) the codon usage in the at least one open reading frame of said modified RNA sequence is adapted to the human codon usage; and/or
(c) said codon adaptation index (CAI) is increased or maximized in the coding sequence of the RNA sequence;
   wherein the amino acid sequence encoded by the at least one modified RNA sequence is preferably not being modified compared to the amino acid sequence encoded by the corresponding unmodified RNA sequence.

Item 22: The combination according to any one of the preceding items, wherein in said RNA encoding at least one epitope of an antigen, said antigen is a tumor antigen selected from: 1A01_HLA-A/m; 1A02; 5T4; ACRBP; AFP; AKAP4; alpha-actinin-_4/m; alpha-methylacyl-coenzyme_A_racemase; ANDR; ART-4; ARTC1/m; AURKB; B2MG; B3GN5; B4GN1; B7H4; BAGE-1; BASI; BCL-2; bcr/abl; beta-catenin/m; BING-4; BIRC7; BRCA1/m; BY55; calreticulin; CAMEL; CASP-8/m; CASPA; cathepsin_B; cathepsin_L; CD1A; CD1B; CD1C; CD1D; CD1E; CD20; CD22; CD276; CD33; CD3E; CD3Z; CD44_Isoform_1; CD44_lsoform_6; CD4; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen_XXIII; COX-2; CP1B1; CSAG2; CT45A1; CT55; CT-_9/BRD6; CTAG2_lsoform_LAGE-1A; CTAG2_Isoform_LAGE-1B; CTCFL; Cten; cyclin_B1; cyclin_D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_Version_1; FCGR3A_Version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8_(GAGE-2D); GASR; GnT-V; GPC3; GPNMB/m; GRM3; HAGE; hepsin; Her2/neu; HLA-A2/m; homeobox_NKX3.1; HOM-TES-85; HPG1; HS71A; HS71B; HST-2; hTERT; iCE; IF2B3; IL10; IL-13Ra2; IL2-RA; IL2-RB; IL2-RG; IL-5; IMP3; ITA5; ITB1; ITB6; kallikrein-2; kallikrein-4; KI20A; KIAA0205; KIF2C; KK-LC-1; LDLR; LGMN; LIRB2; LY6K; MAGA5; MAGA8; MAGAB; MAGE-A10; MAGE-A12; MAGE-A1; MAGE-A2; MAGE-A3; MAGE-A4; MAGE-A6; MAGE-A9; MAGE-B10; MAGE-B16; MAGE-B17; MAGE-_B1; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-_E1; MAGE-E1_(MAGE1); MAGE-E2; MAGE-F1; MAGE-H1; MAGEL2; mammaglobin_A; MART-1/melan-A; MART-2; MC1_R; M-CSF; mesothelin; MITF; MMP1_1; MMP7; MUC-1; MUM-1/m; MUM-2/m; MYCN; MYO1A; MYO1B; MYO1C; MYO1D; MYO1E; MYO1F; MYO1G; MYO1H; NA17; NA88-A; Neo-PAP; NFYC/m; NGEP; NPM; NRCAM; NSE; NUF2; NY-ESO-1; OA1; OGT; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1_-Kinase; Pin-1; PLAC1; PMEL; PML; POTEF; POTE; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-_1; SART-2; SART-3; SEPR; SERPINB5; SIA7F; SIA8A; SIAT9; SIRT2/m; SOX10; SP17; SPNXA; SPXN3; SSX-1; SSX-2; SSX3; SSX-4; ST1A1; STAG2; STAMP-1; STEAP-1; Survivin-2B; survivin; SYCP1; SYT-SSX-1; SYT-SSX-2; TARP; TCRg; TF2AA; TGFB1; TGFR2; TGM-4; TIE2; TKTL1; TPl/m; TRGV11; TRGV9; TRPC1; TRP-p8; TSG10; TSPY1; TVC_(TRGV3); TX101; tyrosinase; TYRP1; TYRP2; UPA; VEGFR1; WT1; and XAGE1 or a variant or fragment thereof; and wherein the at least one RNA is optionally monocistronic, bicistronic or multicistronic.

Item 23: The combination according to item 22, said combination comprising a plurality of at least two, at least three, at least four, at least five and preferably six epitope-encoding RNAs, said epitope-encoding RNAs preferably being monocistronic and encoding
a) at least one epitope of NY-ESO-1, or a fragment, variant or derivative thereof; and
d) at least one epitope of MAGE-C1, or a fragment, variant or derivative thereof; and
e) at least one epitope of MAGE-C2, or a fragment, variant or derivative thereof; and;
f) at least one epitope of Survivin, or a fragment, variant or derivative thereof; and optionally
g) at least one epitope of 5T4, or a fragment, variant or derivative thereof; and optionally
h) at least one epitope of MUC-1, or a fragment, variant or derivative thereof.

Item 24: The combination according to any one of the preceding items, wherein said RNA is an mRNA.

Item 25: The combination according to any one of the preceding items, wherein said RNA encoding at least one epitope of an antigen, and/or said RNA encoding a PD-1 pathway inhibitor and/or said RNA encoding a LAG-3 pathway inhibitor, comprises one or more of the following:
(a) at least one 5' cap structure; and/or
(b) optionally at least one 5'-UTR; and/or
(c) at least one 3'-UTR; and/or
(d) optionally at least one histone stem loop; and
(e) at least one poly(A) sequence and/or poly(C) sequence.

Item 26: The combination according to any one of the preceding items, wherein said RNA encoding at least one epitope of an antigen, and/or said RNA encoding a PD-1 pathway inhibitor and/or said RNA encoding a LAG-3 pathway inhibitor, is complexed or associated with at least one carrier selected from
(a) one or more cationic or polycationic compounds, preferably with cationic or polycationic polymers, cationic or polycationic peptides or proteins including protamine, cationic or polycationic polysaccharides and/or cationic or polycationic lipids; and/or
(b) one or more lipids and thereby forming liposomes, lipid nanoparticles and/or lipoplexes.

Item 27: A pharmaceutical composition comprising the combination according to any one of the preceding items and a pharmaceutically acceptable excipient, preferably a pharmaceutically acceptable carrier.

Item 28: The pharmaceutical composition according to item 27, further comprising one or more of a pharmaceutically acceptable excipient, an adjuvant, a further antigen, a further nucleic acid encoding an epitope, an immunotherapeutic or immunostimulatory agent, preferably an immunostimulatory RNA (isRNA).

Item 29: The pharmaceutical composition according to any one of items 27 or 28, wherein said pharmaceutical composition is a vaccine.

Item 30: A kit-of-parts comprising the combination according to any one of items 1 to 26, or the pharmaceutical composition according to any one of items 27 to 29.

Item 31: The kit-of-parts according to item 30, said kit-of-parts comprising:
(i) at least one RNA, said RNA comprising at least one coding sequence encoding at least one epitope of an antigen as defined in any one of the preceding items; and
(ii) at least one PD-1 pathway inhibitor as defined in any one of the preceding items; and
(iii) at least one LAG-3 pathway inhibitor as defined in any one of the preceding items.

Item 32: The combination according to any one of items 1 to 26,the pharmaceutical composition according to any one of items 27 to 29, or the kit-of-parts according to item 31 for use as a medicament.

Item 33: The combination according to any one of items 1 to 26, the pharmaceutical composition according to any one of items 27 to 29, or the kit-of-parts according to item 31 for use as a vaccine.

Item 34: The combination according to any one of items 1 to 26 or for the use according to item 32 or 33,the pharmaceutical composition according to any one of items 27 to 29 or for the use according to item 32 or 33, or the kit-of-parts according to item 31 or for the use according to item 32 or 33, for use in a method of prophylaxis or treatment of a tumor or cancer disease, an infectious disease, an allergy or an autoimmune disease.

Item 35: The combination according to any one of items 1 to 26 or for the use according to any one of items 32 to 34,the pharmaceutical composition according to any one of items 27 to 29 or for the use according to any one of items 32 to 34, or the kit-of-parts according to item 31 or for the use according to item 32 to 34 wherein said cancer is selected from Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing"s Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin"s Lymphoma, Adult; Hodgkin"s Lymphoma, Childhood; Hodgkin"s Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi"s Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS- Related; Lymphoma, Central Nervous System (Primary); Lymphoma, Cutaneous T-Cell; Lymphoma, Hodgkin"s, Adult; Lymphoma, Hodgkin"s; Childhood; Lymphoma, Hodgkin"s During Pregnancy; Lymphoma, Non-Hodgkin"s, Adult; Lymphoma, Non-Hodgkin"s, Childhood; Lymphoma, Non-Hodgkin"s During Pregnancy; Lymphoma, Primary Central Nervous System; Macroglobulinemia, Waldenstrom"s; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplasia Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin"s Lymphoma, Adult; Non- Hodgkin"s Lymphoma, Childhood; Non-Hodgkin"s Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood", Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin"s Lymphoma; Pregnancy and Non-Hodgkin"s Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland"Cancer, Childhood; Sarcoma, Ewing"s Family of Tumors; Sarcoma, Kaposi"s; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom"s Macro globulinemia; and Wilms" Tumor.

Item 36: The combination according to any one of items 1 to 21 or 23 to 26 or for the use according to any one of items 32 to 34 , the pharmaceutical composition according to any one of items 27 to 29 or for the use according to any one of items 32 to 34, or the kit-of-parts according to item 31 or for the use according to item 32 to 34, wherein said infectious disease is selected from Acinetobacter infections, African sleeping sickness (African trypanosomiasis), AIDS (Acquired immunodeficiency syndrome), Amoebiasis, Anaplasmosis, Anthrax, Appendicitis, Arcanobacterium haemolyticum infections, Argentine hemorrhagic fever, Ascariasis, Aspergillosis, Astrovirus infections, Athlete"s foot, Babesiosis, Bacillus cereus infections, Bacterial meningitis, Bacterial pneumonia, Bacterial vaginosis (BV), Bacteroides infections, Balantidiasis, Baylisascaris infections, Bilharziosis, BK virus infections, Black piedra, Blastocystis hominis infections, Blastomycosis, Bolivian hemorrhagic fever, Borrelia infectionss (Borreliosis), Botulism (and Infant botulism), Bovine tapeworm, Brazilian hemorrhagic fever, Brucellosis, Burkholderia infections, Buruli ulcer, Calicivirus infections (Norovirus and Sapovirus), Campylobacteriosis, Candidiasis (Candidosis), Canine tapeworm infections, Cat-scratch disease, Chagas Disease (American trypanosomiasis), Chancroid, Chickenpox, Chlamydia infections, Chlamydia trachomatis infections, Chlamydophila pneumoniae infections, Cholera, Chromoblastomycosis, Climatic bubo, Clonorchiasis, Clostridium difficile infections, Coccidioidomycosis, Cold, Colorado tick fever (CTF), Common cold (Acute viral rhinopharyngitis; Acute coryza), Condyloma acuminata, Conjunctivitis, Creutzfeldt-Jakob disease (CJD), Crimean-Congo hemorrhagic fever (CCHF), Cryptococcosis, Cryptosporidiosis, Cutaneous larva migrans (CLM), Cutaneous Leishmaniosis, Cyclosporiasis, Cysticercosis, Cytomegalovirus infections, Dengue fever, Dermatophytosis, Dientamoebiasis, Diphtheria, Diphyllobothriasis, Donavanosis, Dracunculiasis, Early summer meningoencephalitis (FSME), Ebola hemorrhagic fever, Echinococcosis, Ehrlichiosis, Enterobiasis (Pinworm infections), Enterococcus infections, Enterovirus infections, Epidemic typhus, Epiglottitis, Epstein-Barr Virus Infectious Mononucleosis, Erythema infectiosum (Fifth disease), Exanthem subitum, Fasciolopsiasis, Fasciolosis, Fatal familial insomnia (FFI), Fifth disease, Filariasis, Fish poisoning (Ciguatera), Fish tapeworm, Flu, Food poisoning by Clostridium perfringens, Fox tapeworm, Free-living amebic infections, Fusobacterium infections, Gas gangrene, Geotrichosis, Gerstmann-Sträussler-Scheinker syndrome (GSS), Giardiasis, Glanders, Gnathostomiasis, Gonorrhea, Granuloma inguinale (Donovanosis), Group A streptococcal infections, Group B streptococcal infections, Haemophilus influenzae infections, Hand foot and mouth disease (HFMD), Hantavirus Pulmonary Syndrome (HPS), Helicobacter pylori infections, Hemolytic-uremic syndrome (HUS), Hemorrhagic fever with renal syndrome (HFRS), Henipavirus infections, Hepatitis A, Hepatitis B, Hepatitis C, Hepatitis D, Hepatitis E, Herpes simplex, Herpes simplex type I, Herpes simplex type II, Herpes zoster, Histoplasmosis, Hollow warts, Hookworm infections, Human bocavirus infections, Human ewingii ehrlichiosis, Human granulocytic anaplasmosis (HGA), Human metapneumovirus infections, Human monocytic ehrlichiosis, Human papillomavirus (HPV) infections, Human parainfluenza virus infections, Hymenolepiasis, Influenza, Isosporiasis, Japanese encephalitis, Kawasaki disease, Keratitis, Kingella kingae infections, Kuru, Lambliasis (Giardiasis), Lassa fever, Legionellosis (Legionnaires" disease, Pontiac fever), Leishmaniasis, Leprosy, Leptospirosis, Lice, Listeriosis, Lyme borreliosis, Lyme disease, Lymphatic filariasis (Elephantiasis), Lymphocytic choriomeningitis, Malaria, Marburg hemorrhagic fever (MHF), Marburg virus, Measles, Melioidosis (Whitmore"s disease), Meningitis, Meningococcal disease, Metagonimiasis, Microsporidiosis, Miniature tapeworm, Miscarriage (prostate inflammation), Molluscum contagiosum (MC), Mononucleosis, Mumps, Murine typhus (Endemic typhus), Mycetoma, Mycoplasma hominis, Mycoplasma pneumonia, Myiasis, Nappy/diaper dermatitis, Neonatal conjunctivitis (Ophthalmia neonatorum), Neonatal sepsis (Chorioamnionitis), Nocardiosis, Noma, Norwalk virus infections, Onchocerciasis (River blindness), Osteomyelitis, Otitis media, Paracoccidioidomycosis (South American blastomycosis), Paragonimiasis, Paratyphus, Pasteurellosis, Pediculosis capitis (Head lice), Pediculosis corporis (Body lice), Pediculosis pubis (Pubic lice, Crab lice), Pelvic inflammatory disease (PID), Pertussis (Whooping cough), Pfeiffer"s glandular fever, Plague, Pneumococcal infections, Pneumocystis pneumonia (PCP), Pneumonia, Polio (childhood lameness), Poliomyelitis, Porcine tapeworm, Prevotella infections, Primary amoebic meningoencephalitis (PAM), Progressive multifocal leukoencephalopathy, Pseudo-croup, Psittacosis, Q fever, Rabbit fever, Rabies, Rat-bite fever, Reiter"s syndrome, Respiratory syncytial virus infections (RSV), Rhinosporidiosis, Rhinovirus infections, Rickettsial infections, Rickettsialpox, Rift Valley fever (RVF), Rocky mountain spotted fever (RMSF), Rotavirus infections, Rubella, Salmonella paratyphus, Salmonella typhus, Salmonellosis, SARS (Severe Acute Respiratory Syndrome), Scabies, Scarlet fever, Schistosomiasis (Bilharziosis), Scrub typhus, Sepsis, Shigellosis (Bacillary dysentery), Shingles, Smallpox (Variola), Soft chancre, Sporotrichosis, Staphylococcal food poisoning, Staphylococcal infections, Strongyloidiasis, Syphilis, Taeniasis, Tetanus, Three-day fever, Tick-borne encephalitis, Tinea barbae (Barber"s itch), Tinea capitis (Ringworm of the Scalp), Tinea corporis (Ringworm of the Body), Tinea cruris (Jock itch), Tinea manuum (Ringworm of the Hand), Tinea nigra, Tinea pedis (Athlete"s foot), Tinea unguium (Onychomycosis), Tinea versicolor (Pityriasis versicolor), Toxocariasis (Ocular Larva Migrans (OLM) and Visceral Larva Migrans (VLM)), Toxoplasmosis, Trichinellosis, Trichomoniasis, Trichuriasis (Whipworm infections), Tripper, Trypanosomiasis (sleeping sickness), Tsutsugamushi disease, Tuberculosis, Tularemia, Typhus, Typhus fever, Ureaplasma urealyticum infections, Vaginitis (Colpitis), Variant Creutzfeldt-Jakob disease (vCJD, nvCJD), Venezuelan equine encephalitis, Venezuelan hemorrhagic fever, Viral pneumonia, Visceral Leishmaniosis, Warts, West Nile Fever, Western equine encephalitis, White piedra (Tinea blanca), Whooping cough, Yeast fungus spots, Yellow fever, Yersinia pseudotuberculosis infections, Yersiniosis, and Zygomycosis.

Item 37: The combination according to any one of items 1 to 26 or for the use according to any one of items 32 to 36, the pharmaceutical composition according to any one of items 27 to 29 or for the use according to any one of items 32 to 36, or the kit-of-parts according to item 31 or for the use according to item 32 to 36, further comprising at least one adjuvant.

Item 38: The combination or pharmaceutical composition or the kit-of-parts for the use according to any one of items 32 to 37, wherein said use includes administering the RNA, the PD-1 pathway inhibitor and the LAG-3 pathway inhibitor sequentially or simultaneously to a subject in need thereof.

Item 39: The combination or pharmaceutical composition or the kit-of-parts for the use according to any one of items 32 to 38, wherein the RNA, the PD-1 pathway inhibitor and the LAG-3 pathway inhibitor are administered to a subject in need thereof via different administration routes.

Item 40: A PD-1 pathway inhibitor and/or a LAG-3 pathway inhibitor as defined in any one of the preceding items for use in therapy in combination with an RNA as defined in any one of the preceding items.

Item 41: An RNA as defined in any one of the preceding items for use in therapy in combination with a PD-1 pathway inhibitor and a LAG-3 pathway inhibitor as defined in any one of the preceding items.

Item 42: A method of treating or preventing cancer, an infectious disease, an autoimmune disease or an allergy, comprising administering to a subject in need thereof a therapeutically effective amount of a combination,pharmaceutical composition, or the kit-of-parts according to any one of the preceding items.

### EXAMPLES

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the appended claims.

### Example 1:

### 1.1 Preparation of DNA and mRNA constructs

For the present examples a DNA sequence, encoding *Gallus gallus* ovalbumin mRNA (R1710) was prepared and used for subsequent *in vitro* transcription reactions.

According to a first preparation, the DNA sequence coding for the above mentioned mRNA was prepared. The construct was prepared by modifying the wild type coding sequence by introducing a GC-optimized sequence for stabilization, followed by a stabilizing sequence derived from the alpha-globin-3'-UTR (muag (mutated alpha-globin-3'-UTR)), a stretch of 64 adenosines (poly-A-sequence), a stretch of 30 cytosines (poly-C-sequence), and a histone stem loop. In SEQ ID NO: 35 the sequence of the corresponding mRNA is shown.

### 1.2 In vitro transcription

The respective DNA plasmid prepared according to Example 1 was transcribed *in vitro* using T7 polymerase. Subsequently the mRNA was purified using PureMessenger^{®} (CureVac, Tübingen, Germany).

### 1.3 Preparation of the vaccine

The mRNA R1710 was complexed with protamine by addition of protamine to the mRNA in the ratio (1:2) (w/w) (adjuvant component). After incubation for 10 min, the same amount of free mRNA R1710 used as antigen-providing RNA was added.

OVA-RNActive vaccine (R1710): comprising an adjuvant component consisting of mRNA coding for *Gallus gallus* ovalbumin (R1710) according to SEQ ID NO. 35 complexed with protamine in a ratio of 2:1 (w/w) and the antigen-providing free mRNA coding for *Gallus gallus* ovalbumin (R1710) according to SEQ ID NO. 35 (ratio 1:1; complexed RNA:free RNA).

### Example 2: Combination of an anti-PD1, anti-LAG-3 antibody and RNA vaccine

To analyze the efficacy of RNA vaccination in combination with dual immune checkpoint inhibition, C57BL/6 mice were subcutaneously implanted with 3x10⁵ E.G7-OVA lymphoma cells per mouse (volume 100 µl in PBS). E.G7-OVA is a mouse T cell lymphoma cell line stably expressing *Gallus gallus* ovalbumin (OVA).

Intradermal vaccination with the RNA vaccine comprising OVA mRNA 1710 (32 µg/mouse/vaccination day) (according to Example 1) and treatment with the anti-PD-1/CD279 monoclonal antibody (190 µg i.p.) + anti-LAG-3 antibody (190 µg i.p.) or an isotype control started on day 4 and was repeated on days 7, 11, 14, 18 and 21. Animals received the antibody injection in the morning and were vaccinated with the RNA vaccine in the afternoon. Animals receiving RNA encoding PpLuc ("PpLuc RNActive") or antagonistic antibodies only served as control.

The anti-PD-1/CD279 antibody (clone RMP1-14, rat IgG2a), anti-LAG-3 antibody and the isotype control antibody (clone 2A3, rat IgG2a) were purchased from BioXCell (West Lebanon, NH, USA).

### Tumor growth

Tumor growth was monitored by measuring the tumor size in 3 dimensions using a calliper. Tumor growth measurement was monitored by measuring the tumor size in 3 dimensions using callipers. The volume was calculated according to the following formula: $volume \left({mm}^{3}\right) = \frac{length \left(mm\right) \times π \times {width}^{2} \left({mm}^{2}\right)}{6}$

### Scoring of health condition of the mice

During tumor growth mice were routinely assessed for their health condition by the tumor distress scoring sheet (observation of behavior and tumor appearance and size). If abnormal behavior or condition of the mice and/or tumors was observed this was recorded and the score is used as a basis for euthanasia.

### Results

The anti-tumoral response induced by the RNA vaccine was significantly enhanced by the combination with PD-1 and LAG-3 immune checkpoint inhibition. Treatment of EG.7-OVA tumor-bearing mice with PD-1 inhibitor, LAG-3 inhibitor or a combination of thereof induced significantly less tumor growth reduction in comparison to the combination of dual PD-1 and LAG-3 checkpoint inhibition with the RNA vaccine. Moreover, the combined treatment with PD-1 and LAG-3 blockade and RNA vaccine induced complete tumor eradication in the majority of the treated animals, whereas no complete tumor remission occurred in mice treated with inhibition of PD-1 and LAG-3 checkpoints alone. Only the combination of RNA vaccine with PD-1 and LAG-3 blockade induced a significant increase in survival in comparison to the single treatments with unspecific RNA vaccine, single checkpoint inhibitors or even the specific RNA vaccine.

## Claims

1. A combination comprising:
(i) at least one RNA, said RNA comprising at least one coding sequence encoding at least one epitope of an antigen; and
(ii) at least one PD-1 pathway inhibitor; and
(iii) at least one LAG-3 pathway inhibitor.

2. The combination according to claim 1, wherein said PD-1 pathway inhibitor and/or said LAG-3 pathway inhibitor are selected from an antibody or a nucleic acid encoding said antibody, a protein or a nucleic acid encoding said protein, a peptide or a nucleic acid encoding said peptide, an antagonistic nucleic acid, and a small organic molecule.

3. The combination according to any one of the preceding claims, wherein
(a) said PD-1 pathway inhibitor is a competitive or non-competitive PD-1 antagonist; and/or
(b) said LAG-3 pathway inhibitor is a competitive or non-competitive LAG-3 antagonist,
and/or
(a) said PD-1 pathway inhibitor binds to PD-1, PD-L1 and/or PD-L2; and/or
(b) said LAG-3 pathway inhibitor binds to LAG-3 and/or MHC-II.

4. The combination according to any one of the preceding claims, wherein said PD-1 pathway inhibitor is an antibody or a variant, fragment or derivative thereof, in particular an antigen-binding variant, fragment or derivative thereof, or a nucleic acid encoding said antibody or a variant, fragment or derivative thereof,
wherein said PD-1 pathway inhibitor is optionally an anti-PD1 antibody selected from Nivolumab; Pembrolizumab; Pidilizumab; BGB-A317; MEDI0680; PDR001; REGN2810; TSR-042; AGEN-2034; AM-0001; BGB-108; BI-754091; CBT-501; ENUM-003; ENUM-388D4; IBI-308; JNJ-63723283; JS-001; JTX-4014; JY-034; MCLA-134; PF-06801591; STIA-1110; 244C8 and 388D4; an anti-PDL1 antibody selected from BMS-936559, Atezolizumab, Durvalumab, Avelumab, KD033, STI-A1014, MCLA-145, and SP142; or an anti-PDL2 antibody selected from rHIgM12B7.

5. The combination according to any one of claims 1 to 3, wherein
(i) said PD-1 pathway inhibitor is an antagonistic binding protein, optionally selected from a fusion protein and a soluble receptor, or a nucleic acid encoding an antagonistic binding protein, optionally selected from a fusion protein and a soluble receptor,
wherein
(ii) said fusion protein optionally comprises (i) a PD-L1 ligand or a domain, fragment or variant thereof; and/or (ii) a PD-L2 ligand or a domain, or a fragment or variant thereof; and optionally (iii) a further entity optionally selected from an Fc immunoglobulin,
wherein
(iii) said fusion protein is further optionally AMP-224.

6. The combination according to claim 5(i), wherein said soluble receptor is a soluble PD-1 receptor or fragment or variant thereof.

7. The combination according to any one of claims 1 to 4, wherein said PD-1 pathway inhibitor is a nucleic acid, preferably an RNA and more preferably an mRNA, encoding a PD-1 pathway inhibitor according to any one of claims 7 to 11 or fragment or variant thereof.

8. The combination according to any one of claims 1 to 3, wherein (i) said PD-1 pathway inhibitor is an antagonistic nucleic acid, optionally selected from a microRNA, an siRNA, an shRNA, an antisense RNA or an aptamer,
or
wherein (ii) said LAG-3 pathway inhibitor is an antibody or a variant, fragment or derivative thereof, in particular an antigen-binding variant, fragment or derivative thereof, or a nucleic acid encoding an antibody or a variant, fragment or derivative thereof, in particular an antigen-binding variant, fragment or derivative thereof,
wherein said antibody is optionally an anti-LAG-3 antibody selected from BMS-986016, LAG525, GSK2831781, BI-754111, ENUM-006, FS-18, IMP-701, IMP-731, TRL-7117, and TSR-033.

9. The combination according to claim or 4 and/or 8(ii), wherein said antibody is a multispecific antibody, preferably a bi- or trispecific antibody specifically binding to LAG-3 and at least one of PD-1, PD-L1 and/or PD-L2, optionally selected from MGD-013 and Sym-016.

10. The combination according to any one of claims 1 to 3, wherein said LAG-3 pathway inhibitor is an antagonistic binding protein or a nucleic acid encoding an antagonistic binding protein,
or
wherein said LAG-3 pathway inhibitor is a nucleic acid, preferably an RNA and more preferably an mRNA, encoding a LAG-3 inhibitor according to any one of claims 13 to 15 or fragment or variant thereof.

11. The combination according to any one of claims 1 to 3, wherein said LAG-3 pathway inhibitor is an antagonistic nucleic acid, optionally selected from a microRNA, a siRNA, a shRNA, an antisense RNA, or an aptamer.

12. The combination according to any one of the preceding claims, wherein said RNA encoding at least one epitope of an antigen, and/or said RNA encoding a PD-1 pathway inhibitor and/or said RNA encoding a LAG-3 pathway inhibitoris an isolated RNA,
and/or
wherein said RNA encoding at least one epitope of an antigen, and/or said RNA encoding a PD-1 pathway inhibitor and/or said RNA encoding a LAG-3 pathway inhibitor, is a stabilized RNA.

13. The combination according to any one of the preceding claims, wherein said RNA encoding at least one epitope of an antigen, and/or said RNA encoding a PD-1 pathway inhibitor and/or said RNA encoding a LAG-3 pathway inhibitor, comprises a modified RNA sequence, wherein in said modified RNA sequence
(a) the G/C content of the at least one open reading frame of said RNA sequence is increased compared to the G/C content of the corresponding coding sequence of the wild-type RNA; and/or
(b) the codon usage in the at least one open reading frame of said modified RNA sequence is adapted to the human codon usage; and/or
(c) said codon adaptation index (CAI) is increased or maximized in the coding sequence of the RNA sequence;
wherein the amino acid sequence encoded by the at least one modified RNA sequence is preferably not being modified compared to the amino acid sequence encoded by the corresponding unmodified RNA sequence.

14. The combination according to any one of the preceding claims, wherein in said RNA encoding at least one epitope of an antigen, said antigen is a tumor antigen selected from: 1A01_HLA-A/m; 1A02; 5T4; ACRBP; AFP; AKAP4; alpha-actinin-_4/m; alpha-methylacyl-coenzyme_A_racemase; ANDR; ART-4; ARTC1/m; AURKB; B2MG; B3GN5; B4GN1; B7H4; BAGE-1; BASI; BCL-2; bcr/abl; beta-catenin/m; BING-4; BIRC7; BRCA1/m; BY55; calreticulin; CAMEL; CASP-8/m; CASPA; cathepsin_B; cathepsin_L; CD1A; CD1B; CD1C; CD1D; CD1E; CD20; CD22; CD276; CD33; CD3E; CD3Z; CD44_lsoform_1; CD44_Isoform_6; CD4; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen_XXIII; COX-2; CP1B1; CSAG2; CT45A1; CT55; CT-_9/BRD6; CTAG2_lsoform_LAGE-1A; CTAG2_Isoform_LAGE-1B; CTCFL; Cten; cyclin_B1; cyclin_D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_Version_1; FCGR3A_Version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8_(GAGE-2D); GASR; GnT-V; GPC3; GPNMB/m; GRM3; HAGE; hepsin; Her2/neu; HLA-A2/m; homeobox_NKX3.1; HOM-TES-85; HPG1; HS71A; HS71B; HST-2; hTERT; iCE; IF2B3; IL10; IL-13Ra2; IL2-RA; IL2-RB; IL2-RG; IL-5; IMP3; ITA5; ITB1; ITB6; kallikrein-2; kallikrein-4; KI20A; KIAA0205; KIF2C; KK-LC-1; LDLR; LGMN; LIRB2; LY6K; MAGA5; MAGA8; MAGAB; MAGE-A10; MAGE-A12; MAGE-A1; MAGE-A2; MAGE-A3; MAGE-A4; MAGE-A6; MAGE-A9; MAGE-B10; MAGE-B16; MAGE-B17; MAGE-_B1; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-_E1; MAGE-E1_(MAGE1); MAGE-E2; MAGE-F1; MAGE-H1; MAGEL2; mammaglobin_A; MART-1/melan-A; MART-2; MC1_R; M-CSF; mesothelin; MITF; MMP1_1; MMP7; MUC-1; MUM-1/m; MUM-2/m; MYCN; MYO1A; MYO1B; MYO1C; MYO1D; MYO1E; MYO1F; MYO1G; MYO1H; NA17; NA88-A; Neo-PAP; NFYC/m; NGEP; NPM; NRCAM; NSE; NUF2; NY-ESO-1; OA1; OGT; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1_-Kinase; Pin-1; PLAC1; PMEL; PML; POTEF; POTE; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-_1; SART-2; SART-3; SEPR; SERPINB5; SIA7F; SIA8A; SIAT9; SIRT2/m; SOX10; SP17; SPNXA; SPXN3; SSX-1; SSX-2; SSX3; SSX-4; ST1A1; STAG2; STAMP-1; STEAP-1; Survivin-2B; survivin; SYCP1; SYT-SSX-1; SYT-SSX-2; TARP; TCRg; TF2AA; TGFB1; TGFR2; TGM-4; TIE2; TKTL1; TPI/m; TRGV11; TRGV9; TRPC1; TRP-p8; TSG10; TSPY1; TVC_(TRGV3); TX101; tyrosinase; TYRP1; TYRP2; UPA; VEGFR1; WT1; and XAGE1 or a variant or fragment thereof; and wherein the at least one RNA is optionally monocistronic, bicistronic or multicistronic.

15. The combination according to claim 14, said combination comprising a plurality of at least two, at least three, at least four, at least five and preferably six epitope-encoding RNAs, said epitope-encoding RNAs preferably being monocistronic and encoding
a) at least one epitope of NY-ESO-1, or a fragment, variant or derivative thereof; and
d) at least one epitope of MAGE-C1, or a fragment, variant or derivative thereof; and
e) at least one epitope of MAGE-C2, or a fragment, variant or derivative thereof; and;
f) at least one epitope of Survivin, or a fragment, variant or derivative thereof; and optionally
g) at least one epitope of 5T4, or a fragment, variant or derivative thereof; and optionally
h) at least one epitope of MUC-1, or a fragment, variant or derivative thereof.

16. The combination according to any one of the preceding claims, wherein said RNA is an mRNA.

17. The combination according to any one of the preceding claims, wherein said RNA encoding at least one epitope of an antigen, and/or said RNA encoding a PD-1 pathway inhibitor and/or said RNA encoding a LAG-3 pathway inhibitor, comprises one or more of the following:
(a) at least one 5' cap structure; and/or
(b) optionally at least one 5'-UTR; and/or
(c) at least one 3'-UTR; and/or
(d) optionally at least one histone stem loop; and
(e) at least one poly(A) sequence and/or poly(C) sequence.

18. The combination according to any one of the preceding claims, wherein said RNA encoding at least one epitope of an antigen, and/or said RNA encoding a PD-1 pathway inhibitor and/or said RNA encoding a LAG-3 pathway inhibitor, is complexed or associated with at least one carrier selected from
(a) one or more cationic or polycationic compounds, preferably with cationic or polycationic polymers, cationic or polycationic peptides or proteins including protamine, cationic or polycationic polysaccharides and/or cationic or polycationic lipids; and/or
(b) one or more lipids and thereby forming liposomes, lipid nanoparticles and/or lipoplexes.

19. A pharmaceutical composition comprising the combination according to any one of the preceding claims and a pharmaceutically acceptable excipient, preferably a pharmaceutically acceptable carrier,
optionally further comprising one or more of a pharmaceutically acceptable excipient, an adjuvant, a further antigen, a further nucleic acid encoding an epitope, an immunotherapeutic or immunostimulatory agent, preferably an immunostimulatory RNA (isRNA).

20. The pharmaceutical composition according to claim 19, wherein said pharmaceutical composition is a vaccine.

21. A kit-of-parts comprising the combination according to any one of claims 1 to 18, or the pharmaceutical composition according to any one of claims 19 to 20.

22. The kit-of-parts according to claim 21, said kit-of-parts comprising:
(i) at least one RNA, said RNA comprising at least one coding sequence encoding at least one epitope of an antigen as defined in any one of the preceding claims; and
(ii) at least one PD-1 pathway inhibitor as defined in any one of the preceding claims; and
(iii) at least one LAG-3 pathway inhibitor as defined in any one of the preceding claims.

23. The combination according to any one of claims 1 to 18, the pharmaceutical composition according to any one of claims 19 to 20, or the kit-of-parts according to claim 22 for use as a medicament,
or
for use as a vaccine.

24. The combination according to any one of claims 1 to 18 or for the use according to claim23,the pharmaceutical composition according to claim 19 or 20 or for the use according to claim23, or the kit-of-parts according to claim 22or for the use according to claim23, for use in a method of prophylaxis or treatment of a tumor or cancer disease, an infectious disease, an allergy or an autoimmune disease.

25. The combination according to any one of claims 1 to 18 or for the use according to any one of claims 23 to 24,the pharmaceutical composition according to claim 19 or 20 or for the use according to any one of claims 23 to 24, or the kit-of-parts according to claim 22 or for the use according to claim 23 to 24 wherein said cancer is selected from Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing"s Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin"s Lymphoma, Adult; Hodgkin"s Lymphoma, Childhood; Hodgkin"s Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi"s Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS- Related; Lymphoma, Central Nervous System (Primary); Lymphoma, Cutaneous T-Cell; Lymphoma, Hodgkin"s, Adult; Lymphoma, Hodgkin"s; Childhood; Lymphoma, Hodgkin"s During Pregnancy; Lymphoma, Non-Hodgkin"s, Adult; Lymphoma, Non-Hodgkin"s, Childhood; Lymphoma, Non-Hodgkin"s During Pregnancy; Lymphoma, Primary Central Nervous System; Macroglobulinemia, Waldenstrom"s; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplasia Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin"s Lymphoma, Adult; Non- Hodgkin"s Lymphoma, Childhood; Non-Hodgkin"s Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood", Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin"s Lymphoma; Pregnancy and Non-Hodgkin"s Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland"Cancer, Childhood; Sarcoma, Ewing"s Family of Tumors; Sarcoma, Kaposi"s; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom"s Macro globulinemia; and Wilms" Tumor.

26. The combination according to any one of claims 1 to 13 or 15 to 19 or for the use according to any one of claims 23 to 24 , the pharmaceutical composition according to claim 19 or 20 or for the use according to any one of claims 23 to 24, or the kit-of-parts according to claim 22 or for the use according to claim 23 to 24, wherein said infectious disease is selected from Acinetobacter infections, African sleeping sickness (African trypanosomiasis), AIDS (Acquired immunodeficiency syndrome), Amoebiasis, Anaplasmosis, Anthrax, Appendicitis, Arcanobacterium haemolyticum infections, Argentine hemorrhagic fever, Ascariasis, Aspergillosis, Astrovirus infections, Athlete"s foot, Babesiosis, Bacillus cereus infections, Bacterial meningitis, Bacterial pneumonia, Bacterial vaginosis (BV), Bacteroides infections, Balantidiasis, Baylisascaris infections, Bilharziosis, BK virus infections, Black piedra, Blastocystis hominis infections, Blastomycosis, Bolivian hemorrhagic fever, Borrelia infectionss (Borreliosis), Botulism (and Infant botulism), Bovine tapeworm, Brazilian hemorrhagic fever, Brucellosis, Burkholderia infections, Buruli ulcer, Calicivirus infections (Norovirus and Sapovirus), Campylobacteriosis, Candidiasis (Candidosis), Canine tapeworm infections, Cat-scratch disease, Chagas Disease (American trypanosomiasis), Chancroid, Chickenpox, Chlamydia infections, Chlamydia trachomatis infections, Chlamydophila pneumoniae infections, Cholera, Chromoblastomycosis, Climatic bubo, Clonorchiasis, Clostridium difficile infections, Coccidioidomycosis, Cold, Colorado tick fever (CTF), Common cold (Acute viral rhinopharyngitis; Acute coryza), Condyloma acuminata, Conjunctivitis, Creutzfeldt-Jakob disease (CJD), Crimean-Congo hemorrhagic fever (CCHF), Cryptococcosis, Cryptosporidiosis, Cutaneous larva migrans (CLM), Cutaneous Leishmaniosis, Cyclosporiasis, Cysticercosis, Cytomegalovirus infections, Dengue fever, Dermatophytosis, Dientamoebiasis, Diphtheria, Diphyllobothriasis, Donavanosis, Dracunculiasis, Early summer meningoencephalitis (FSME), Ebola hemorrhagic fever, Echinococcosis, Ehrlichiosis, Enterobiasis (Pinworm infections), Enterococcus infections, Enterovirus infections, Epidemic typhus, Epiglottitis, Epstein-Barr Virus Infectious Mononucleosis, Erythema infectiosum (Fifth disease), Exanthem subitum, Fasciolopsiasis, Fasciolosis, Fatal familial insomnia (FFI), Fifth disease, Filariasis, Fish poisoning (Ciguatera), Fish tapeworm, Flu, Food poisoning by Clostridium perfringens, Fox tapeworm, Free-living amebic infections, Fusobacterium infections, Gas gangrene, Geotrichosis, Gerstmann-Sträussler-Scheinker syndrome (GSS), Giardiasis, Glanders, Gnathostomiasis, Gonorrhea, Granuloma inguinale (Donovanosis), Group A streptococcal infections, Group B streptococcal infections, Haemophilus influenzae infections, Hand foot and mouth disease (HFMD), Hantavirus Pulmonary Syndrome (HPS), Helicobacter pylori infections, Hemolytic-uremic syndrome (HUS), Hemorrhagic fever with renal syndrome (HFRS), Henipavirus infections, Hepatitis A, Hepatitis B, Hepatitis C, Hepatitis D, Hepatitis E, Herpes simplex, Herpes simplex type I, Herpes simplex type II, Herpes zoster, Histoplasmosis, Hollow warts, Hookworm infections, Human bocavirus infections, Human ewingii ehrlichiosis, Human granulocytic anaplasmosis (HGA), Human metapneumovirus infections, Human monocytic ehrlichiosis, Human papillomavirus (HPV) infections, Human parainfluenza virus infections, Hymenolepiasis, Influenza, isosporiasis, Japanese encephalitis, Kawasaki disease, Keratitis, Kingella kingae infections, Kuru, Lambliasis (Giardiasis), Lassa fever, Legionellosis (Legionnaires" disease, Pontiac fever), Leishmaniasis, Leprosy, Leptospirosis, Lice, Listeriosis, Lyme borreliosis, Lyme disease, Lymphatic filariasis (Elephantiasis), Lymphocytic choriomeningitis, Malaria, Marburg hemorrhagic fever (MHF), Marburg virus, Measles, Melioidosis (Whitmore"s disease), Meningitis, Meningococcal disease, Metagonimiasis, Microsporidiosis, Miniature tapeworm, Miscarriage (prostate inflammation), Molluscum contagiosum (MC), Mononucleosis, Mumps, Murine typhus (Endemic typhus), Mycetoma, Mycoplasma hominis, Mycoplasma pneumonia, Myiasis, Nappy/diaper dermatitis, Neonatal conjunctivitis (Ophthalmia neonatorum), Neonatal sepsis (Chorioamnionitis), Nocardiosis, Noma, Norwalk virus infections, Onchocerciasis (River blindness), Osteomyelitis, Otitis media, Paracoccidioidomycosis (South American blastomycosis), Paragonimiasis, Paratyphus, Pasteurellosis, Pediculosis capitis (Head lice), Pediculosis corporis (Body lice), Pediculosis pubis (Pubic lice, Crab lice), Pelvic inflammatory disease (PID), Pertussis (Whooping cough), Pfeiffer's glandular fever, Plague, Pneumococcal infections, Pneumocystis pneumonia (PCP), Pneumonia, Polio (childhood lameness), Poliomyelitis, Porcine tapeworm, Prevotella infections, Primary amoebic meningoencephalitis (PAM), Progressive multifocal leukoencephalopathy, Pseudo-croup, Psittacosis, Q fever, Rabbit fever, Rabies, Rat-bite fever, Reiter"s syndrome, Respiratory syncytial virus infections (RSV), Rhinosporidiosis, Rhinovirus infections, Rickettsial infections, Rickettsialpox, Rift Valley fever (RVF), Rocky mountain spotted fever (RMSF), Rotavirus infections, Rubella, Salmonella paratyphus, Salmonella typhus, Salmonellosis, SARS (Severe Acute Respiratory Syndrome), Scabies, Scarlet fever, Schistosomiasis (Bilharziosis), Scrub typhus, Sepsis, Shigellosis (Bacillary dysentery), Shingles, Smallpox (Variola), Soft chancre, Sporotrichosis, Staphylococcal food poisoning, Staphylococcal infections, Strongyloidiasis, Syphilis, Taeniasis, Tetanus, Three-day fever, Tick-borne encephalitis, Tinea barbae (Barber"s itch), Tinea capitis (Ringworm of the Scalp), Tinea corporis (Ringworm of the Body), Tinea cruris (Jock itch), Tinea manuum (Ringworm of the Hand), Tinea nigra, Tinea pedis (Athlete"s foot), Tinea unguium (Onychomycosis), Tinea versicolor (Pityriasis versicolor), Toxocariasis (Ocular Larva Migrans (OLM) and Visceral Larva Migrans (VLM)), Toxoplasmosis, Trichinellosis, Trichomoniasis, Trichuriasis (Whipworm infections), Tripper, Trypanosomiasis (sleeping sickness), Tsutsugamushi disease, Tuberculosis, Tularemia, Typhus, Typhus fever, Ureaplasma urealyticum infections, Vaginitis (Colpitis), Variant Creutzfeldt-Jakob disease (vCJD, nvCJD), Venezuelan equine encephalitis, Venezuelan hemorrhagic fever, Viral pneumonia, Visceral Leishmaniosis, Warts, West Nile Fever, Western equine encephalitis, White piedra (Tinea blanca), Whooping cough, Yeast fungus spots, Yellow fever, Yersinia pseudotuberculosis infections, Yersiniosis, and Zygomycosis.

27. The combination according to any one of claims 1 to 18 or for the use according to any one of claims 23 to 26, the pharmaceutical composition according to any one of claims 19 to 20 or for the use according to any one of claims 23 to 26, or the kit-of-parts according to claim 22 or for the use according to claim 23 to 26, further comprising at least one adjuvant.

28. The combination or pharmaceutical composition or the kit-of-parts for the use according to any one of claims 23 to 27, wherein said use includes administering the RNA, the PD-1 pathway inhibitor and the LAG-3 pathway inhibitor sequentially or simultaneously to a subject in need thereof,
and/or
wherein the RNA, the PD-1 pathway inhibitor and the LAG-3 pathway inhibitor are administered to a subject in need thereof via different administration routes.

29. A PD-1 pathway inhibitor and/or a LAG-3 pathway inhibitor as defined in any one of the preceding claims for use in therapy in combination with an RNA as defined in any one of the preceding claims.

30. An RNA as defined in any one of the preceding claims for use in therapy in combination with a PD-1 pathway inhibitor and a LAG-3 pathway inhibitor as defined in any one of the preceding claims.
